# EUROPEAN PATENT SPECIFICATION

(11) **EP 1 558 728 B1**
(45) Date of publication and mention of the grant of the patent: **27.06.2007**
(21) Application number: 03810380.0
(22) Date of filing: 07.11.2003
(51) Int. Cl.: C12N 9/10, C07H 5/06

(54) **METHODS TO IDENTIFY AGENTS MODULATING FUNCTIONS OF POLYPEPTIDE GALNAC-TRANSFERASES, PHARMACEUTICAL COMPOSITIONS COMPRISING SUCH AGENTS AND THE USE OF SUCH AGENTS FOR PREPARING MEDICAMENTS**
VERFAHREN ZUR IDENTIFIZIERUNG VON DIE FUNKTIONEN VON POLYPEPTID-GALNAC-TRANSFERASEN MODULIERENDEN AGENTIEN, SOLCHE AGENTIEN UMFASSENDE PHARMAZEUTISCHE ZUSAMMENSETZUNGEN UND VERWENDUNG SOLCHER AGENTIEN ZUR HERSTELLUNG VON ARZNEIMITTELN
METHODES SERVANT A IDENTIFIER DES AGENTS MODULANT LES FONCTIONS DE GALNAC-TRANSFERASES DE POLYPEPTIDES, COMPOSITIONS PHARMACEUTIQUES CONTENANT CES AGENTS ET UTILISATION DE CES AGENTS POUR PREPARER DES MEDICAMENTS

(30) Priority: 08.11.2002 US 425204 P
(43) Date of publication of application: 03.08.2005
(73) Proprietor: Glycozym ApS, 2970 Horsholm (DK)
(72) Inventor: CLAUSEN, Henrik, DK-2840 Holte (DK); BENNETT, Eric, Paul, DK-2800 Lyngby (DK)
(74) Representative: Olsen, Lars Pallisgaard
(86) International application number: PCT/DK2003/000763
(87) International publication number: WO 2004/042075

(56) References cited:
- WO-A-01/85215
- US-A- 2003 186 850
- TENNO MARI ET AL: "Identification of two cysteine residues involved in the binding of UDP-GalNAc to UDP-GalNAc:polypeptide N-acetylgalactosaminyltransferase 1 (GalNAc-T1)." EUROPEAN JOURNAL OF BIOCHEMISTRY / FEBS. GERMANY SEP 2002, vol. 269, no. 17, September 2002 (2002-09), pages 4308-4316, XP002277318 ISSN: 0014-2956
- HAGEN F K ET AL: "Structure-function analysis of the UDP-N-acetyl-D-galactosamine:polyp eptide N-acetylgalactosaminyltransferase. Essential residues lie in a predicted active site cleft resembling a lactose repressor fold." THE JOURNAL OF BIOLOGICAL CHEMISTRY. UNITED STATES 5 MAR 1999, vol. 274, no. 10, 5 March 1999 (1999-03-05), pages 6797-6803, XP002277319 ISSN: 0021-9258
- HASSAN H ET AL: "The lectin domain of UDP-N-acetyl-D-galactosamine: polypeptide N-acetylgalactosaminyltransferase-T4 directs its glycopeptide specificities." THE JOURNAL OF BIOLOGICAL CHEMISTRY. UNITED STATES 8 DEC 2000, vol. 275, no. 49, 8 December 2000 (2000-12-08), pages 38197-38205, XP002277320 ISSN: 0021-9258
- IMBERTY A ET AL: "Fold recognition and molecular modeling of a lectin-like domain in UDP-GalNac:polypeptide N-acetylgalactosaminyltransferases." PROTEIN ENGINEERING. ENGLAND DEC 1997, vol. 10, no. 12, December 1997 (1997-12), pages 1353-1356, XP002277321 ISSN: 0269-2139

### SEQUENCE LISTING

<110> ApS, Glycosym
   <120> Methods to identify agents modulating functions of polypeptide GalNAc-transfereases, pharmaceutical compositions comprising such agents and the use of such agents for preparing medicaments
<130> P200201742WO
<140> PCT/DK03/00763
   <141> 2003-11-07
<150> US 60/425,204
   <151> 2002-11-08
<160> 108
<170> PatentIn version 3.2
<210> 1
   <211> 513
   <212> DNA
   <213> Homo sapiens
<220>
   <221> CDS
   <222> (10)..(513)
   <223> GalNAC-T1 lectin domain CDS
<400> 1
   aaagaagct tat gga gat ata tcg tca aga gtt ggt cta aga cac aaa cta      51
             Tyr Gly Asp Ile Ser Ser Arg Val Gly Leu Arg His Lys Leu
             1               5                   10
   caa tgc aaa cct ttt tcc tgg tac cta gag aat ata tat cct gat tct        99
   Gln Cys Lys Pro Phe Ser Trp Tyr Leu Glu Asn Ile Tyr Pro Asp Ser
   15                  20                  25                  30
   caa att cca cgt cac tat ttc tca ttg gga gag ata cga aat gtg gaa       147
   Gln Ile Pro Arg His Tyr Phe Ser Leu Gly Glu Ile Arg Asn Val Glu
                   35                  40                  45
   acg aat cag tgt cta gat aac atg gct aga aaa gag aat gaa aaa gtt       195
   Thr Asn Gln Cys Leu Asp Asn Met Ala Arg Lys Glu Asn Glu Lys Val
               50                  55                  60
   gga att ttt aat tgc cat ggt atg ggg ggt aat cag gtt ttc tct tat       243
   Gly Ile Phe Asn Cys His Gly Met Gly Gly Asn Gln Val Phe Ser Tyr
           65                  70                  75
   act gcc aac aaa gaa att aga aca gat gac ctt tgc ttg gat gtt tcc       291
   Thr Ala Asn Lys Glu Ile Arg Thr Asp Asp Leu Cys Leu Asp Val Ser
       80                  85                  90
   aaa ctt aat ggc cca gtt aca atg ctc aaa tgc cac cac cta aaa ggc       339
   Lys Leu Asn Gly Pro Val Thr Met Leu Lys Cys His His Leu Lys Gly
   95                  100                 105                 110
   aac caa ctc tgg gag tat gac cca gtg aaa tta acc ctg cag cat gtg       387
   Asn Gln Leu Trp Glu Tyr Asp Pro Val Lys Leu Thr Leu Gln His Val
                   115                 120                 125
   aac agt aat cag tgc ctg gat aaa gcc aca gaa gag gat agc cag gtg       435
   Asn Ser Asn Gln Cys Leu Asp Lys Ala Thr Glu Glu Asp Ser Gln Val
               130                 135                 140
   ccc agc att aga gac tgc aat gga agt cgg tcc cag cag tgg ctt ctt       483
   Pro Ser Ile Arg Asp Cys Asn Gly Ser Arg Ser Gln Gln Trp Leu Leu
           145                 150                 155
   cga aac gtc acc ctt cca gaa ata ttc tga                               513
   Arg Asn Val Thr Leu Pro Glu Ile Phe
       160                 165
<210> 2
   <211> 167
   <212> PRT
   <213> Homo sapiens
<400> 2
   Tyr Gly Asp Ile Ser Ser Arg Val Gly Leu Arg His Lys Leu Gln Cys
   1               5                   10                  15
   Lys Pro Phe Ser Trp Tyr Leu Glu Asn Ile Tyr Pro Asp Ser Gln Ile
               20                  25                  30
   Pro Arg His Tyr Phe Ser Leu Gly Glu Ile Arg Asn Val Glu Thr Asn
           35                  40                  45
   Gln Cys Leu Asp Asn Met Ala Arg Lys Glu Asn Glu Lys Val Gly Ile
       50                  55                  60
   Phe Asn Cys His Gly Met Gly Gly Asn Gln Val Phe Ser Tyr Thr Ala
   65                  70                  75                  80
   Asn Lys Glu Ile Arg Thr Asp Asp Leu Cys Leu Asp Val Ser Lys Leu
                   85                  90                  95
   Asn Gly Pro Val Thr Met Leu Lys Cys His His Leu Lys Gly Asn Gln
               100                 105                 110
   Leu Trp Glu Tyr Asp Pro Val Lys Leu Thr Leu Gln His Val Asn Ser
           115                 120                 125
   Asn Gln Cys Leu Asp Lys Ala Thr Glu Glu Asp Ser Gln Val Pro Ser
       130                 135                 140
   Ile Arg Asp Cys Asn Gly Ser Arg Ser Gln Gln Trp Leu Leu Arg Asn
   145                 150                 155                 160
   Val Thr Leu Pro Glu Ile Phe
                   165
<210> 3
   <211> 417
   <212> DNA
   <213> Homo sapiens
<220>
   <221> CDS
   <222> (1)..(417)
   <223> GalNAc-T2 lectin domain
<400> 3
   tat cca gag tta agg gtt cca gac cat cag gat ata gct ttt ggg gcc      48
   Tyr Pro Glu Leu Arg Val Pro Asp His Gln Asp Ile Ala Phe Gly Ala
   1               5                   10                  15
   ttg cag cag gga act aac tgc ctc gac act ttg gga cac ttt gct gat      96
   Leu Gln Gln Gly Thr Asn Cys Leu Asp Thr Leu Gly His Phe Ala Asp
               20                  25                  30
   ggt gtg gtt gga gtt tat gaa tgt cac aat gct ggg gga aac cag gaa     144
   Gly Val Val Gly Val Tyr Glu Cys His Asn Ala Gly Gly Asn Gln Glu
           35                  40                  45
   tgg gcc ttg acg aag gag aag tcg gtg aag cac atg gat ttg tgc ctt     192
   Trp Ala Leu Thr Lys Glu Lys Ser Val Lys His Met Asp Leu Cys Leu
       50                  55                  60
   act gtg gtg gac cgg gca ccg ggc tct ctt ata aag ctg cag ggc tgc     240
   Thr Val Val Asp Arg Ala Pro Gly Ser Leu Ile Lys Leu Gln Gly Cys
   65                  70                  75                  80
   cga gaa aat gac agc aga cag aaa tgg gaa cag atc gag ggc aac tcc     288
   Arg Glu Asn Asp Ser Arg Gln Lys Trp Glu Gln Ile Glu Gly Asn Ser
                   85                  90                  95
   aag ctg agg cac gtg ggc agc aac ctg tgc ctg gac agt cgc acg gcc     336
   Lys Leu Arg His Val Gly Ser Asn Leu Cys Leu Asp Ser Arg Thr Ala
               100                 105                 110
   aag agc ggg ggc cta agc gtg gag gtg tgt ggc ccg gcc ctt tcg cag     384
   Lys Ser Gly Gly Leu Ser Val Glu Val Cys Gly Pro Ala Leu Ser Gln
           115                 120                 125
   cag tgg aag ttc acg ctc aac ctg cag cag tag                         417
   Gln Trp Lys Phe Thr Leu Asn Leu Gln Gln
       130                 135
<210> 4
   <211> 138
   <212> PRT
   <213> Homo sapiens
<400> 4
   Tyr Pro Glu Leu Arg Val Pro Asp His Gln Asp Ile Ala Phe Gly Ala
   1               5                   10                  15
   Leu Gln Gln Gly Thr Asn Cys Leu Asp Thr Leu Gly His Phe Ala Asp
               20                  25                  30
   Gly Val Val Gly Val Tyr Glu Cys His Asn Ala Gly Gly Asn Gln Glu
           35                  40                  45
   Trp Ala Leu Thr Lys Glu Lys Ser Val Lys His Met Asp Leu Cys Leu
       50                  55                  60
   Thr Val Val Asp Arg Ala Pro Gly Ser Leu Ile Lys Leu Gln Gly Cys
   65                  70                  75                  80
   Arg Glu Asn Asp Ser Arg Gln Lys Trp Glu Gln Ile Glu Gly Asn Ser
   85 90 95
   Lys Leu Arg His Val Gly Ser Asn Leu Cys Leu Asp Ser Arg Thr Ala
   100 105 110
   Lys Ser Gly Gly Leu Ser Val Glu Val Cys Gly Pro Ala Leu Ser Gln
   115 120 125
   Gln Trp Lys Phe Thr Leu Asn Leu Gln Gln
   130 135
<210> 5
   <211> 507
   <212> DNA
   <213> Homo sapiens
<220>
   <221> CDS
   <222> (4)..(507)
   <223> GalNAc-T3 lectin domain
<400> 5
   tca ttt ggt gat ctt tca aaa aga ttt gaa ata aaa cac cgt ctt cgg      48
       Phe Gly Asp Leu Ser Lys Arg Phe Glu Ile Lys His Arg Leu Arg
       1               5                   10                  15
   tgt aaa aat ttt aca tgg tat ctg aac aac att tat cca gag gtg tat      96
   Cys Lys Asn Phe Thr Trp Tyr Leu Asn Asn Ile Tyr Pro Glu Val Tyr
                   20                  25                  30
   gtg cca gac ctt aat cct gtt ata tct gga tac att aaa agc gtt ggt     144
   Val Pro Asp Leu Asn Pro Val Ile Ser Gly Tyr Ile Lys Ser Val Gly
               35                  40                  45
   cag cct cta tgt ctg gat gtt gga gaa aac aat caa gga ggc aaa cca     192
   Gln Pro Leu Cys Leu Asp Val Gly Glu Asn Asn Gln Gly Gly Lys Pro
           50                  55                  60
   tta att atg tat aca tgt cat gga ctt ggg gga aac cag tac ttt gaa     240
   Leu Ile Met Tyr Thr Cys His Gly Leu Gly Gly Asn Gln Tyr Phe Glu
       65                  70                  75
   tac tct gct caa cat gaa att cgg cac aac atc cag aag gaa tta tgt     288
   Tyr Ser Ala Gln His Glu Ile Arg His Asn Ile Gln Lys Glu Leu Cys
   80                  85                  90                  95
   ctt cat gct gct caa ggt ctc gtt cag ctg aag gca tgt acc tac aaa     336
   Leu His Ala Ala Gln Gly Leu Val Gln Leu Lys Ala Cys Thr Tyr Lys
                   100                 105                 110
   ggt cac aag aca gtt gtc act gga gag cag ata tgg gag atc cag aag     384
   Gly His Lys Thr Val Val Thr Gly Glu Gln Ile Trp Glu Ile Gln Lys
               115                 120                 125
   gat caa ctt cta tac aat cca ttc tta aaa atg tgc ctt tca gca aat     432
   Asp Gln Leu Leu Tyr Asn Pro Phe Leu Lys Met Cys Leu Ser Ala Asn
           130                 135                 140
   gga gag cat cca agt tta gtg tca tgc aac cca tca gat cca ctc caa     480
   Gly Glu His Pro Ser Leu Val Ser Cys Asn Pro Ser Asp Pro Leu Gln
       145                 150                 155
   aaa tgg ata ctt agc caa aat gat taa                                 507
   Lys Trp Ile Leu Ser Gln Asn Asp
   160                 165
<210> 6
   <211> 167
   <212> PRT
   <213> Homo sapiens
<400> 6
   Phe Gly Asp Leu Ser Lys Arg Phe Glu Ile Lys His Arg Leu Arg Cys
   1               5                   10                  15
   Lys Asn Phe Thr Trp Tyr Leu Asn Asn Ile Tyr Pro Glu Val Tyr Val
               20                  25                  30
   Pro Asp Leu Asn Pro Val Ile Ser Gly Tyr Ile Lys Ser Val Gly Gln
           35                  40                  45
   Pro Leu Cys Leu Asp Val Gly Glu Asn Asn Gln Gly Gly Lys Pro Leu
       50                  55                  60
   Ile Met Tyr Thr Cys His Gly Leu Gly Gly Asn Gln Tyr Phe Glu Tyr
   65                  70                  75                  80
   Ser Ala Gln His Glu Ile Arg His Asn Ile Gln Lys Glu Leu Cys Leu
                   85                  90                  95
   His Ala Ala Gln Gly Leu Val Gln Leu Lys Ala Cys Thr Tyr Lys Gly
               100                 105                 110
   His Lys Thr Val Val Thr Gly Glu Gln Ile Trp Glu Ile Gln Lys Asp
           115                 120                 125
   Gln Leu Leu Tyr Asn Pro Phe Leu Lys Met Cys Leu Ser Ala Asn Gly
       130                 135                 140
   Glu His Pro Ser Leu Val Ser Cys Asn Pro Ser Asp Pro Leu Gln Lys
   145                 150                 155                 160
   Trp Ile Leu Ser Gln Asn Asp
                   165
<210> 7
   <211> 423
   <212> DNA
   <213> Homo sapiens
<220>
   <221> misc_feature
   <223> GalNAc-T4 lectin DNA sequence
<400> 7
   gaggatagac caggctggca tggggctatt cgcagtagag ggatctcgtc tgaatgttta      60
   gattataatt ctcctgacaa caaccccaca ggtgctaacc tttcactgtt tggatgccat    120
   ggtcaaggag gcaatcaatt ctttgaatat acttcaaaca aagaaataag gtttaattct    180
   gtgacagagt tatgtgcaga ggtacctgag caaaaaaatt atgtgggaat gcaaaattgt    240
   cccaaagatg ggttccctgt accagcaaac attatttggc attttaaaga agatggaact    300
   atttttcacc cacactcagg actgtgtctt agtgcttatc ggacaccgga gggccgacct    360
   gatgtacaaa tgagaacttg tgatgctcta gataaaaatc aaatttggag ttttgagaaa    420
   tag                                                                  423
<210> 8
   <211> 174
   <212> PRT
   <213> Homo sapiens
<220>
   <221> misc_feature
   <223> GalNAc-T4 lectin domain protein sequence
<400> 8
   Ala Tyr Gly Asp Ile Ser Glu Arg Lys Leu Leu Arg Glu Arg Leu Arg
   1               5                   10                  15
   Cys Lys Ser Phe Asp Trp Tyr Leu Lys Asn Val Phe Pro Asn Leu His
               20                  25                  30
   Val Pro Glu Asp Arg Pro Gly Trp His Gly Ala Ile Arg Ser Arg Gly
           35                  40                  45
   Ile Ser Ser Glu Cys Leu Asp Tyr Asn Ser Pro Asp Asn Asn Pro Thr
       50                  55                  60
   Gly Ala Asn Leu Ser Leu Phe Gly Cys His Gly Gln Gly Gly Asn Gln
   65                  70                  75                  80
   Phe Phe Glu Tyr Thr Ser Asn Lys Glu Ile Arg Phe Asn Ser Val Thr
                   85                  90                  95
   Glu Leu Cys Ala Glu Val Pro Glu Gln Lys Asn Tyr Val Gly Met Gln
               100                 105                 110
   Asn Cys Pro Lys Asp Gly Phe Pro Val Pro Ala Asn Ile Ile Trp His
           115                 120                 125
   Phe Lys Glu Asp Gly Thr Ile Phe His Pro His Ser Gly Leu Cys Leu
       130                 135                 140
   Ser Ala Tyr Arg Thr Pro Glu Gly Arg Pro Asp Val Gln Met Arg Thr
   145                 150                 155                 160
   Cys Asp Ala Leu Asp Lys Asn Gln Ile Trp Ser Phe Glu Lys
                   165                 170
<210> 9
   <211> 510
   <212> DNA
   <213> Homo sapiens
<220>
   <221> CDS
   <222> (4)..(510)
   <223> GalNAc-T5 lectin domain
<400> 9
   tta gat gtt ggc aac ctc acc cag caa agg gag ctg cga aag aaa ctg        48
       Asp Val Gly Asn Leu Thr Gln Gln Arg Glu Leu Arg Lys Lys Leu
       1               5                   10                  15
   aag tgc aaa agt ttc aaa tgg tac ttg gag aat gtc ttt cct gac tta        96
   Lys Cys Lys Ser Phe Lys Trp Tyr Leu Glu Asn Val Phe Pro Asp Leu
                   20                  25                  30
   agg gct ccc att gtg aga gct agt ggt gtg ctt att aat gtg gct ttg       144
   Arg Ala Pro Ile Val Arg Ala Ser Gly Val Leu Ile Asn Val Ala Leu
               35                  40                  45
   ggt aaa tgc att tcc att gaa aac act aca gtc att ctg gaa gac tgc       192
   Gly Lys Cys Ile Ser Ile Glu Asn Thr Thr Val Ile Leu Glu Asp Cys
           50                  55                  60
   gat ggg agc aaa gag ctt caa caa ttt aat tac acc tgg tta aga ctt       240
   Asp Gly Ser Lys Glu Leu Gln Gln Phe Asn Tyr Thr Trp Leu Arg Leu
       65                  70                  75
   att aaa tgt gga gaa tgg tgt ata gcc ccc atc cct gat aaa gga gcc       288
   Ile Lys Cys Gly Glu Trp Cys Ile Ala Pro Ile Pro Asp Lys Gly Ala
   80                  85                  90                  95
   gta agg ctg cac cct tgt gat aac aga aac aaa ggg cta aaa tgg ctg       336
   Val Arg Leu His Pro Cys Asp Asn Arg Asn Lys Gly Leu Lys Trp Leu
                   100                 105                 110
   cat aaa tca aca tca gtc ttt cat cca gaa ctg gtg aat cac att gtt       384
   His Lys Ser Thr Ser Val Phe His Pro Glu Leu Val Asn His Ile Val
               115                 120                 125
   ttt gaa aac aat cag caa tta tta tgc ttg gaa gga aat ttt tct caa       432
   Phe Glu Asn Asn Gln Gln Leu Leu Cys Leu Glu Gly Asn Phe Ser Gln
           130                 135                 140
   aag atc ctg aaa gta gct gcc tgt gac cca gtg aag cca tat caa aag       480
   Lys Ile Leu Lys Val Ala Ala Cys Asp Pro Val Lys Pro Tyr Gln Lys
       145                 150                 155
   tgg aaa ttt gaa aaa tat tat gaa gcc tga                               510
   Trp Lys Phe Glu Lys Tyr Tyr Glu Ala
   160                 165
<210> 10
   <211> 168
   <212> PRT
   <213> Homo sapiens
<400> 10
   Asp Val Gly Asn Leu Thr Gln Gln Arg Glu Leu Arg Lys Lys Leu Lys
   1 5 10 15
   Cys Lys Ser Phe Lys Trp Tyr Leu Glu Asn Val Phe Pro Asp Leu Arg
   20 25 30
   Ala Pro Ile Val Arg Ala Ser Gly Val Leu Ile Asn Val Ala Leu Gly
   35 40 45
   Lys Cys Ile Ser Ile Glu Asn Thr Thr Val Ile Leu Glu Asp Cys Asp
   50 55 60
   Gly Ser Lys Glu Leu Gln Gln Phe Asn Tyr Thr Trp Leu Arg Leu Ile
   65 70 75 80
   Lys Cys Gly Glu Trp Cys Ile Ala Pro Ile Pro Asp Lys Gly Ala Val
   85 90 95
   Arg Leu His Pro Cys Asp Asn Arg Asn Lys Gly Leu Lys Trp Leu His
   100 105 110
   Lys Ser Thr Ser Val Phe His Pro Glu Leu Val Asn His Ile Val Phe
   115 120 125
   Glu Asn Asn Gln Gln Leu Leu Cys Leu Glu Gly Asn Phe Ser Gln Lys
   130 135 140
   Ile Leu Lys Val Ala Ala Cys Asp Pro Val Lys Pro Tyr Gln Lys Trp
   145 150 155 160
   Lys Phe Glu Lys Tyr Tyr Glu Ala
   165
<210> 11
   <211> 498
   <212> DNA
   <213> Homo sapiens
<220>
   <221> CDS
   <222> (1)..(498)
   <223> GalNAc-T6 lectin domain
<400> 11
   tcc ttc ggt gac att tcg gaa cga ctg cag ctg agg gaa caa ctg cac      48
   Ser Phe Gly Asp Ile Ser Glu Arg Leu Gln Leu Arg Glu Gln Leu His
   1               5                   10                  15
   tgt cac aac ttt tcc tgg tac ctg cac aat gtc tac cca gag atg ttt      96
   Cys His Asn Phe Ser Trp Tyr Leu His Asn Val Tyr Pro Glu Met Phe
               20                  25                  30
   gtt cct gac ctg acg ccc acc ttc tat ggt gcc atc aag aac ctc ggc      144
   Val Pro Asp Leu Thr Pro Thr Phe Tyr Gly Ala Ile Lys Asn Leu Gly
           35                  40                  45
   acc aac caa tgc ctg gat gtg ggt gag aac aac cgc ggg ggg aag ccc      192
   Thr Asn Gln Cys Leu Asp Val Gly Glu Asn Asn Arg Gly Gly Lys Pro
       50                  55                  60
   ctc atc atg tac tcc tgc cac ggc ctt ggc ggc aac cag tac ttt gag      240
   Leu Ile Met Tyr Ser Cys His Gly Leu Gly Gly Asn Gln Tyr Phe Glu
   65                  70                  75                  80
   tac aca act cag agg gac ctt cgc cac aac atc gca aag cag ctg tgt      288
   Tyr Thr Thr Gln Arg Asp Leu Arg His Asn Ile Ala Lys Gln Leu Cys
                   85                  90                  95
   cta cat gtc agc aag ggt gct ctg ggc ctt ggg agc tgt cac ttc act      336
   Leu His Val Ser Lys Gly Ala Leu Gly Leu Gly Ser Cys His Phe Thr
               100                 105                 110
   ggc aag aat agc cag gtc ccc aag gac gag gaa tgg gaa ttg gcc cag      384
   Gly Lys Asn Ser Gln Val Pro Lys Asp Glu Glu Trp Glu Leu Ala Gln
           115                 120                 125
   gat cag ctc atc agg aac tca gga tct ggt acc tgc ctg aca tcc cag      432
   Asp Gln Leu Ile Arg Asn Ser Gly Ser Gly Thr Cys Leu Thr Ser Gln
       130                 135                 140
   gac aaa aag cca gcc atg gcc ccc tgc aat ccc agt gac ccc cat cag      480
   Asp Lys Lys Pro Ala Met Ala Pro Cys Asn Pro Ser Asp Pro His Gln
   145                 150                 155                 160
   ttg tgg ctc ttt gtc tag                                              498
   Leu Trp Leu Phe Val
                   165
<210> 12
   <211> 165
   <212> PRT
   <213> Homo sapiens
<400> 12
   Ser Phe Gly Asp Ile Ser Glu Arg Leu Gln Leu Arg Glu Gln Leu His
   1               5                   10                  15
   Cys His Asn Phe Ser Trp Tyr Leu His Asn Val Tyr Pro Glu Met Phe
               20                  25                  30
   Val Pro Asp Leu Thr Pro Thr Phe Tyr Gly Ala Ile Lys Asn Leu Gly
           35                  40                  45
   Thr Asn Gln Cys Leu Asp Val Gly Glu Asn Asn Arg Gly Gly Lys Pro
       50                  55                  60
   Leu Ile Met Tyr Ser Cys His Gly Leu Gly Gly Asn Gln Tyr Phe Glu
   65                  70                  75                  80
   Tyr Thr Thr Gln Arg Asp Leu Arg His Asn Ile Ala Lys Gln Leu Cys
                   85                  90                  95
   Leu His Val Ser Lys Gly Ala Leu Gly Leu Gly Ser Cys His Phe Thr
               100                 105                 110
   Gly Lys Asn Ser Gln Val Pro Lys Asp Glu Glu Trp Glu Leu Ala Gln
           115                 120                 125
   Asp Gln Leu Ile Arg Asn Ser Gly Ser Gly Thr Cys Leu Thr Ser Gln
       130                 135                 140
   Asp Lys Lys Pro Ala Met Ala Pro Cys Asn Pro Ser Asp Pro His Gln
   145                 150                 155                 160
   Leu Trp Leu Phe Val
                   165
<210> 13
   <211> 501
   <212> DNA
   <213> Homo sapiens
<220>
   <221> CDS
   <222> (1)..(501)
   <223> GalNAc-T7 lectin domain
<400> 13
   tat ggg gat ata tcg gag ctg aaa aaa ttt cga gaa gat cac aac tgc      48
   Tyr Gly Asp Ile Ser Glu Leu Lys Lys Phe Arg Glu Asp His Asn Cys
   1               5                   10                  15
   caa agt ttt aag tgg ttc atg gaa gaa ata gct tat gat atc acc tca      96
   Gln Ser Phe Lys Trp Phe Met Glu Glu Ile Ala Tyr Asp Ile Thr Ser
               20                  25                  30
   cac tac cct ttg cca ccc aaa aat gtt gac tgg gga gaa atc aga ggc     144
   His Tyr Pro Leu Pro Pro Lys Asn Val Asp Trp Gly Glu Ile Arg Gly
           35                  40                  45
   ttc gaa act gct tac tgc att gat agc atg gga aaa aca aat gga ggc     192
   Phe Glu Thr Ala Tyr Cys Ile Asp Ser Met Gly Lys Thr Asn Gly Gly
       50                  55                  60
   ttt gtt gaa cta gga ccc tgc cac agg atg gga ggg aat cag ctt ttc     240
   Phe Val Glu Leu Gly Pro Cys His Arg Met Gly Gly Asn Gln Leu Phe
   65                  70                  75                  80
   aga atc aat gaa gca aat caa ctc atg cag tat gac cag tgt ttg aca     288
   Arg Ile Asn Glu Ala Asn Gln Leu Met Gln Tyr Asp Gln Cys Leu Thr
                   85                  90                  95
   aag gga gct gat gga tca aaa gtt atg att aca cac tgt aat cta aat     336
   Lys Gly Ala Asp Gly Ser Lys Val Met Ile Thr His Cys Asn Leu Asn
               100                 105                 110
   gaa ttt aag gaa tgg cag tac ttc aag aac ctg cac aga ttt act cat     384
   Glu Phe Lys Glu Trp Gln Tyr Phe Lys Asn Leu His Arg Phe Thr His
           115                 120                 125
   att cct tca gga aag tgt tta gat cgc tca gag gtc ctg cat caa gta     432
   Ile Pro Ser Gly Lys Cys Leu Asp Arg Ser Glu Val Leu His Gln Val
       130                 135                 140
   ttc atc tcc aat tgt gac tcc agt aaa acg act caa aaa tgg gaa atg     480
   Phe Ile Ser Asn Cys Asp Ser Ser Lys Thr Thr Gln Lys Trp Glu Met
   145                 150                 155                 160
   aat aac atc cat agt gtt tag                                         501
   Asn Asn Ile His Ser Val
                   165
<210> 14
   <211> 166
   <212> PRT
   <213> Homo sapiens
<400> 14
   Tyr Gly Asp Ile Ser Glu Leu Lys Lys Phe Arg Glu Asp His Asn Cys
   1 5 10 15
   Gln Ser Phe Lys Trp Phe Met Glu Glu Ile Ala Tyr Asp Ile Thr Ser
   20 25 30
   His Tyr Pro Leu Pro Pro Lys Asn Val Asp Trp Gly Glu Ile Arg Gly
   35 40 45
   Phe Glu Thr Ala Tyr Cys Ile Asp Ser Met Gly Lys Thr Asn Gly Gly
   50 55 60
   Phe Val Glu Leu Gly Pro Cys His Arg Met Gly Gly Asn Gln Leu Phe
   65 70 75 80
   Arg Ile Asn Glu Ala Asn Gln Leu Met Gln Tyr Asp Gln Cys Leu Thr
   85 90 95
   Lys Gly Ala Asp Gly Ser Lys Val Met Ile Thr His Cys Asn Leu Asn
   100 105 110
   Glu Phe Lys Glu Trp Gln Tyr Phe Lys Asn Leu His Arg Phe Thr His
   115 120 125
   Ile Pro Ser Gly Lys Cys Leu Asp Arg Ser Glu Val Leu His Gln Val
   130 135 140
   Phe Ile Ser Asn Cys Asp Ser Ser Lys Thr Thr Gln Lys Trp Glu Met
   145 150 155 160
   Asn Asn Ile His Ser Val
   165
<210> 15
   <211> 534
   <212> DNA
   <213> Homo sapiens
<220>
   <221> misc_feature
   <223> GalNAc-T8 lectin domain DNA sequence
<400> 15
   gacgtttctt ccagaatggc actccgggaa aaactgaaat gtaaaacttt tgactggtac 60
   ctgaaaaatg tttatccact cttgaagcca ctccacacca tcgtgggcta tggaagaatg 120
   aaaaacctat tggatgaaaa tgtctgcttg gatcagggac ccgttccagg caacaccccc 180
   atcatgtatt actgccatga attcagctca cagaatgtct actatcacct aactggggag 240
   ctctatgtgg gacaactgat tgcagaggcc agtgctagtg atcgctgcct gacagaccct 300
   ggcaaggcgg agaagcccac cttagaacca tgctccaagg cagctaagaa tagactgcat 360
   atatattggg attttaaacc gggaggagct gtcataaaca gagataccaa gcggtgtctg 420
   gagatgaaga aggatctttt gggtagccac gtgcttgtgc tccagacctg tagcacgcaa 480
   gtgtgggaaa tccagcacac tgtcagagac tggggtcaga ccaacagcca gtga 534
<210> 16
   <211> 179
   <212> PRT
   <213> Homo sapiens
<220>
   <221> BINDING
   <222> (1)..(179)
   <223> GalNAc-T8 lectin binding domain protein sequence
<400> 16
   Phe Gly Asp Val Ser Ser Arg Met Ala Leu Arg Glu Lys Leu Lys Cys
   1               5                   10                  15
   Lys Thr Phe Asp Trp Tyr Leu Lys Asn Val Tyr Pro Leu Leu Lys Pro
               20                  25                  30
   Leu His Thr Ile Val Gly Tyr Gly Arg Met Lys Asn Leu Leu Asp Glu
           35                  40                  45
   Asn Val Cys Leu Asp Gln Gly Pro Val Pro Gly Asn Thr Pro Ile Met
       50                  55                  60
   Tyr Tyr Cys His Glu Phe Ser Ser Gln Asn Val Tyr Tyr His Leu Thr
   65                  70                  75                  80
   Gly Glu Leu Tyr Val Gly Gln Leu Ile Ala Glu Ala Ser Ala Ser Asp
                   85                  90                  95
   Arg Cys Leu Thr Asp Pro Gly Lys Ala Glu Lys Pro Thr Leu Glu Pro
               100                 105                 110
   Cys Ser Lys Ala Ala Lys Asn Arg Leu His Ile Tyr Trp Asp Phe Lys
           115                 120                 125
   Pro Gly Gly Ala Val Ile Asn Arg Asp Thr Lys Arg Cys Leu Glu Met
       130                 135                 140
   Lys Lys Asp Leu Leu Gly Ser His Val Leu Val Leu Gln Thr Cys Ser
   145                 150                 155                 160
   Thr Gln Val Trp Glu Ile Gln His Thr Val Arg Asp Trp Gly Gln Thr
                   165                 170                 175
   Asn Ser Gln
<210> 17
   <211> 534
   <212> DNA
   <213> Homo sapiens
<220>
   <221> CDS
   <222> (1)..(534)
   <223> GalNAc-T9 lectin domain
<400> 17
   ttc ggg gac gtg tct gag agg ctg gcc ctg cgt cag agg ctg aag tgt      48
   Phe Gly Asp Val Ser Glu Arg Leu Ala Leu Arg Gln Arg Leu Lys Cys
   1               5                   10                  15
   cgc agc ttc aag tgg tac ctg gag aac gtg tac ccg gag atg agg gtc      96
   Arg Ser Phe Lys Trp Tyr Leu Glu Asn Val Tyr Pro Glu Met Arg Val
               20                  25                  30
   tac aac aac acc ctc acg tac gga gag gtg aga aac agc aaa gcc agt      144
   Tyr Asn Asn Thr Leu Thr Tyr Gly Glu Val Arg Asn Ser Lys Ala Ser
           35                  40                  45
   gcc tac tgt ctg gac cag gga gcg gag gac ggc gac cgg gcg atc ctc      192
   Ala Tyr Cys Leu Asp Gln Gly Ala Glu Asp Gly Asp Arg Ala Ile Leu
       50                  55                  60
   tac ccc tgc cac ggg atg tcc tcc cag ctg gtg cgg tac agc gct gac      240
   Tyr Pro Cys His Gly Met Ser Ser Gln Leu Val Arg Tyr Ser Ala Asp
   65                  70                  75                  80
   ggc ctg ctg cag ctg ggg cct ctg ggc tcc aca gcc ttc ttg cct gac      288
   Gly Leu Leu Gln Leu Gly Pro Leu Gly Ser Thr Ala Phe Leu Pro Asp
                   85                  90                  95
   tcc aag tgt ctg gtg gat gac ggc acg ggc cgc atg ccc acc ctg aag      336
   Ser Lys Cys Leu Val Asp Asp Gly Thr Gly Arg Met Pro Thr Leu Lys
               100                 105                 110
   agg tgt gag gat gtg gcg cgg cca aca cag cgg ctg tgg gac ttc acc      384
   Arg Cys Glu Asp Val Ala Arg Pro Thr Gln Arg Leu Trp Asp Phe Thr
           115                 120                 125
   cag agt ggc ccc att gtg agc cgg gcc acg ggc cgc tgc ctg gag gtg      432
   Gln Ser Gly Pro Ile Val Ser Arg Ala Thr Gly Arg Cys Leu Glu Val
       130                 135                 140
   gag atg tcc aaa gat gcc aac ttt ggg ctc cgg ctg gtg gta cag agg      480
   Glu Met Ser Lys Asp Ala Asn Phe Gly Leu Arg Leu Val Val Gln Arg
   145                 150                 155                 160
   tgc tcg ggg cag aag tgg atg atc aga aac tgg atc aaa cac gca cgg      528
   Cys Ser Gly Gln Lys Trp Met Ile Arg Asn Trp Ile Lys His Ala Arg
                   165                 170                 175
   cac tga                                                              534
   His
<210> 18
   <211> 177
   <212> PRT
   <213> Homo sapiens
<400> 18
   Phe Gly Asp Val Ser Glu Arg Leu Ala Leu Arg Gln Arg Leu Lys Cys
   1               5                   10                  15
   Arg Ser Phe Lys Trp Tyr Leu Glu Asn Val Tyr Pro Glu Met Arg Val
               20                  25                  30
   Tyr Asn Asn Thr Leu Thr Tyr Gly Glu Val Arg Asn Ser Lys Ala Ser
           35                  40                  45
   Ala Tyr Cys Leu Asp Gln Gly Ala Glu Asp Gly Asp Arg Ala Ile Leu
       50                  55                  60
   Tyr Pro Cys His Gly Met Ser Ser Gln Leu Val Arg Tyr Ser Ala Asp
   65                  70                  75                  80
   Gly Leu Leu Gln Leu Gly Pro Leu Gly Ser Thr Ala Phe Leu Pro Asp
                   85                  90                  95
   Ser Lys Cys Leu Val Asp Asp Gly Thr Gly Arg Met Pro Thr Leu Lys
               100                 105                 110
   Arg Cys Glu Asp Val Ala Arg Pro Thr Gln Arg Leu Trp Asp Phe Thr
           115                 120                 125
   Gln Ser Gly Pro Ile Val Ser Arg Ala Thr Gly Arg Cys Leu Glu Val
       130                 135                 140
   Glu Met Ser Lys Asp Ala Asn Phe Gly Leu Arg Leu Val Val Gln Arg
   145                 150                 155                 160
   Cys Ser Gly Gln Lys Trp Met Ile Arg Asn Trp Ile Lys His Ala Arg
                   165                 170                 175
   His
<210> 19
   <211> 564
   <212> DNA
   <213> Homo sapiens
<220>
   <221> CDS
   <222> (1)..(564)
   <223> GalNAc-T10 lectin domain
<400> 19
   gct ggg gat gtc gca gtc cag aaa aag ctc cgc agc tcc ctt aac tgc      48
   Ala Gly Asp Val Ala Val Gln Lys Lys Leu Arg Ser Ser Leu Asn Cys
   1               5                   10                  15
   aag agt ttc aag tgg ttt atg acg aag ata gcc tgg gac ctg ccc aaa      96
   Lys Ser Phe Lys Trp Phe Met Thr Lys Ile Ala Trp Asp Leu Pro Lys
               20                  25                  30
   ttc tac cca ccc gtg gag ccc ccg gct gca gct tgg ggg gag atc cga     144
   Phe Tyr Pro Pro Val Glu Pro Pro Ala Ala Ala Trp Gly Glu Ile Arg
           35                  40                  45
   aat gtg ggc aca ggg ctg tgt gca gac aca aag cac ggg gcc ttg ggc     192
   Asn Val Gly Thr Gly Leu Cys Ala Asp Thr Lys His Gly Ala Leu Gly
       50                  55                  60
   tcc cca cta agg cta gag ggc tgc gtc cga ggc cgt ggg gag gct gcc     240
   Ser Pro Leu Arg Leu Glu Gly Cys Val Arg Gly Arg Gly Glu Ala Ala
   65                  70                  75                  80
   tgg aac aac atg cag gta ttc acc ttc acc tgg aga gag gac atc cgg     288
   Trp Asn Asn Met Gln Val Phe Thr Phe Thr Trp Arg Glu Asp Ile Arg
                   85                  90                  95
   cct gga gac ccc cag cac acc aag aag ttc tgc ttt gat gcc att tcc     336
   Pro Gly Asp Pro Gln His Thr Lys Lys Phe Cys Phe Asp Ala Ile Ser
               100                 105                 110
   cac acc agc cct gtc acg ctg tac gac tgc cac agc atg aag ggc aac     384
   His Thr Ser Pro Val Thr Leu Tyr Asp Cys His Ser Met Lys Gly Asn
           115                 120                 125
   cag ctg tgg aaa tac cgc aaa gac aag acc ctg tac cac cct gtc agt     432
   Gln Leu Trp Lys Tyr Arg Lys Asp Lys Thr Leu Tyr His Pro Val Ser
       130                 135                 140
   ggc agc tgc atg gac tgc agt gaa agt gac cat agg atc ttc atg aac     480
   Gly Ser Cys Met Asp Cys Ser Glu Ser Asp His Arg Ile Phe Met Asn
   145                 150                 155                 160
   acc tgc aac cca tcc tct ctc acc cag cag tgg ctg ttt gaa cac acc     528
   Thr Cys Asn Pro Ser Ser Leu Thr Gln Gln Trp Leu Phe Glu His Thr
                   165                 170                 175
   aac tca aca gtc ttg gaa aaa ttc aat agg aac tga                     564
   Asn Ser Thr Val Leu Glu Lys Phe Asn Arg Asn
               180                 185
<210> 20
   <211> 187
   <212> PRT
   <213> Homo sapiens
<400> 20
   Ala Gly Asp Val Ala Val Gln Lys Lys Leu Arg Ser Ser Leu Asn Cys
   1               5                   10                  15
   Lys Ser Phe Lys Trp Phe Met Thr Lys Ile Ala Trp Asp Leu Pro Lys
               20                  25                  30
   Phe Tyr Pro Pro Val Glu Pro Pro Ala Ala Ala Trp Gly Glu Ile Arg
   35 40 45
   Asn Val Gly Thr Gly Leu Cys Ala Asp Thr Lys His Gly Ala Leu Gly
   50 55 60
   Ser Pro Leu Arg Leu Glu Gly Cys Val Arg Gly Arg Gly Glu Ala Ala
   65 70 75 80
   Trp Asn Asn Met Gln Val Phe Thr Phe Thr Trp Arg Glu Asp Ile Arg
   85 90 95
   Pro Gly Asp Pro Gln His Thr Lys Lys Phe Cys Phe Asp Ala Ile Ser
   100 105 110
   His Thr Ser Pro Val Thr Leu Tyr Asp Cys His Ser Met Lys Gly Asn
   115 120 125
   Gln Leu Trp Lys Tyr Arg Lys Asp Lys Thr Leu Tyr His Pro Val Ser
   130 135 140
   Gly Ser Cys Met Asp Cys Ser Glu Ser Asp His Arg Ile Phe Met Asn
   145 150 155 160
   Thr Cys Asn Pro Ser Ser Leu Thr Gln Gln Trp Leu Phe Glu His Thr
   165 170 175
   Asn Ser Thr Val Leu Glu Lys Phe Asn Arg Asn
   180 185
<210> 21
   <211> 549
   <212> DNA
   <213> Homo sapiens
<220>
   <221> CDS
   <222> (4)..(549)
   <223> GalNAc-T11 lectin domain
<400> 21
   tgc aat atc agt gag cgt gtg gaa ctg aga aag aag ttg ggc tgt aaa      48
       Asn Ile Ser Glu Arg Val Glu Leu Arg Lys Lys Leu Gly Cys Lys
       1               5                   10                  15
   tca ttt aaa tgg tat ttg gat aat gta tac cca gag atg cag ata tct      96
   Ser Phe Lys Trp Tyr Leu Asp Asn Val Tyr Pro Glu Met Gln Ile Ser
                   20                  25                  30
   ggg tcc cac gcc aaa ccc caa caa ccc att ttt gtc aat aga ggg cca     144
   Gly Ser His Ala Lys Pro Gln Gln Pro Ile Phe Val Asn Arg Gly Pro
               35                  40                  45
   aaa cga ccc aaa gtc ctt caa cgt gga agg ctc tat cac ctc cag acc     192
   Lys Arg Pro Lys Val Leu Gln Arg Gly Arg Leu Tyr His Leu Gln Thr
           50                  55                  60
   aac aaa tgc ctg gtg gcc cag ggc cgc cca agt cag aag gga ggt ctc     240
   Asn Lys Cys Leu Val Ala Gln Gly Arg Pro Ser Gln Lys Gly Gly Leu
       65                  70                  75
   gtg gtg ctt aag gcc tgt gac tac agt gac cca aat cag atc tgg atc     288
   Val Val Leu Lys Ala Cys Asp Tyr Ser Asp Pro Asn Gln Ile Trp Ile
   80                  85                  90                  95
   tat aat gaa gag cat gaa ttg gtt tta aat agt ctc ctt tgt cta gat     336
   Tyr Asn Glu Glu His Glu Leu Val Leu Asn Ser Leu Leu Cys Leu Asp
                   100                 105                 110
   atg tca gag act cgc tca tca gac ccg cca cgg ctc atg aaa tgc cac     384
   Met Ser Glu Thr Arg Ser Ser Asp Pro Pro Arg Leu Met Lys Cys His
               115                 120                 125
   ggg tca gga gga tcc cag cag tgg acc ttt ggg aaa aac aat cgg cta     432
   Gly Ser Gly Gly Ser Gln Gln Trp Thr Phe Gly Lys Asn Asn Arg Leu
           130                 135                 140
   tac cag gtg tcg gtt gga cag tgc ctg aga gca gtg gat ccc ctg ggt     480
   Tyr Gln Val Ser Val Gly Gln Cys Leu Arg Ala Val Asp Pro Leu Gly
       145                 150                 155
   cag aag ggc tct gtc gcc atg gcg atc tgc gat ggc tcc tct tca cag     528
   Gln Lys Gly Ser Val Ala Met Ala Ile Cys Asp Gly Ser Ser Ser Gln
   160                 165                 170                 175
   cag tgg cat ttg gaa ggt taa                                         549
   Gln Trp His Leu Glu Gly
                   180
<210> 22
   <211> 181
   <212> PRT
   <213> Homo sapiens
<400> 22
   Asn Ile Ser Glu Arg Val Glu Leu Arg Lys Lys Leu Gly Cys Lys Ser
   1               5                   10                  15
   Phe Lys Trp Tyr Leu Asp Asn Val Tyr Pro Glu Met Gln Ile Ser Gly
               20                  25                  30
   Ser His Ala Lys Pro Gln Gln Pro Ile Phe Val Asn Arg Gly Pro Lys
           35                  40                  45
   Arg Pro Lys Val Leu Gln Arg Gly Arg Leu Tyr His Leu Gln Thr Asn
       50                  55                  60
   Lys Cys Leu Val Ala Gln Gly Arg Pro Ser Gln Lys Gly Gly Leu Val
   65                  70                  75                  80
   Val Leu Lys Ala Cys Asp Tyr Ser Asp Pro Asn Gln Ile Trp Ile Tyr
                   85                  90                  95
   Asn Glu Glu His Glu Leu Val Leu Asn Ser Leu Leu Cys Leu Asp Met
               100                 105                 110
   Ser Glu Thr Arg Ser Ser Asp Pro Pro Arg Leu Met Lys Cys His Gly
           115                 120                 125
   Ser Gly Gly Ser Gln Gln Trp Thr Phe Gly Lys Asn Asn Arg Leu Tyr
       130                 135                 140
   Gln Val Ser Val Gly Gln Cys Leu Arg Ala Val Asp Pro Leu Gly Gln
   145                 150                 155                 160
   Lys Gly Ser Val Ala Met Ala Ile Cys Asp Gly Ser Ser Ser Gln Gln
                   165                 170                 175
   Trp His Leu Glu Gly
               180
<210> 23
   <211> 525
   <212> DNA
   <213> Homo sapiens
<220>
   <221> CDS
   <222> (4)..(525)
   <223> GalNAc-T12 lectin domain
<400> 23
   tgg gat gtg aca gag agg aag cag ctc cgg gac aag ctc cag tgt aaa      48
       Asp Val Thr Glu Arg Lys Gln Leu Arg Asp Lys Leu Gln Cys Lys
       1               5                   10                  15
   gac ttc aag tgg ttc ttg gag act gtg tat cca gaa ctg cat gtg cct      96
   Asp Phe Lys Trp Phe Leu Glu Thr Val Tyr Pro Glu Leu His Val Pro
                   20                  25                  30
   gag gac agg cct ggc ttc ttc ggg atg ctc cag aac aaa gga cta aca     144
   Glu Asp Arg Pro Gly Phe Phe Gly Met Leu Gln Asn Lys Gly Leu Thr
               35                  40                  45
   gac tac tgc ttt gac tat aac cct ccc gat gaa aac cag att gtg gga     192
   Asp Tyr Cys Phe Asp Tyr Asn Pro Pro Asp Glu Asn Gln Ile Val Gly
           50                  55                  60
   cac cag gtc att ctg tac ctc tgt cat ggg atg ggc cag aat cag ttt     240
   His Gln Val Ile Leu Tyr Leu Cys His Gly Met Gly Gln Asn Gln Phe
       65                  70                  75
   ttc gag tac acg tcc cag aaa gaa ata cgc tat aac acc cac cag cct     288
   Phe Glu Tyr Thr Ser Gln Lys Glu Ile Arg Tyr Asn Thr His Gln Pro
   80                  85                  90                  95
   gag ggc tgc att gct gtg gaa gca gga atg gat acc ctt atc atg cat     336
   Glu Gly Cys Ile Ala Val Glu Ala Gly Met Asp Thr Leu Ile Met His
                   100                 105                 110
   ctc tgc gaa gaa act gcc cca gag aat cag aag ttc atc ttg cag gag     384
   Leu Cys Glu Glu Thr Ala Pro Glu Asn Gln Lys Phe Ile Leu Gln Glu
               115                 120                 125
   gat gga tct tta ttt cac gaa cag tcc aag aaa tgt gtc cag gct gcg     432
   Asp Gly Ser Leu Phe His Glu Gln Ser Lys Lys Cys Val Gln Ala Ala
           130                 135                 140
   agg aag gag tcg agt gac agt ttc gtt cca ctc tta cga gac tgc acc     480
   Arg Lys Glu Ser Ser Asp Ser Phe Val Pro Leu Leu Arg Asp Cys Thr
       145                 150                 155
   aac tcg gat cat cag aaa tgg ttc ttc aaa gag cgc atg tta tga         525
   Asn Ser Asp His Gln Lys Trp Phe Phe Lys Glu Arg Met Leu
   160                 165                 170
<210> 24
   <211> 173
   <212> PRT
   <213> Homo sapiens
<400> 24
   Asp Val Thr Glu Arg Lys Gln Leu Arg Asp Lys Leu Gln Cys Lys Asp
   1               5                   10                  15
   Phe Lys Trp Phe Leu Glu Thr Val Tyr Pro Glu Leu His Val Pro Glu
               20                  25                  30
   Asp Arg Pro Gly Phe Phe Gly Met Leu Gln Asn Lys Gly Leu Thr Asp
           35                  40                  45
   Tyr Cys Phe Asp Tyr Asn Pro Pro Asp Glu Asn Gln Ile Val Gly His
       50                  55                  60
   Gln Val Ile Leu Tyr Leu Cys His Gly Met Gly Gln Asn Gln Phe Phe
   65                  70                  75                  80
   Glu Tyr Thr Ser Gln Lys Glu Ile Arg Tyr Asn Thr His Gln Pro Glu
                   85                  90                  95
   Gly Cys Ile Ala Val Glu Ala Gly Met Asp Thr Leu Ile Met His Leu
               100                 105                 110
   Cys Glu Glu Thr Ala Pro Glu Asn Gln Lys Phe Ile Leu Gln Glu Asp
           115                 120                 125
   Gly Ser Leu Phe His Glu Gln Ser Lys Lys Cys Val Gln Ala Ala Arg
       130                 135                 140
   Lys Glu Ser Ser Asp Ser Phe Val Pro Leu Leu Arg Asp Cys Thr Asn
   145                 150                 155                 160
   Ser Asp His Gln Lys Trp Phe Phe Lys Glu Arg Met Leu
                   165                 170
<210> 25
   <211> 528
   <212> DNA
   <213> Homo sapiens
<220>
   <221> CDS
   <222> (4)..(528)
   <223> GalNAc-T13 lectin domain
<400> 25
   tct gag aag cca gac tgc atg gaa cgc ttg cag ctg caa agg aga ctg          48
       Glu Lys Pro Asp Cys Met Glu Arg Leu Gln Leu Gln Arg Arg Leu
       1               5                   10                  15
   ggt tgt cgg aca ttc cac tgg ttt ctg gct aat gtc tac cct gag ctg          96
   Gly Cys Arg Thr Phe His Trp Phe Leu Ala Asn Val Tyr Pro Glu Leu
                   20                  25                  30
   tac cca tct gaa ccc agg ccc agt ttc tct gga aag ctc cac aac act         144
   Tyr Pro Ser Glu Pro Arg Pro Ser Phe Ser Gly Lys Leu His Asn Thr
               35                  40                  45
   gga ctt ggg ctc tgt gca gac tgc cag gca gaa ggg gac atc ctg ggc         192
   Gly Leu Gly Leu Cys Ala Asp Cys Gln Ala Glu Gly Asp Ile Leu Gly
           50                  55                  60
   tgt ccc atg gtg ttg gct cct tgc agt gac agc cgg cag caa cag tac         240
   Cys Pro Met Val Leu Ala Pro Cys Ser Asp Ser Arg Gln Gln Gln Tyr
       65                  70                  75
   ctg cag cac acc agc agg aag gag att cac ttt ggc agc cca cag cac         288
   Leu Gln His Thr Ser Arg Lys Glu Ile His Phe Gly Ser Pro Gln His
   80                  85                  90                  95
   ctg tgc ttt gct gtc agg cag gag cag gtg att ctt cag aac tgc acg         336
   Leu Cys Phe Ala Val Arg Gln Glu Gln Val Ile Leu Gln Asn Cys Thr
                   100                 105                 110
   gag gaa ggc ctg gcc atc cac cag cag cac tgg gac ttc cag gag aat         384
   Glu Glu Gly Leu Ala Ile His Gln Gln His Trp Asp Phe Gln Glu Asn
               115                 120                 125
   ggg atg att gtc cac att ctt tct ggg aaa tgc atg gaa gct gtg gtg         432
   Gly Met Ile Val His Ile Leu Ser Gly Lys Cys Met Glu Ala Val Val
           130                 135                 140
   caa gaa aac aat aaa gat ttg tac ctg cgt ccg tgt gat gga aaa gcc         480
   Gln Glu Asn Asn Lys Asp Leu Tyr Leu Arg Pro Cys Asp Gly Lys Ala
       145                 150                 155
   cgc cag cag tgg cgt ttt gac cag atc aat gct gtg gat gaa cga tga         528
   Arg Gln Gln Trp Arg Phe Asp Gln Ile Asn Ala Val Asp Glu Arg
   160                 165                 170
<210> 26
   <211> 174
   <212> PRT
   <213> Homo sapiens
<400> 26
   Glu Lys Pro Asp Cys Met Glu Arg Leu Gln Leu Gln Arg Arg Leu Gly
   1               5                   10                  15
   Cys Arg Thr Phe His Trp Phe Leu Ala Asn Val Tyr Pro Glu Leu Tyr
               20                  25                  30
   Pro Ser Glu Pro Arg Pro Ser Phe Ser Gly Lys Leu His Asn Thr Gly
           35                  40                  45
   Leu Gly Leu Cys Ala Asp Cys Gln Ala Glu Gly Asp Ile Leu Gly Cys
   50 55 60
   Pro Met Val Leu Ala Pro Cys Ser Asp Ser Arg Gln Gln Gln Tyr Leu
   65 70 75 80
   Gln His Thr Ser Arg Lys Glu Ile His Phe Gly Ser Pro Gln His Leu
   85 90 95
   Cys Phe Ala Val Arg Gln Glu Gln Val Ile Leu Gln Asn Cys Thr Glu
   100 105 110
   Glu Gly Leu Ala Ile His Gln Gln His Trp Asp Phe Gln Glu Asn Gly
   115 120 125
   Met Ile Val His Ile Leu Ser Gly Lys Cys Met Glu Ala Val Val Gln
   130 135 140
   Glu Asn Asn Lys Asp Leu Tyr Leu Arg Pro Cys Asp Gly Lys Ala Arg
   145 150 155 160
   Gln Gln Trp Arg Phe Asp Gln Ile Asn Ala Val Asp Glu Arg
   165 170
<210> 27
   <211> 498
   <212> DNA
   <213> Homo sapiens
<220>
   <221> CDS
   <222> (1)..(498)
   <223> GalNAc-T14 lectin domain
<400> 27
   tat gga gat gtg tca gtc aga aaa aca cta aga gaa aat ctg aag tgt       48
   Tyr Gly Asp Val Ser Val Arg Lys Thr Leu Arg Glu Asn Leu Lys Cys
   1               5                   10                  15
   aag ccc ttt tct tgg tac cta gaa aac atc tat ccg gac tcc cag atc       96
   Lys Pro Phe Ser Trp Tyr Leu Glu Asn Ile Tyr Pro Asp Ser Gln Ile
               20                  25                  30
   cca aga cgt tat tac tca ctt ggt gag ata aga aat gtt gaa acc aat      144
   Pro Arg Arg Tyr Tyr Ser Leu Gly Glu Ile Arg Asn Val Glu Thr Asn
           35                  40                  45
   cag tgt tta gac aac atg ggc cgc aag gaa aat gaa aaa gtg ggt ata      192
   Gln Cys Leu Asp Asn Met Gly Arg Lys Glu Asn Glu Lys Val Gly Ile
       50                  55                  60
   ttc aac tgt cat ggt atg gga gga aat cag gta ttt tct tac act gct      240
   Phe Asn Cys His Gly Met Gly Gly Asn Gln Val Phe Ser Tyr Thr Ala
   65                  70                  75                  80
   gac aaa gaa atc cga acc gat gac ttg tgc ttg gat gtt tct aga ctc      288
   Asp Lys Glu Ile Arg Thr Asp Asp Leu Cys Leu Asp Val Ser Arg Leu
                   85                  90                  95
   aat gga cct gta atc atg tta aaa tgc cac cat atg aga gga aat cag      336
   Asn Gly Pro Val Ile Met Leu Lys Cys His His Met Arg Gly Asn Gln
               100                 105                 110
   tta tgg gaa tat gat gct gag aga ctc acg ttg cga cat gtt aac agt      384
   Leu Trp Glu Tyr Asp Ala Glu Arg Leu Thr Leu Arg His Val Asn Ser
           115                 120                 125
   aac caa tgt ctc gat gaa cct tct gaa gaa gac aaa atg gtg cct aca      432
   Asn Gln Cys Leu Asp Glu Pro Ser Glu Glu Asp Lys Met Val Pro Thr
       130                 135                 140
   atg cag gac tgt agt gga agc aga tcc caa cag tgg ctg cta agg aac      480
   Met Gln Asp Cys Ser Gly Ser Arg Ser Gln Gln Trp Leu Leu Arg Asn
   145                 150                 155                 160
   atg acc ttg ggc aca tga                                              498
   Met Thr Leu Gly Thr
                   165
<210> 28
   <211> 165
   <212> PRT
   <213> Homo sapiens
<400> 28
   Tyr Gly Asp Val Ser Val Arg Lys Thr Leu Arg Glu Asn Leu Lys Cys
   1               5                   10                  15
   Lys Pro Phe Ser Trp Tyr Leu Glu Asn Ile Tyr Pro Asp Ser Gln Ile
               20                  25                  30
   Pro Arg Arg Tyr Tyr Ser Leu Gly Glu Ile Arg Asn Val Glu Thr Asn
           35                  40                  45
   Gln Cys Leu Asp Asn Met Gly Arg Lys Glu Asn Glu Lys Val Gly Ile
       50                  55                  60
   Phe Asn Cys His Gly Met Gly Gly Asn Gln Val Phe Ser Tyr Thr Ala
   65                  70                  75                  80
   Asp Lys Glu Ile Arg Thr Asp Asp Leu Cys Leu Asp Val Ser Arg Leu
                   85                  90                  95
   Asn Gly Pro Val Ile Met Leu Lys Cys His His Met Arg Gly Asn Gln
               100                 105                 110
   Leu Trp Glu Tyr Asp Ala Glu Arg Leu Thr Leu Arg His Val Asn Ser
           115                 120                 125
   Asn Gln Cys Leu Asp Glu Pro Ser Glu Glu Asp Lys Met Val Pro Thr
       130                 135                 140
   Met Gln Asp Cys Ser Gly Ser Arg Ser Gln Gln Trp Leu Leu Arg Asn
   145                 150                 155                 160
   Met Thr Leu Gly Thr
               165
<210> 29
   <211> 516
   <212> DNA
   <213> Homo sapiens
<220>
   <221> misc_feature
   <223> GalNAc-T15 lectin domain DNA sequence
<400> 29
   tcgggaatgt tgagagcaga ttggacctga ggaagaatct gcgctgccag agcttcaagt     60
   ggtacctgga gaatatctac cctgaactca gcatccccaa ggagtcctcc atccagaagg    120
   gcaatatccg acagagacag aagtgcctgg aatctcaaag gcagaacaac caagaaaccc    180
   caaacctaaa gttgagcccc tgtgccaagg tcaaaggcga agatgcaaag tcccaggtat    240
   gggccttcac atacacccag aagatcctcc aggaggagct gtgcctgtca gtcatcacct    300
   tgttccctgg cgccccagtg gttcttgtcc tttgcaagaa tggagatgac cgacagcaat    360
   ggaccaaaac tggttcccac atcgagcaca tagcatccca cctctgcctc gatacagata    420
   tgttcggtga tggcaccgag aacggcaagg aaatcggcgt caacccatgt gagtcctcac    480
   tcatgagcca gcactgggac atggtgagtt cttgag                              516
<210> 30
   <211> 171
   <212> PRT
   <213> Homo sapiens
<220>
   <221> BINDING
   <222> (1)..(171)
   <223> GalNAC-T15 lectin domain protein sequence
<400> 30
   Phe Gly Asn Val Glu Ser Arg Leu Asp Leu Arg Lys Asn Leu Arg Cys
   1               5                   10                  15
   Gln Ser Phe Lys Trp Tyr Leu Glu Asn Ile Tyr Pro Glu Leu Ser Ile
               20                  25                  30
   Pro Lys Glu Ser Ser Ile Gln Lys Gly Asn Ile Arg Gln Arg Gln Lys
           35                  40                  45
   Cys Leu Glu Ser Gln Arg Gln Asn Asn Gln Glu Thr Pro Asn Leu Lys
       50                  55                  60
   Leu Ser Pro Cys Ala Lys Val Lys Gly Glu Asp Ala Lys Ser Gln Val
   65                  70                  75                  80
   Trp Ala Phe Thr Tyr Thr Gln Lys Ile Leu Gln Glu Glu Leu Cys Leu
                   85                  90                  95
   Ser Val Ile Thr Leu Phe Pro Gly Ala Pro Val Val Leu Val Leu Cys
               100                 105                 110
   Lys Asn Gly Asp Asp Arg Gln Gln Trp Thr Lys Thr Gly Ser His Ile
           115                 120                 125
   Glu His Ile Ala Ser His Leu Cys Leu Asp Thr Asp Met Phe Gly Asp
       130                 135                 140
   Gly Thr Glu Asn Gly Lys Glu Ile Gly Val Asn Pro Cys Glu Ser Ser
   145                 150                 155                 160
   Leu Met Ser Gln His Trp Asp Met Val Ser Ser
                   165                 170
<210> 31
   <211> 492
   <212> DNA
   <213> Homo sapiens
<220>
   <221> CDS
   <222> (1)..(492)
   <223> GalNAc-T16 lectin domain
<400> 31
   agt gtg gct acg cgg ata gag cag agg aag aag atg aac tgc aag tcc      48
   Ser Val Ala Thr Arg Ile Glu Gln Arg Lys Lys Met Asn Cys Lys Ser
   1               5                   10                  15
   ttc cgc tgg tac ctg gag aac gtc tac cca gag ctc acg gtc ccc gtg      96
   Phe Arg Trp Tyr Leu Glu Asn Val Tyr Pro Glu Leu Thr Val Pro Val
               20                  25                  30
   aag gaa gca ctc ccc ggc atc att aag cag ggg gtg aac tgc tta gaa     144
   Lys Glu Ala Leu Pro Gly Ile Ile Lys Gln Gly Val Asn Cys Leu Glu
           35                  40                  45
   tct cag ggc cag aac aca gct ggt gac ttc ctg ctt gga atg ggg atc     192
   Ser Gln Gly Gln Asn Thr Ala Gly Asp Phe Leu Leu Gly Met Gly Ile
       50                  55                  60
   tgc aga ggg tct gcc aag aac ccg cag ccc gcc cag gca tgg ctg ttc     240
   Cys Arg Gly Ser Ala Lys Asn Pro Gln Pro Ala Gln Ala Trp Leu Phe
   65                  70                  75                  80
   agt gac cac ctc atc cag cag cag ggg aag tgc ctg gct gcc acc tcc     288
   Ser Asp His Leu Ile Gln Gln Gln Gly Lys Cys Leu Ala Ala Thr Ser
                   85                  90                  95
   acc tta atg tcc tcc cct gga tcc cca gtc ata ctg cag atg tgc aac     336
   Thr Leu Met Ser Ser Pro Gly Ser Pro Val Ile Leu Gln Met Cys Asn
               100                 105                 110
   cct aga gaa ggc aag cag aaa tgg agg aga aaa gga tct ttc atc cag     384
   Pro Arg Glu Gly Lys Gln Lys Trp Arg Arg Lys Gly Ser Phe Ile Gln
           115                 120                 125
   cat tca gtc agt ggc ctc tgc ctg gag aca aag cct gcc cag ctg gtg     432
   His Ser Val Ser Gly Leu Cys Leu Glu Thr Lys Pro Ala Gln Leu Val
       130                 135                 140
   acc agc aag tgt cag gct gac gcc cag gcc cag cag tgg cag ctg ttg     480
   Thr Ser Lys Cys Gln Ala Asp Ala Gln Ala Gln Gln Trp Gln Leu Leu
   145                 150                 155                 160
   cca cac aca tga                                                     492
   Pro His Thr
<210> 32
   <211> 163
   <212> PRT
   <213> Homo sapiens
<400> 32
   Ser Val Ala Thr Arg Ile Glu Gln Arg Lys Lys Met Asn Cys Lys Ser
   1               5                   10                  15
   Phe Arg Trp Tyr Leu Glu Asn Val Tyr Pro Glu Leu Thr Val Pro Val
               20                  25                  30
   Lys Glu Ala Leu Pro Gly Ile Ile Lys Gln Gly Val Asn Cys Leu Glu
           35                  40                  45
   Ser Gln Gly Gln Asn Thr Ala Gly Asp Phe Leu Leu Gly Met Gly Ile
       50                  55                  60
   Cys Arg Gly Ser Ala Lys Asn Pro Gln Pro Ala Gln Ala Trp Leu Phe
   65                  70                  75                  80
   Ser Asp His Leu Ile Gln Gln Gln Gly Lys Cys Leu Ala Ala Thr Ser
                   85                  90                  95
   Thr Leu Met Ser Ser Pro Gly Ser Pro Val Ile Leu Gln Met Cys Asn
               100                 105                 110
   Pro Arg Glu Gly Lys Gln Lys Trp Arg Arg Lys Gly Ser Phe Ile Gln
           115                 120                 125
   His Ser Val Ser Gly Leu Cys Leu Glu Thr Lys Pro Ala Gln Leu Val
       130                 135                 140
   Thr Ser Lys Cys Gln Ala Asp Ala Gln Ala Gln Gln Trp Gln Leu Leu
   145                 150                 155                 160
   Pro His Thr
<210> 33
   <211> 33
   <212> DNA
   <213> Homo sapiens
<220>
   <221> misc_binding
   <222> (1)..(33)
   <223> Gal-NAC-T1 lectin domain forward primer
<400> 33
   caaaggaagc ttatggagat atatcgtcaa gag 33
<210> 34
   <211> 43
   <212> DNA
   <213> Homo sapiens
<220>
   <221> misc_binding
   <222> (1)..(43)
   <223> GalNAc-T1 lectin domain reverse primer
<400> 34
   gcaagctcga ggcggccgct cagaatattt ctggaagggt gac 43
<210> 35
   <211> 37
   <212> DNA
   <213> Homo sapiens
<220>
   <221> misc_binding
   <222> (1)..(37)
   <223> GalNAc-T2 lectin domain forward primer
<400> 35
   caaggaagct tcttatggaa atattcagag cagattg 37
<210> 36
   <211> 38
   <212> DNA
   <213> Homo sapiens
<220>
   <221> misc_binding
   <222> (1)..(38)
   <223> GalNAc-T2 lectin domain reverse primer
<400> 36
   gcaagctcga ggcggccgcc tactgctgca ggttgagc 38
<210> 37
   <211> 37
   <212> DNA
   <213> Homo sapiens
<220>
   <221> misc_binding
   <222> (1)..(37)
   <223> GalNAc-T3 lectin domain forward primer
<400> 37
   caaggaagct tcatttggtg atctttcaaa aagattt 37
<210> 38
   <211> 40
   <212> DNA
   <213> Homo sapiens
<220>
   <221> misc_binding
   <222> (1)..(40)
   <223> GalNAc-T3 lectin domain reverse primer
<400> 38
   gcaagctcga ggcggccgca ggaacactta atcattttgg 40
<210> 39
   <211> 28
   <212> DNA
   <213> Homo sapiens
<220>
   <221> misc_binding
   <222> (1)..(28)
   <223> GalNAc-T4 lectin domain forward primer
<400> 39
   agaaaagaag cttatggtga tatttctg 28
<210> 40
   <211> 28
   <212> DNA
   <213> Homo sapiens
<220>
   <221> misc_binding
   <222> (1)..(28)
   <223> GalNAc-T4 lectin domain reverse primer(EBHC307)
<400> 40
   agcggatccg acgaagtgct gttgtgct 28
<210> 41
   <211> 35
   <212> DNA
   <213> Homo sapiens
<220>
   <221> misc_binding
   <222> (1)..(35)
   <223> GalNAc-T5 lectin domain forward primer
<400> 41
   caaggaagct ttagatgttg gcaacctcac ccagc 35
<210> 42
   <211> 44
   <212> DNA
   <213> Homo sapiens
<220>
   <221> misc_binding
   <222> (1)..(44)
   <223> GalNAC-T5 lectin domain reverse primer
<400> 42
   gcaagctcga ggcggccgca agcatcagtt acacttcagg cttc 44
<210> 43
   <211> 33
   <212> DNA
   <213> Homo sapiens
<220>
   <221> misc_binding
   <222> (1)..(33)
   <223> GalNAC-T6 lectin domain forward primer
<400> 43
   caaggaagct tccttcggtg acatttcgga acg 33
<210> 44
   <211> 39
   <212> DNA
   <213> Homo sapiens
<220>
   <221> misc_binding
   <222> (1)..(39)
   <223> GalNAc-T6 lectin domain reverse primer
<400> 44
   gcaagctcga ggcggccgct gggtcctaga caaagagcc 39
<210> 45
   <211> 33
   <212> DNA
   <213> Homo sapiens
<220>
   <221> misc_binding
   <222> (1)..(33)
   <223> GalNAc-T7 lectin forward primer
<400> 45
   agaaaagaag cttatgggga tatatcggag ctg 33
<210> 46
   <211> 44
   <212> DNA
   <213> Homo sapiens
<220>
   <221> misc_binding
   <222> (1)..(44)
   <223> GalNAc-T7 lectin domain reverse primer
<400> 46
   gcaagctcga ggcggccgct ctctaaacac tatggatgtt attc 44
<210> 47
   <211> 34
   <212> DNA
   <213> Homo sapiens
<220>
   <221> misc_binding
   <222> (1)..(34)
   <223> GalNAc-T8 lectin domain forward primer
<400> 47
   caaggaagct tttggagacg tttcttccag aatg 34
<210> 48
   <211> 42
   <212> DNA
   <213> Homo sapiens
<220>
   <221> misc_binding
   <222> (1)..(42)
   <223> GalNAc-T8 lectin domain reverse primer
<400> 48
   gcaagctcga ggcggccgct cactggctgt tggtctgacc cc 42
<210> 49
   <211> 34
   <212> DNA
   <213> Homo sapiens
<220>
   <221> misc_binding
   <222> (1)..(34)
   <223> GalNAc-T9 lectin domain forward primer
<400> 49
   caaggaagct ttcggggacg tgtctgagag gctg 34
<210> 50
   <211> 42
   <212> DNA
   <213> Homo sapiens
<220>
   <221> misc_binding
   <222> (1)..(42)
   <223> GalNAC-T9 lectin domain reverse primer
<400> 50
   gcaagctcga ggcggccgct cagtgccgtg cgtgtttgat cc 42
<210> 51
   <211> 34
   <212> DNA.
   <213> Homo sapiens
<220>
   <221> misc_binding
   <222> (1)..(34)
   <223> GalNAC-T10 lectin domain forward primer
<400> 51
   caaggaagct tccgctgggg atgtcgcagt ccag 34
<210> 52
   <211> 41
   <212> DNA
   <213> Homo sapiens
<220>
   <221> misc_binding
   <222> (1)..(41)
   <223> GalNAC-T10 lectin domain reverse primer
<400> 52
   gcaagctcga ggcggccgct cagttcctat tgaatttttc c 41
<210> 53
   <211> 32
   <212> DNA
   <213> Homo sapiens
<220>
   <221> misc_binding
   <222> (1)..(32)
   <223> GalNAC-T11 lectin domain forward primer
<400> 53
   caaggaagct tgcaatatca gtgagcgtgt gg 32
<210> 54
   <211> 40
   <212> DNA
   <213> Homo sapiens
<220>
   <221> misc_binding
   <222> (1)..(40)
   <223> GalNAC-T11 lectin domain reverse primer
<400> 54
   gcaagctcga ggcggccgcc caccttaacc ttccaaatgc 40
<210> 55
   <211> 31
   <212> DNA
   <213> Homo sapiens
<220>
   <221> misc_binding
   <222> (1)..(31)
   <223> GalNAc-T12 lectin domain forward primer
<400> 55
   caaggaagct tgggatgtga cagagaggaa g 31
<210> 56
   <211> 45
   <212> DNA
   <213> Homo sapiens
<220>
   <221> misc_binding
   <222> (1)..(45)
   <223> GalNAc-T12 lectin domain reverse primer
<400> 56
   gcaagctcga ggcggccgct cataacatgc gctctttgaa gaacc 45
<210> 57
   <211> 32
   <212> DNA
   <213> Homo sapiens
<220>
   <221> misc_binding
   <222> (1)..(32)
   <223> GalNAC-T13 lectin domain forward primer
<400> 57
   caaggaagct tctgagaagc cagactgcat gg 32
<210> 58
   <211> 41
   <212> DNA
   <213> Homo sapiens
<220>
   <221> misc_binding
   <222> (1)..(41)
   <223> GalNAc-T13 lectin domain reverse primer
<400> 58
   gcaagctcga ggcggccgct catcgttcat ccacagcatt g 41
<210> 59
   <211> 36
   <212> DNA
   <213> Homo sapiens
<220>
   <221> misc_binding
   <222> (1)..(36)
   <223> GalNAc-T14 lectin domain forward primer
<400> 59
   caaggaagct tatggagatg tgtcagtcag aaaaac 36
<210> 60
   <211> 42
   <212> DNA
   <213> Homo sapiens
<220>
   <221> misc_binding
   <222> (1)..(42)
   <223> GalNAC-T14 lectin domain reverse primer
<400> 60
   gcaagctcga ggcggccgct catgtgccca aggtcatgtt cc 42
<210> 61
   <211> 34
   <212> DNA
   <213> Homo sapiens
<220>
   <221> misc_binding
   <222> (1)..(34)
   <223> GalNAc-T15 lectin domain forward primer
<400> 61
   caaggaagct ttcgggaatg ttgagagcag attg 34
<210> 62
   <211> 43
   <212> DNA
   <213> Homo sapiens
<220>
   <221> misc_binding
   <222> (I)..(43)
   <223> GalNAc-T15 lectin domain reverse primer
<400> 62
   gcaagctcga ggcggccgct caagaactca ccatgtccca gtg 43
<210> 63
   <211> 40
   <212> DNA
   <213> Homo sapiens
<220>
   <221> misc_binding
   <222> (1)..(40)
   <223> GalNAC-T16 lectin domain forward primer
<400> 63
   caaggaagct tgcagtgtgg ctacgcggat agagcagagg 40
<210> 64
   <211> 42
   <212> DNA
   <213> Homo sapiens
<220>
   <221> misc_binding
   <222> (1)..(42)
   <223> GalNAc-T16 lectin domain reverse primer
<400> 64
   gcaagctcga ggcggccgct catgtgtgtg gcaacagctg cc 42
<210> 65
   <211> 1746
   <212> DNA
   <213> Homo sapiens
<220>
   <221> CDS
   <222> (1)..(1746)
   <223> Human GalNAc-T12 DNA sequence
<400> 65
   atg tgg ggg cgc acg gcg cgg cgg cgc tgc ccg cgg gaa ctg cgg cgc    48
   ggc cgg gag gcg ctg ttg gtg ctc ctg gcg cta ctg gcg ttg gcc ggg    96
   ctg ggc tcg gtg ctg cgg gcg cag cgt ggg gcc ggg gcc ggg gct gcc    144
   gag ccg gga ccc ccg cgc acc ccg cgc ccc ggg cgg cgc gag ccg gtc    192
   atg ccg cgg ccg ccg gtg ccg gcg aac gcg ctg ggc gcg cgg ggc gag    240
   gcg gtg cgg ctg cag ctg cag ggc gag gag ctg cgg ctg cag gag gag    288
   agc gtg cgg ctg cac cag att aac atc tac ctc agc gac cgc atc tca    336
   ctg cac cgc cgc ctg ccc gag cgc tgg aac ccg ctg tgc aaa gag aag    384
   aaa tat gat tat gat aat ttg ccc agg aca tct gtt atc ata gca ttt    432
   tat aat gaa gcc tgg tca act ctc ctt cgg aca gtt tac agt gtc ctt    480
   gag aca tcc ccg gat atc ctg cta gaa gaa gtg atc ctt gta gat gac    528
   tac agt gat aga gag cac ctg aag gag cgc ttg gcc aat gag ctt tcg    576
   gga ctg ccc aag gtg cgc ctg atc cgc gcc aac aag aga gag ggc ctg    624
   gtg cga gcc cgg ctg ctg ggg gcg tct gcg gcg agg ggc gat gtt ctg    672
   acc ttc ctg gac tgt cac tgt gaa tgc cac gaa ggg tgg ctg gag ccg    720
   ctg ctg cag agg atc cat gaa gag gag tcg gca gtg gtg tgc ccg gtg      768
   att gat gtg atc gac tgg aac acc ttc gaa tac ctg ggg aac tcc ggg      816
   gag ccc cag atc ggc ggt ttc gac tgg agg ctg gtg ttc acg tgg cac      864
   aca gtt cct gag agg gag agg ata cgg atg caa tcc ccc gtc gat gtc      912
   atc agg tct cca aca atg gct ggt ggg ctg ttt gct gtg agt aag aaa      960
   tat ttt gaa tat ctg ggg tct tat gat aca gga atg gaa gtt tgg gga     1008
   gga gaa aac ctc gaa ttt tcc ttt agg atc tgg cag tgt ggt ggg gtt     1056
   ctg gaa aca cac cca tgt tcc cat gtt ggc cat gtt ttc ccc aag caa     1104
   gct ccc tac tcc cgc aac aag gct ctg gcc aac agt gtt cgt gca gct     1152
   gaa gta tgg atg gat gaa ttt aaa gag ctc tac tac cat cgc aac ccc     1200
   cgt gcc cgc ttg gaa cct ttt ggg gat gtg aca gag agg aag cag ctc     1248
   cgg gac aag ctc cag tgt aaa gac ttc aag tgg ttc ttg gag act gtg     1296
   tat cca gaa ctg cat gtg cct gag gac agg cct ggc ttc ttc ggg atg     1344
   ctc cag aac aaa gga cta aca gac tac tgc ttt gac tat aac cct ccc     1392
   gat gaa aac cag att gtg gga cac cag gtc att ctg tac ctc tgt cat     1440
   ggg atg ggc cag aat cag ttt ttc gag tac acg tcc cag aaa gaa ata     1488
   cgc tat aac acc cac cag cct gag ggc tgc att gct gtg gaa gca gga     1536
   atg gat acc ctt atc atg cat ctc tgc gaa gaa act gcc cca gag aat      1584
   cag aag ttc atc ttg cag gag gat gga tct tta ttt cac gaa cag tcc      1632
   aag aaa tgt gtc cag gct gcg agg aag gag tcg agt gac agt ttc gtt      1680
   cca ctc tta cga gac tgc acc aac tcg gat cat cag aaa tgg ttc ttc      1728
   aaa gag cgc atg tta tga                                              1746
<210> 66
   <211> 581
   <212> PRT
   <213> Homo sapiens
<400> 66
<210> 67
   <211> 1920
   <212> DNA
   <213> Homo sapiens
<220>
   <221> CDS
   <222> (1)..(1920)
   <223> Human GalNAc-T13 DNA sequence
<400> 67
   atg ctc cta agg aag cga tac agg cac aga cca tgc aga ctc cag ttc      48
   Met Leu Leu Arg Lys Arg Tyr Arg His Arg Pro Cys Arg Leu Gln Phe
   1               5                   10                  15
   ctc ctg ctg ctc ctg atg ctg gga tgc gtc ctg atg atg gtg gcg atg      96
   Leu Leu Leu Leu Leu Met Leu Gly Cys Val Leu Met Met Val Ala Met
               20                  25                  30
   ttg cac cct ccc cac cac acc ctg cac cag act gtc aca gcc caa gcc     144
   Leu His Pro Pro His His Thr Leu His Gln Thr Val Thr Ala Gln Ala
           35                  40                  45
   agc aag cac agc cct gaa gcc agg tac cgc ctg gac ttt ggg gaa tcc     192
   Ser Lys His Ser Pro Glu Ala Arg Tyr Arg Leu Asp Phe Gly Glu Ser
           50                  55                  60
   cag gat tgg gta ctg gaa gct gag gat gag ggt gaa gag tac agc cct      240
   Gln Asp Trp Val Leu Glu Ala Glu Asp Glu Gly Glu Glu Tyr Ser Pro
   65                  70                  75                  80
   ctg gag ggc ctg cca ccc ttt atc tca ctg cgg gag gat cag ctg ctg      288
   Leu Glu Gly Leu Pro Pro Phe Ile Ser Leu Arg Glu Asp Gln Leu Leu
                   85                  90                  95
   gtg gcc gtg gcc tta ccc cag gcc aga agg aac cag agc cag ggc agg      336
   Val Ala Val Ala Leu Pro Gln Ala Arg Arg Asn Gln Ser Gln Gly Arg
               100                 105                 110
   aga ggt ggg agc tac cgc ctc atc aag cag cca agg agg cag gat aag      384
   Arg Gly Gly Ser Tyr Arg Leu Ile Lys Gln Pro Arg Arg Gln Asp Lys
           115                 120                 125
   gaa gcc cca aag agg gac tgg ggg gct gat gag gac ggg gag gtg tct      432
   Glu Ala Pro Lys Arg Asp Trp Gly Ala Asp Glu Asp Gly Glu Val Ser
       130                 135                 140
   gaa gaa gag gag ttg acc ccg ttc agc ctg gac cca cgt ggc ctc cag      480
   Glu Glu Glu Glu Leu Thr Pro Phe Ser Leu Asp Pro Arg Gly Leu Gln
   145                 150                 155                 160
   gag gca ctc agt gcc cgc atc ccc ctc cag agg gct ctg ccc gag gtg      528
   Glu Ala Leu Ser Ala Arg Ile Pro Leu Gln Arg Ala Leu Pro Glu Val
                   165                 170                 175
   cgg cac cca ctg tgt ctg cag cag cac cct cag gac agc ctg ccc aca      576
   Arg His Pro Leu Cys Leu Gln Gln His Pro Gln Asp Ser Leu Pro Thr
               180                 185                 190
   gcc agc gtc atc ctc tgt ttc cat gat gag gcc tgg tcc act ctc ctg      624
   Ala Ser Val Ile Leu Cys Phe His Asp Glu Ala Trp Ser Thr Leu Leu
           195                 200                 205
   cgg act gta cac agc atc ctc gac aca gtg ccc agg gcc ttc ctg aag      672
   Arg Thr Val His Ser Ile Leu Asp Thr Val Pro Arg Ala Phe Leu Lys
       210                 215                 220
   gag atc atc ctc gtg gac gac ctc agc cag caa gga caa ctc aag tct      720
   Glu Ile Ile Leu Val Asp Asp Leu Ser Gln Gln Gly Gln Leu Lys Ser
   225                 230                 235                 240
   gct ctc agc gaa tat gtg gcc agg ctg gag ggg gtg aag tta ctc agg      768
   Ala Leu Ser Glu Tyr Val Ala Arg Leu Glu Gly Val Lys Leu Leu Arg
                   245                 250                 255
   agc aac aag agg ctg ggt gcc atc agg gcc cgg atg ctg ggg gcc acc      816
   Ser Asn Lys Arg Leu Gly Ala Ile Arg Ala Arg Met Leu Gly Ala Thr
               260                 265                 270
   aga gcc acc ggg gat gtg ctc gtc ttc atg gat gcc cac tgc gag tgc      864
   Arg Ala Thr Gly Asp Val Leu Val Phe Met Asp Ala His Cys Glu Cys
           275                 280                 285
   cac cca ggc tgg ctg gag ccc ctc ctc agc aga ata gct ggt gac agg      912
   His Pro Gly Trp Leu Glu Pro Leu Leu Ser Arg Ile Ala Gly Asp Arg
       290                 295                 300
   agc cga gtg gta tct ccg gtg ata gat gtg att gac tgg aag act ttc      960
   Ser Arg Val Val Ser Pro Val Ile Asp Val Ile Asp Trp Lys Thr Phe
   305                 310                 315                 320
   cag tat tac ccc tca aag gac ctg cag cgt ggg gtg ttg gac tgg aag     1008
   Gln Tyr Tyr Pro Ser Lys Asp Leu Gln Arg Gly Val Leu Asp Trp Lys
                   325                 330                 335
   ctg gat ttc cac tgg gaa cct ttg cca gag cat gtg agg aag gcc ctc      1056
   Leu Asp Phe His Trp Glu Pro Leu Pro Glu His Val Arg Lys Ala Leu
               340                 345                 350
   cag tcc ccc ata agc ccc atc agg agc cct gtg gtg ccc gga gag gtg      1104
   Gln Ser Pro Ile Ser Pro Ile Arg Ser Pro Val Val Pro Gly Glu Val
           355                 360                 365
   gtg gcc atg gac aga cat tac ttc caa aac act gga gcg tat gac tct      1152
   Val Ala Met Asp Arg His Tyr Phe Gln Asn Thr Gly Ala Tyr Asp Ser
       370                 375                 380
   ctt atg tcg ctg cga ggt ggt gaa aac ctc gaa ctg tct ttc aag gcc      1200
   Leu Met Ser Leu Arg Gly Gly Glu Asn Leu Glu Leu Ser Phe Lys Ala
   385                 390                 395                 400
   tgg ctc tgt ggt ggc tct gtt gaa atc ctt ccc tgc tct cgg gta gga      1248
   Trp Leu Cys Gly Gly Ser Val Glu Ile Leu Pro Cys Ser Arg Val Gly
                   405                 410                 415
   cac atc tac caa aat cag gat tcc cat tcc ccc ctc gac cag gag gcc      1296
   His Ile Tyr Gln Asn Gln Asp Ser His Ser Pro Leu Asp Gln Glu Ala
               420                 425                 430
   acc ctg agg aac agg gtt cgc att gct gag acc tgg ctg ggg tca ttc      1344
   Thr Leu Arg Asn Arg Val Arg Ile Ala Glu Thr Trp Leu Gly Ser Phe
           435                 440                 445
   aaa gaa acc ttc tac aag cat agc cca gag gcc ttc tcc ttg agc aag      1392
   Lys Glu Thr Phe Tyr Lys His Ser Pro Glu Ala Phe Ser Leu Ser Lys
       450                 455                 460
   gct gag aag cca gac tgc atg gaa cgc ttg cag ctg caa agg aga ctg      1440
   Ala Glu Lys Pro Asp Cys Met Glu Arg Leu Gln Leu Gln Arg Arg Leu
   465                 470                 475                 480
   ggt tgt cgg aca ttc cac tgg ttt ctg gct aat gtc tac cct gag ctg      1488
   Gly Cys Arg Thr Phe His Trp Phe Leu Ala Asn Val Tyr Pro Glu Leu
                   485                 490                 495
   tac cca tct gaa ccc agg ccc agt ttc tct gga aag ctc cac aac act      1536
   Tyr Pro Ser Glu Pro Arg Pro Ser Phe Ser Gly Lys Leu His Asn Thr
               500                 505                 510
   gga ctt ggg ctc tgt gca gac tgc cag gca gaa ggg gac atc ctg ggc      1584
   Gly Leu Gly Leu Cys Ala Asp Cys Gln Ala Glu Gly Asp Ile Leu Gly
           515                 520                 525
   tgt ccc atg gtg ttg gct cct tgc agt gac agc cgg cag caa cag tac      1632
   Cys Pro Met Val Leu Ala Pro Cys Ser Asp Ser Arg Gln Gln Gln Tyr
       530                 535                 540
   ctg cag cac acc agc agg aag gag att cac ttt ggc agc cca cag cac      1680
   Leu Gln His Thr Ser Arg Lys Glu Ile His Phe Gly Ser Pro Gln His
   545                 550                 555                 560
   ctg tgc ttt gct gtc agg cag gag cag gtg att ctt cag aac tgc acg      1728
   Leu Cys Phe Ala Val Arg Gln Glu Gln Val Ile Leu Gln Asn Cys Thr
                   565                 570                 575
   gag gaa ggc ctg gcc atc cac cag cag cac tgg gac ttc cag gag aat      1776
   Glu Glu Gly Leu Ala Ile His Gln Gln His Trp Asp Phe Gln Glu Asn
               580                 585                 590
   ggg atg att gtc cac att ctt tct ggg aaa tgc atg gaa gct gtg gtg      1824
   Gly Met Ile Val His Ile Leu Ser Gly Lys Cys Met Glu Ala Val Val
           595                 600                 605
   caa gaa aac aat aaa gat ttg tac ctg cgt ccg tgt gat gga aaa gcc      1872
   Gln Glu Asn Asn Lys Asp Leu Tyr Leu Arg Pro Cys Asp Gly Lys Ala
       610                 615                 620
   cgc cag cag tgg cgt ttt gac cag atc aat gct gtg gat gaa cga tga      1920
   Arg Gln Gln Trp Arg Phe Asp Gln Ile Asn Ala Val Asp Glu Arg
   625                 630                 635
<210> 68
   <211> 639
   <212> PRT
   <213> Homo sapiens
<400> 68
   Met Leu Leu Arg Lys Arg Tyr Arg His Arg Pro Cys Arg Leu Gln Phe
   1               5                   10                  15
   Leu Leu Leu Leu Leu Met Leu Gly Cys Val Leu Met Met Val Ala Met
               20                  25                  30
   Leu His Pro Pro His His Thr Leu His Gln Thr Val Thr Ala Gln Ala
           35                  40                  45
   Ser Lys His Ser Pro Glu Ala Arg Tyr Arg Leu Asp Phe Gly Glu Ser
       50                  55                  60
   Gln Asp Trp Val Leu Glu Ala Glu Asp Glu Gly Glu Glu Tyr Ser Pro
   65                  70                  75                  80
   Leu Glu Gly Leu Pro Pro Phe Ile Ser Leu Arg Glu Asp Gln Leu Leu
                   85                  90                  95
   Val Ala Val Ala Leu Pro Gln Ala Arg Arg Asn Gln Ser Gln Gly Arg
               100                 105                 110
   Arg Gly Gly Ser Tyr Arg Leu Ile Lys Gln Pro Arg Arg Gln Asp Lys
           115                 120                 125
   Glu Ala Pro Lys Arg Asp Trp Gly Ala Asp Glu Asp Gly Glu Val Ser
       130                 135                 140
   Glu Glu Glu Glu Leu Thr Pro Phe Ser Leu Asp Pro Arg Gly Leu Gln
   145                 150                 155                 160
   Glu Ala Leu Ser Ala Arg Ile Pro Leu Gln Arg Ala Leu Pro Glu Val
                   165                 170                 175
   Arg His Pro Leu Cys Leu Gln Gln His Pro Gln Asp Ser Leu Pro Thr
               180                 185                 190
   Ala Ser Val Ile Leu Cys Phe His Asp Glu Ala Trp Ser Thr Leu Leu
           195                 200                 205
   Arg Thr Val His Ser Ile Leu Asp Thr Val Pro Arg Ala Phe Leu Lys
       210                 215                 220
   Glu Ile Ile Leu Val Asp Asp Leu Ser Gln Gln Gly Gln Leu Lys Ser
   225                 230                 235                 240
   Ala Leu Ser Glu Tyr Val Ala Arg Leu Glu Gly Val Lys Leu Leu Arg
                   245                 250                 255
   Ser Asn Lys Arg Leu Gly Ala Ile Arg Ala Arg Met Leu Gly Ala Thr
               260                 265                 270
   Arg Ala Thr Gly Asp Val Leu Val Phe Met Asp Ala His Cys Glu Cys
           275                 280                 285
   His Pro Gly Trp Leu Glu Pro Leu Leu Ser Arg Ile Ala Gly Asp Arg
       290                 295                 300
   Ser Arg Val Val Ser Pro Val Ile Asp Val Ile Asp Trp Lys Thr Phe
   305                 310                 315                 320
   Gln Tyr Tyr Pro Ser Lys Asp Leu Gln Arg Gly Val Leu Asp Trp Lys
                   325                 330                 335
   Leu Asp Phe His Trp Glu Pro Leu Pro Glu His Val Arg Lys Ala Leu
               340                 345                 350
   Gln Ser Pro Ile Ser Pro Ile Arg Ser Pro Val Val Pro Gly Glu Val
           355                 360                 365
   Val Ala Met Asp Arg His Tyr Phe Gln Asn Thr Gly Ala Tyr Asp Ser
       370                 375                 380
   Leu Met Ser Leu Arg Gly Gly Glu Asn Leu Glu Leu Ser Phe Lys Ala
   385                 390                 395                 400
   Trp Leu Cys Gly Gly Ser Val Glu Ile Leu Pro Cys Ser Arg Val Gly
                   405                 410                 415
   His Ile Tyr Gln Asn Gln Asp Ser His Ser Pro Leu Asp Gln Glu Ala
               420                 425                 430
   Thr Leu Arg Asn Arg Val Arg Ile Ala Glu Thr Trp Leu Gly Ser Phe
           435                 440                 445
   Lys Glu Thr Phe Tyr Lys His Ser Pro Glu Ala Phe Ser Leu Ser Lys
       450                 455                 460
   Ala Glu Lys Pro Asp Cys Met Glu Arg Leu Gln Leu Gln Arg Arg Leu
   465                 470                 475                 480
   Gly Cys Arg Thr Phe His Trp Phe Leu Ala Asn Val Tyr Pro Glu Leu
   485 490 495
   Tyr Pro Ser Glu Pro Arg Pro Ser Phe Ser Gly Lys Leu His Asn Thr
   500 505 510
   Gly Leu Gly Leu Cys Ala Asp Cys Gln Ala Glu Gly Asp Ile Leu Gly
   515 520 525
   Cys Pro Met Val Leu Ala Pro Cys Ser Asp Ser Arg Gln Gln Gln Tyr
   530 535 540
   Leu Gln His Thr Ser Arg Lys Glu Ile His Phe Gly Ser Pro Gln His
   545 550 555 560
   Leu Cys Phe Ala Val Arg Gln Glu Gln Val Ile Leu Gln Asn Cys Thr
   565 570 575
   Glu Glu Gly Leu Ala Ile His Gln Gln His Trp Asp Phe Gln Glu Asn
   580 585 590
   Gly Met Ile Val His Ile Leu Ser Gly Lys Cys Met Glu Ala Val Val
   595 600 605
   Gln Glu Asn Asn Lys Asp Leu Tyr Leu Arg Pro Cys Asp Gly Lys Ala
   610 615 620
   Arg Gln Gln Trp Arg Phe Asp Gln Ile Asn Ala Val Asp Glu Arg
   625 630 635
<210> 69
   <211> 1671
   <212> DNA
   <213> Homo sapiens
<220>
   <221> CDS
   <222> (1)..(1671)
   <223> Human GalNAc-T14 DNA sequence
<400> 69
   atg agg aga ttt gtc tac tgc aag gtg gtt cta gcc act tcg ctg atg      48
   Met Arg Arg Phe Val Tyr Cys Lys Val Val Leu Ala Thr Ser Leu Met
   1               5                   10                  15
   tgg gtt ctt gtt gat gtc ttc tta ctg ctg tac ttc agt gaa tgt aac      96
   Trp Val Leu Val Asp Val Phe Leu Leu Leu Tyr Phe Ser Glu Cys Asn
               20                  25                  30
   aaa tgt gat gac aag aag gag aga tct ctg ctg cct gca ttg agg gct     144
   Lys Cys Asp Asp Lys Lys Glu Arg Ser Leu Leu Pro Ala Leu Arg Ala
           35                  40                  45
   gtt att tca aga aac caa gaa ggg cca gga gaa atg gga aaa gct gtg     192
   Val Ile Ser Arg Asn Gln Glu Gly Pro Gly Glu Met Gly Lys Ala Val
           50                  55                  60
   ttg att cct aaa gat gac cag gag aaa atg aaa gag ctg ttt aaa atc      240
   Leu Ile Pro Lys Asp Asp Gln Glu Lys Met Lys Glu Leu Phe Lys Ile
   65                  70                  75                  80
   aat cag ttt aac ctt atg gcc agt gat ttg att gcc ctt aat aga agt      288
   Asn Gln Phe Asn Leu Met Ala Ser Asp Leu Ile Ala Leu Asn Arg Ser
                   85                  90                  95
   ctg cca gat gta aga tta gaa gga tgt aag aca aaa gtc tac cct gat      336
   Leu Pro Asp Val Arg Leu Glu Gly Cys Lys Thr Lys Val Tyr Pro Asp
               100                 105                 110
   gaa ctt cca aac aca agt gta gtc att gtg ttt cat aat gaa gct tgg      384
   Glu Leu Pro Asn Thr Ser Val Val Ile Val Phe His Asn Glu Ala Trp
           115                 120                 125
   agc act ctc ctt aga act gtt tac agt gtg ata aat cgt tcc cca cac      432
   Ser Thr Leu Leu Arg Thr Val Tyr Ser Val Ile Asn Arg Ser Pro His
       130                 135                 140
   tat cta ctc tca gag gtc atc ttg gta gat gat gcc agt gaa aga gat      480
   Tyr Leu Leu Ser Glu Val Ile Leu Val Asp Asp Ala Ser Glu Arg Asp
   145                 150                 155                 160
   ttt ctc aag ttg aca tta gag aat tac gtg aaa aat tta gaa gtg cca      528
   Phe Leu Lys Leu Thr Leu Glu Asn Tyr Val Lys Asn Leu Glu Val Pro
                   165                 170                 175
   gta aaa att att agg atg gaa gaa cgc tct ggg tta ata cgt gcc cgt      576
   Val Lys Ile Ile Arg Met Glu Glu Arg Ser Gly Leu Ile Arg Ala Arg
               180                 185                 190
   ctt cga gga gca gct gct tca aaa ggg cag gtc ata act ttt ctt gat      624
   Leu Arg Gly Ala Ala Ala Ser Lys Gly Gln Val Ile Thr Phe Leu Asp
           195                 200                 205
   gca cac tgt gaa tgc acg tta gga tgg ctg gag cct ttg ctg gca aga      672
   Ala His Cys Glu Cys Thr Leu Gly Trp Leu Glu Pro Leu Leu Ala Arg
       210                 215                 220
   ata aag gaa gac agg aaa acg gtt gtc tgc cct atc att gat gtg att      720
   Ile Lys Glu Asp Arg Lys Thr Val Val Cys Pro Ile Ile Asp Val Ile
   225                 230                 235                 240
   agt gat gat act ttt gaa tat atg gct ggg tca gac atg act tat ggg      768
   Ser Asp Asp Thr Phe Glu Tyr Met Ala Gly Ser Asp Met Thr Tyr Gly
                   245                 250                 255
   ggt ttt aac tgg aaa ctg aat ttc cgc tgg tat cct gtt ccc caa aga      816
   Gly Phe Asn Trp Lys Leu Asn Phe Arg Trp Tyr Pro Val Pro Gln Arg
               260                 265                 270
   gaa atg gac agg agg aaa gga gac aga aca tta cct gtc agg acc cct      864
   Glu Met Asp Arg Arg Lys Gly Asp Arg Thr Leu Pro Val Arg Thr Pro
           275                 280                 285
   act atg gct ggt ggc cta ttt tct att gac aga aac tac ttt gaa gag      912
   Thr Met Ala Gly Gly Leu Phe Ser Ile Asp Arg Asn Tyr Phe Glu Glu
       290                 295                 300
   ata gga act tac gat gca gga atg gat atc tgg ggt gga gag aat ctt      960
   Ile Gly Thr Tyr Asp Ala Gly Met Asp Ile Trp Gly Gly Glu Asn Leu
   305                 310                 315                 320
   gaa atg tct ttt agg att tgg caa tgt gga ggc tcc ttg gag att gtt     1008
   Glu Met Ser Phe Arg Ile Trp Gln Cys Gly Gly Ser Leu Glu Ile Val
                   325                 330                 335
   act tgc tcc cat gtt ggt cat gtt ttt cgg aag gca act cca tac act      1056
   Thr Cys Ser His Val Gly His Val Phe Arg Lys Ala Thr Pro Tyr Thr
               340                 345                 350
   ttt cct ggt ggc act ggt cat gtc atc aac aag aac aac agg aga ctg      1104
   Phe Pro Gly Gly Thr Gly His Val Ile Asn Lys Asn Asn Arg Arg Leu
           355                 360                 365
   gca gaa gtt tgg atg gat gaa ttt aaa gat ttc ttc tac atc ata tcc      1152
   Ala Glu Val Trp Met Asp Glu Phe Lys Asp Phe Phe Tyr Ile Ile Ser
       370                 375                 380
   cca ggt gtt gtc aaa gtg gat tat gga gat gtg tca gtc aga aaa aca      1200
   Pro Gly Val Val Lys Val Asp Tyr Gly Asp Val Ser Val Arg Lys Thr
   385                 390                 395                 400
   cta aga gaa aat ctg aag tgt aag ccc ttt tct tgg tac cta gaa aac      1248
   Leu Arg Glu Asn Leu Lys Cys Lys Pro Phe Ser Trp Tyr Leu Glu Asn
                   405                 410                 415
   atc tat ccg gac tcc cag atc cca aga cgt tat tac tca ctt ggt gag      1296
   Ile Tyr Pro Asp Ser Gln Ile Pro Arg Arg Tyr Tyr Ser Leu Gly Glu
               420                 425                 430
   ata aga aat gtt gaa acc aat cag tgt tta gac aac atg ggc cgc aag      1344
   Ile Arg Asn Val Glu Thr Asn Gln Cys Leu Asp Asn Met Gly Arg Lys
           435                 440                 445
   gaa aat gaa aaa gtg ggt ata ttc aac tgt cat ggt atg gga gga aat      1392
   Glu Asn Glu Lys Val Gly Ile Phe Asn Cys His Gly Met Gly Gly Asn
       450                 455                 460
   cag gta ttt tct tac act gct gac aaa gaa atc cga acc gat gac ttg      1440
   Gln Val Phe Ser Tyr Thr Ala Asp Lys Glu Ile Arg Thr Asp Asp Leu
   465                 470                 475                 480
   tgc ttg gat gtt tct aga ctc aat gga cct gta atc atg tta aaa tgc      1488
   Cys Leu Asp Val Ser Arg Leu Asn Gly Pro Val Ile Met Leu Lys Cys
                   485                 490                 495
   cac cat atg aga gga aat cag tta tgg gaa tat gat gct gag aga ctc      1536
   His His Met Arg Gly Asn Gln Leu Trp Glu Tyr Asp Ala Glu Arg Leu
               500                 505                 510
   acg ttg cga cat gtt aac agt aac caa tgt ctc gat gaa cct tct gaa      1584
   Thr Leu Arg His Val Asn Ser Asn Gln Cys Leu Asp Glu Pro Ser Glu
           515                 520                 525
   gaa gac aaa atg gtg cct aca atg cag gac tgt agt gga agc aga tcc      1632
   Glu Asp Lys Met Val Pro Thr Met Gln Asp Cys Ser Gly Ser Arg Ser
       530                 535                 540
   caa cag tgg ctg cta agg aac atg acc ttg ggc aca tga                  1671
   Gln Gln Trp Leu Leu Arg Asn Met Thr Leu Gly Thr
   545                 550                 555
<210> 70
   <211> 556
   <212> PRT
   <213> Homo sapiens
<400> 70
   Met Arg Arg Phe Val Tyr Cys Lys Val Val Leu Ala Thr Ser Leu Met
   1 5 10 15
   Trp Val Leu Val Asp Val Phe Leu Leu Leu Tyr Phe Ser Glu Cys Asn
   20 25 30
   Lys Cys Asp Asp Lys Lys Glu Arg Ser Leu Leu Pro Ala Leu Arg Ala
   35 40 45
   Val Ile Ser Arg Asn Gln Glu Gly Pro Gly Glu Met Gly Lys Ala Val
   50 55 60
   Leu Ile Pro Lys Asp Asp Gln Glu Lys Met Lys Glu Leu Phe Lys Ile
   65 70 75 80
   Asn Gln Phe Asn Leu Met Ala Ser Asp Leu Ile Ala Leu Asn Arg Ser
   85 90 95
   Leu Pro Asp Val Arg Leu Glu Gly Cys Lys Thr Lys Val Tyr Pro Asp
   100 105 110
   Glu Leu Pro Asn Thr Ser Val Val Ile Val Phe His Asn Glu Ala Trp
   115 120 125
   Ser Thr Leu Leu Arg Thr Val Tyr Ser Val Ile Asn Arg Ser Pro His
   130 135 140
   Tyr Leu Leu Ser Glu Val Ile Leu Val Asp Asp Ala Ser Glu Arg Asp
   145 150 155 160
   Phe Leu Lys Leu Thr Leu Glu Asn Tyr Val Lys Asn Leu Glu Val Pro
   165 170 175
   Val Lys Ile Ile Arg Met Glu Glu Arg Ser Gly Leu Ile Arg Ala Arg
   180 185 190
   Leu Arg Gly Ala Ala Ala Ser Lys Gly Gln Val Ile Thr Phe Leu Asp
   195 200 205
   Ala His Cys Glu Cys Thr Leu Gly Trp Leu Glu Pro Leu Leu Ala Arg
   210 215 220
   Ile Lys Glu Asp Arg Lys Thr Val Val Cys Pro Ile Ile Asp Val Ile
   225 230 235 240
   Ser Asp Asp Thr Phe Glu Tyr Met Ala Gly Ser Asp Met Thr Tyr Gly
   245 250 255
   Gly Phe Asn Trp Lys Leu Asn Phe Arg Trp Tyr Pro Val Pro Gln Arg
   260 265 270
   Glu Met Asp Arg Arg Lys Gly Asp Arg Thr Leu Pro Val Arg Thr Pro
   275 280 285
   Thr Met Ala Gly Gly Leu Phe Ser Ile Asp Arg Asn Tyr Phe Glu Glu
       290                 295                 300
   Ile Gly Thr Tyr Asp Ala Gly Met Asp Ile Trp Gly Gly Glu Asn Leu
   305                 310                 315                 320
   Glu Met Ser Phe Arg Ile Trp Gln Cys Gly Gly Ser Leu Glu Ile Val
                   325                 330                 335
   Thr Cys Ser His Val Gly His Val Phe Arg Lys Ala Thr Pro Tyr Thr
               340                 345                 350
   Phe Pro Gly Gly Thr Gly His Val Ile Asn Lys Asn Asn Arg Arg Leu
           355                 360                 365
   Ala Glu Val Trp Met Asp Glu Phe Lys Asp Phe Phe Tyr Ile Ile Ser
       370                 375                 380
   Pro Gly Val Val Lys Val Asp Tyr Gly Asp Val Ser Val Arg Lys Thr
   385                 390                 395                 400
   Leu Arg Glu Asn Leu Lys Cys Lys Pro Phe Ser Trp Tyr Leu Glu Asn
                   405                 410                 415
   Ile Tyr Pro Asp Ser Gln Ile Pro Arg Arg Tyr Tyr Ser Leu Gly Glu
               420                 425                 430
   Ile Arg Asn Val Glu Thr Asn Gln Cys Leu Asp Asn Met Gly Arg Lys
           435                 440                 445
   Glu Asn Glu Lys Val Gly Ile Phe Asn Cys His Gly Met Gly Gly Asn
       450                 455                 460
   Gln Val Phe Ser Tyr Thr Ala Asp Lys Glu Ile Arg Thr Asp Asp Leu
   465                 470                 475                 480
   Cys Leu Asp Val Ser Arg Leu Asn Gly Pro Val Ile Met Leu Lys Cys
                   485                 490                 495
   His His Met Arg Gly Asn Gln Leu Trp Glu Tyr Asp Ala Glu Arg Leu
               500                 505                 510
   Thr Leu Arg His Val Asn Ser Asn Gln Cys Leu Asp Glu Pro Ser Glu
           515                 520                 525
   Glu Asp Lys Met Val Pro Thr Met Gln Asp Cys Ser Gly Ser Arg Ser
       530                 535                 540
   Gln Gln Trp Leu Leu Arg Asn Met Thr Leu Gly Thr
   545                 550                 555
<210> 71
   <211> 1911
   <212> DNA
   <213> Homo sapiens
<220>
   <221> CDS
   <222> (1)..(1659)
   <223> Human GlaNAC-T15 DNA sequence
<400> 71
   atg cgg cgc ctg act cgt cgg ctg gtt ctg cca gtc ttc ggg gtg ctc        48
   Met Arg Arg Leu Thr Arg Arg Leu Val Leu Pro Val Phe Gly Val Leu
   1               5                   10                  15
   tgg atc acg gtg ctg ctg ttc ttc tgg gta acc aag agg aag ttg gag        96
   Trp Ile Thr Val Leu Leu Phe Phe Trp Val Thr Lys Arg Lys Leu Glu
               20                  25                  30
   gtg ccg acg gga cct gaa gtg cag acc cct aag cct tcg gac gct gac       144
   Val Pro Thr Gly Pro Glu Val Gln Thr Pro Lys Pro Ser Asp Ala Asp
           35                  40                  45
   tgg gac gac ctg tgg gac cag ttt gat gag cgg cgg tat ctg aat gcc       192
   Trp Asp Asp Leu Trp Asp Gln Phe Asp Glu Arg Arg Tyr Leu Asn Ala
       50                  55                  60
   aaa aag tgg cgc gtt ggt gac gac ccc tat aag ctg tat gct ttc aac       240
   Lys Lys Trp Arg Val Gly Asp Asp Pro Tyr Lys Leu Tyr Ala Phe Asn
   65                  70                  75                  80
   cag cgg gag agt gag cgg atc tcc agc aat cgg gcc atc ccg gac act       288
   Gln Arg Glu Ser Glu Arg Ile Ser Ser Asn Arg Ala Ile Pro Asp Thr
                   85                  90                  95
   cgc cat ctg aga tgc aca ctg ctg gtg tat tgc acg gac ctt cca ccc       336
   Arg His Leu Arg Cys Thr Leu Leu Val Tyr Cys Thr Asp Leu Pro Pro
               100                 105                 110
   act agc atc atc atc acc ttc cac aac gaa gcc cgc tcc acg ctg ctc       384
   Thr Ser Ile Ile Ile Thr Phe His Asn Glu Ala Arg Ser Thr Leu Leu
           115                 120                 125
   agg acc atc cgc agt gta tta aac cgc acc cct acg cat ctg atc cgg       432
   Arg Thr Ile Arg Ser Val Leu Asn Arg Thr Pro Thr His Leu Ile Arg
       130                 135                 140
   gaa atc ata tta gtg gat gac ttc agc aat gac cct gat gac tgt aaa       480
   Glu Ile Ile Leu Val Asp Asp Phe Ser Asn Asp Pro Asp Asp Cys Lys
   145                 150                 155                 160
   cag ctc atc aaa ttg ccc aag gtg aaa tgc ttg cgc aat aat gaa cgg       528
   Gln Leu Ile Lys Leu Pro Lys Val Lys Cys Leu Arg Asn Asn Glu Arg
                   165                 170                 175
   caa ggt ctg gtc cgg tcc cgg att cgg ggc gct gac atc gcc cag ggc       576
   Gln Gly Leu Val Arg Ser Arg Ile Arg Gly Ala Asp Ile Ala Gln Gly
               180                 185                 190
   acc act ctg act ttc ctc gac agc cac tgt gag gtg aac agg gac tgg       624
   Thr Thr Leu Thr Phe Leu Asp Ser His Cys Glu Val Asn Arg Asp Trp
           195                 200                 205
   ctc cag cct ctg ttg cac agg gtc aaa gaa gac tac acg cgg gtg gtg       672
   Leu Gln Pro Leu Leu His Arg Val Lys Glu Asp Tyr Thr Arg Val Val
           210                 215                 220
   tgc cct gtg atc gat atc att aac ctg gac acc ttc acc tac atc gag       720
   Cys Pro Val Ile Asp Ile Ile Asn Leu Asp Thr Phe Thr Tyr Ile Glu
   225                 230                 235                 240
   tct gcc tcg gag ctc aga ggg ggg ttt gac tgg agc ctc cac ttc cag       768
   Ser Ala Ser Glu Leu Arg Gly Gly Phe Asp Trp Ser Leu His Phe Gln
                   245                 250                 255
   tgg gag cag ctc tcc cca gag cag aag ctc ggc gcc tgg acc cca cgg       816
   Trp Glu Gln Leu Ser Pro Glu Gln Lys Leu Gly Ala Trp Thr Pro Arg
               260                 265                 270
   aag ccc atc agg act cct atc ata gct gga ggg ctc ttc gtg atc gac       864
   Lys Pro Ile Arg Thr Pro Ile Ile Ala Gly Gly Leu Phe Val Ile Asp
           275                 280                 285
   aaa gct tgg ttt gat tac ctg ggg aaa tat gat atg gac atg gac atc       912
   Lys Ala Trp Phe Asp Tyr Leu Gly Lys Tyr Asp Met Asp Met Asp Ile
       290                 295                 300
   tgg ggt ggg gag aac ttt gaa atc tcc ttc cga gtg tgg atg tgc ggg       960
   Trp Gly Gly Glu Asn Phe Glu Ile Ser Phe Arg Val Trp Met Cys Gly
   305                 310                 315                 320
   ggc agc cta gag atc gtc ccc tgc agc cga gtg ggg cac gtc ttc cgg      1008
   Gly Ser Leu Glu Ile Val Pro Cys Ser Arg Val Gly His Val Phe Arg
                   325                 330                 335
   aag aag cac ccc tac gtt ttc cct gat gga aat gcc aac acg tat ata      1056
   Lys Lys His Pro Tyr Val Phe Pro Asp Gly Asn Ala Asn Thr Tyr Ile
               340                 345                 350
   aag aac acc aag cgg aca gct gaa gtg tgg atg gat gaa tac aag caa      1104
   Lys Asn Thr Lys Arg Thr Ala Glu Val Trp Met Asp Glu Tyr Lys Gln
           355                 360                 365
   tac tat tac gct gcc cgg cca ttc gcc ctg gag agg ccc ttc ggg aat      1152
   Tyr Tyr Tyr Ala Ala Arg Pro Phe Ala Leu Glu Arg Pro Phe Gly Asn
       370                 375                 380
   gtt gag agc aga ttg gac ctg agg aag aat ctg cgc tgc cag agc ttc      1200
   Val Glu Ser Arg Leu Asp Leu Arg Lys Asn Leu Arg Cys Gln Ser Phe
   385                 390                 395                 400
   aag tgg tac ctg gag aat atc tac cct gaa ctc agc atc ccc aag gag      1248
   Lys Trp Tyr Leu Glu Asn Ile Tyr Pro Glu Leu Ser Ile Pro Lys Glu
                   405                 410                 415
   tcc tcc atc cag aag ggc aat atc cga cag aga cag aag tgc ctg gaa      1296
   Ser Ser Ile Gln Lys Gly Asn Ile Arg Gln Arg Gln Lys Cys Leu Glu
               420                 425                 430
   tct caa agg cag aac aac caa gaa acc cca aac cta aag ttg agc ccc      1344
   Ser Gln Arg Gln Asn Asn Gln Glu Thr Pro Asn Leu Lys Leu Ser Pro
           435                 440                 445
   tgt gcc aag gtc aaa ggc gaa gat gca aag tcc cag gta tgg gcc ttc      1392
   Cys Ala Lys Val Lys Gly Glu Asp Ala Lys Ser Gln Val Trp Ala Phe
       450                 455                 460
   aca tac acc cag cag atc ctc cag gag gag ctg tgc ctg tca gtc atc      1440
   Thr Tyr Thr Gln Gln Ile Leu Gln Glu Glu Leu Cys Leu Ser Val Ile
   465                 470                 475                 480
   acc ttg ttc cct ggc gcc cca gtg gtt ctt gtc ctt tgc aag aat gga      1488
   Thr Leu Phe Pro Gly Ala Pro Val Val Leu Val Leu Cys Lys Asn Gly
                       485                  490                  495
   gat gac cga cag caa tgg acc aaa act ggt tcc cac atc gag cac ata     1536
   Asp Asp Arg Gln Gln Trp Thr Lys Thr Gly Ser His Ile Glu His Ile
               500                 505                 510
   gca tcc cac ctc tgc ctc gat aca gat atg ttc ggt gat ggc acc gag     1584
   Ala Ser His Leu Cys Leu Asp Thr Asp Met Phe Gly Asp Gly Thr Glu
           515                 520                 525
   aac ggc aag gaa atc gtc gtc aac cca tgt gag tcc tca ctc atg agc     1632
   Asn Gly Lys Glu Ile Val Val Asn Pro Cys Glu Ser Ser Leu Met Ser
       530                 535                 540
   cag cac tgg gac atg gtg agc tct tga ggacccctgc cagaagcagc           1679
   Gln His Trp Asp Met Val Ser Ser
   545                 550
   aagggccatg gggtggtgct tccctggacc agaacagact ggaaactggg cagcaagcag   1739
   cctgcaacca cctcagacat cctggactgg gaggtggagg cagagccccc caggacagga   1799
   gcaactgtct cagggaggac agaggaaaac atcacaagcc aatggggctc aaagacaaat   1859
   cccacatgtt ctcaaggccg ttaagttcca gtcctggcca gtcattccct ga           1911
<210> 72
   <211> 552
   <212> PRT
   <213> Homo sapiens
<400> 72
   Met Arg Arg Leu Thr Arg Arg Leu Val Leu Pro Val Phe Gly Val Leu
   1               5                   10                  15
   Trp Ile Thr Val Leu Leu Phe Phe Trp Val Thr Lys Arg Lys Leu Glu
               20                  25                  30
   Val Pro Thr Gly Pro Glu Val Gln Thr Pro Lys Pro Ser Asp Ala Asp
           35                  40                  45
   Trp Asp Asp Leu Trp Asp Gln Phe Asp Glu Arg Arg Tyr Leu Asn Ala
       50                  55                  60
   Lys Lys Trp Arg Val Gly Asp Asp Pro Tyr Lys Leu Tyr Ala Phe Asn
   65                  70                  75                  80
   Gln Arg Glu Ser Glu Arg Ile Ser Ser Asn Arg Ala Ile Pro Asp Thr
                   85                  90                  95
   Arg His Leu Arg Cys Thr Leu Leu Val Tyr Cys Thr Asp Leu Pro Pro
               100                 105                 110
   Thr Ser Ile Ile Ile Thr Phe His Asn Glu Ala Arg Ser Thr Leu Leu
           115                 120                 125
   Arg Thr Ile Arg Ser Val Leu Asn Arg Thr Pro Thr His Leu Ile Arg
       130                 135                 140
   Glu Ile Ile Leu Val Asp Asp Phe Ser Asn Asp Pro Asp Asp Cys Lys
   145 150 155 160
   Gln Leu Ile Lys Leu Pro Lys Val Lys Cys Leu Arg Asn Asn Glu Arg
   165 170 175
   Gln Gly Leu Val Arg Ser Arg Ile Arg Gly Ala Asp Ile Ala Gln Gly
   180 185 190
   Thr Thr Leu Thr Phe Leu Asp Ser His Cys Glu Val Asn Arg Asp Trp
   195 200 205
   Leu Gln Pro Leu Leu His Arg Val Lys Glu Asp Tyr Thr Arg Val Val
   210 215 220
   Cys Pro Val Ile Asp Ile Ile Asn Leu Asp Thr Phe Thr Tyr Ile Glu
   225 230 235 240
   Ser Ala Ser Glu Leu Arg Gly Gly Phe Asp Trp Ser Leu His Phe Gln
   245 250 255
   Trp Glu Gln Leu Ser Pro Glu Gln Lys Leu Gly Ala Trp Thr Pro Arg
   260 265 270
   Lys Pro Ile Arg Thr Pro Ile Ile Ala Gly Gly Leu Phe Val Ile Asp
   275 280 285
   Lys Ala Trp Phe Asp Tyr Leu Gly Lys Tyr Asp Met Asp Met Asp Ile
   290 295 300
   Trp Gly Gly Glu Asn Phe Glu Ile Ser Phe Arg Val Trp Met Cys Gly
   305 310 315 320
   Gly Ser Leu Glu Ile Val Pro Cys Ser Arg Val Gly His Val Phe Arg
   325 330 335
   Lys Lys His Pro Tyr Val Phe Pro Asp Gly Asn Ala Asn Thr Tyr Ile
   340 345 350
   Lys Asn Thr Lys Arg Thr Ala Glu Val Trp Met Asp Glu Tyr Lys Gln
   355 360 365
   Tyr Tyr Tyr Ala Ala Arg Pro Phe Ala Leu Glu Arg Pro Phe Gly Asn
   370 375 380
   Val Glu Ser Arg Leu Asp Leu Arg Lys Asn Leu Arg Cys Gln Ser Phe
   385 390 395 400
   Lys Trp Tyr Leu Glu Asn Ile Tyr Pro Glu Leu Ser Ile Pro Lys Glu
   405 410 415
   Ser Ser Ile Gln Lys Gly Asn Ile Arg Gln Arg Gln Lys Cys Leu Glu
   420 425 430
   Ser Gln Arg Gln Asn Asn Gln Glu Thr Pro Asn Leu Lys Leu Ser Pro
   435 440 445
   Cys Ala Lys Val Lys Gly Glu Asp Ala Lys Ser Gln Val Trp Ala Phe
   450 455 460
   Thr Tyr Thr Gln Gln Ile Leu Gln Glu Glu Leu Cys Leu Ser Val Ile
   465 470 475 480
   Thr Leu Phe Pro Gly Ala Pro Val Val Leu Val Leu Cys Lys Asn Gly
   485 490 495
   Asp Asp Arg Gln Gln Trp Thr Lys Thr Gly Ser His Ile Glu His Ile
   500 505 510
   Ala Ser His Leu Cys Leu Asp Thr Asp Met Phe Gly Asp Gly Thr Glu
   515 520 525
   Asn Gly Lys Glu Ile Val Val Asn Pro Cys Glu Ser Ser Leu Met Ser
   530 535 540
   Gln His Trp Asp Met Val Ser Ser
   545 550
<210> 73
   <211> 1677
   <212> DNA
   <213> Homo sapiens
<220>
   <221> CDS
   <222> (1)..(1677)
   <223> Human GalNAc-T16 DNA sequence
<400> 73
   atg agg aag atc cgc gcc aat gcc atc gcc atc ctg acc gta gcc tgg       48
   Met Arg Lys Ile Arg Ala Asn Ala Ile Ala Ile Leu Thr Val Ala Trp
   1               5                   10                  15
   atc ctg ggc act ttc tac tac tta tgg cag gac aac cga gcc cac gca       96
   Ile Leu Gly Thr Phe Tyr Tyr Leu Trp Gln Asp Asn Arg Ala His Ala
               20                  25                  30
   gca tcc tcc ggc ggc cgg ggc gcg cag agg gca ggc agg agg tcg gag      144
   Ala Ser Ser Gly Gly Arg Gly Ala Gln Arg Ala Gly Arg Arg Ser Glu
           35                  40                  45
   cag ctc cgc gag gac cgc acc atc ccg ctc att gtg aca gga act ccc      192
   Gln Leu Arg Glu Asp Arg Thr Ile Pro Leu Ile Val Thr Gly Thr Pro
       50                  55                  60
   tcg aaa ggc ttt gat gag aag gcc tac ctg tcg gcc aag cag ctg aag      240
   Ser Lys Gly Phe Asp Glu Lys Ala Tyr Leu Ser Ala Lys Gln Leu Lys
   65                  70                  75                  80
   gct gga gag gac ccc tac aga cag cac gcc ttc aac cag ctg gag agt      288
   Ala Gly Glu Asp Pro Tyr Arg Gln His Ala Phe Asn Gln Leu Glu Ser
                   85                  90                  95
   gac aag ctg agc cca gac cgg ccc atc cgg gac acc cgc cat tac agc      336
   Asp Lys Leu Ser Pro Asp Arg Pro Ile Arg Asp Thr Arg His Tyr Ser
               100                 105                 110
   tgc cca tct gtg tcc tac tcc tcg gac ctg cca gcc acc agc gtc atc      384
   Cys Pro Ser Val Ser Tyr Ser Ser Asp Leu Pro Ala Thr Ser Val Ile
           115                 120                 125
   atc acc ttc cac aat gag gcc cgt tcc acc ctg ctg cgc aca gtg aag      432
   Ile Thr Phe His Asn Glu Ala Arg Ser Thr Leu Leu Arg Thr Val Lys
       130                 135                 140
   agt gtc ctg aac cga act cct gcc aac ttg atc cag gag atc att tta      480
   Ser Val Leu Asn Arg Thr Pro Ala Asn Leu Ile Gln Glu Ile Ile Leu
   145                 150                 155                 160
   gtg gat gac ttc agc tca gat ccg gaa gac tgt cta ctc ctg acc agg      528
   Val Asp Asp Phe Ser Ser Asp Pro Glu Asp Cys Leu Leu Leu Thr Arg
                   165                 170                 175
   atc ccc aag gtc aag tgc ctg cgc aat gat cgg cgg gaa ggg ctg atc      576
   Ile Pro Lys Val Lys Cys Leu Arg Asn Asp Arg Arg Glu Gly Leu Ile
               180                 185                 190
   cgg tcc cga gtg cgt ggg gcg gac gtg gct gca gct acc gtt ctc acc      624
   Arg Ser Arg Val Arg Gly Ala Asp Val Ala Ala Ala Thr Val Leu Thr
           195                 200                 205
   ttt ctg gat agc cac tgc gaa gtg aac acc gag tgg ctg ccg ccc atg      672
   Phe Leu Asp Ser His Cys Glu Val Asn Thr Glu Trp Leu Pro Pro Met
       210                 215                 220
   ctg cag cgg gtg aag gag gac cac acc cgc gtg gtg agt ccc atc att      720
   Leu Gln Arg Val Lys Glu Asp His Thr Arg Val Val Ser Pro Ile Ile
   225                 230                 235                 240
   gat gtc atc agt ctg gat aat ttt gcc tac ctt gca gca tct gct gac      768
   Asp Val Ile Ser Leu Asp Asn Phe Ala Tyr Leu Ala Ala Ser Ala Asp
                   245                 250                 255
   ctt cgt gga ggg ttc gac tgg agc ctg cat ttc aag tgg gag cag atc      816
   Leu Arg Gly Gly Phe Asp Trp Ser Leu His Phe Lys Trp Glu Gln Ile
               260                 265                 270
   cct ctt gag cag aag atg acc cgg aca gac ccc acc agg ccc ata agg      864
   Pro Leu Glu Gln Lys Met Thr Arg Thr Asp Pro Thr Arg Pro Ile Arg
           275                 280                 285
   acg cct gtc ata gct gga gga atc ttc gtg atc gac aag tcc tgg ttt      912
   Thr Pro Val Ile Ala Gly Gly Ile Phe Val Ile Asp Lys Ser Trp Phe
       290                 295                 300
   aac cac ttg gga aag tat gat gcc cag atg gac atc tgg ggg gga gag      960
   Asn His Leu Gly Lys Tyr Asp Ala Gln Met Asp Ile Trp Gly Gly Glu
   305                 310                 315                 320
   aat ttt gag ctc tcc ttc agg gtg tgg atg tgt ggt ggc agt ctg gag     1008
   Asn Phe Glu Leu Ser Phe Arg Val Trp Met Cys Gly Gly Ser Leu Glu
                   325                 330                 335
   atc gtc ccc tgc agc cgg gtg ggc cat gtc ttc agg aaa cgg cac ccc     1056
   Ile Val Pro Cys Ser Arg Val Gly His Val Phe Arg Lys Arg His Pro
                       340                 345                 350
   tac aac ttc cct gag ggt aat gcc ctc acc tac atc agg aat act aag      1104
   Tyr Asn Phe Pro Glu Gly Asn Ala Leu Thr Tyr Ile Arg Asn Thr Lys
           355                 360                 365
   cgc act gca gaa gtg tgg atg gat gaa tac aag caa tac tac tat gag      1152
   Arg Thr Ala Glu Val Trp Met Asp Glu Tyr Lys Gln Tyr Tyr Tyr Glu
       370                 375                 380
   gcc cgg ccc tcg gcc atc ggg aag gcc ttc ggc agt gtg gct acg cgg      1200
   Ala Arg Pro Ser Ala Ile Gly Lys Ala Phe Gly Ser Val Ala Thr Arg
   385                 390                 395                 400
   ata gag cag agg aag aag atg aac tgc aag tcc ttc cgc tgg tac ctg      1248
   Ile Glu Gln Arg Lys Lys Met Asn Cys Lys Ser Phe Arg Trp Tyr Leu
                   405                 410                 415
   gag aac gtc tac cca gag ctc acg gtc ccc gtg aag gaa gca ctc ccc      1296
   Glu Asn Val Tyr Pro Glu Leu Thr Val Pro Val Lys Glu Ala Leu Pro
               420                 425                 430
   ggc atc att aag cag ggg gtg aac tgc tta gaa tct cag ggc cag aac      1344
   Gly Ile Ile Lys Gln Gly Val Asn Cys Leu Glu Ser Gln Gly Gln Asn
           435                 440                 445
   aca gct ggt gac ttc ctg ctt gga atg ggg atc tgc aga ggg tct gcc      1392
   Thr Ala Gly Asp Phe Leu Leu Gly Met Gly Ile Cys Arg Gly Ser Ala
       450                 455                 460
   aag aac ccg cag ccc gcc cag gca tgg ctg ttc agt gac cac ctc atc      1440
   Lys Asn Pro Gln Pro Ala Gln Ala Trp Leu Phe Ser Asp His Leu Ile
   465                 470                 475                 480
   cag cag cag ggg aag tgc ctg gct gcc acc tcc acc tta atg tcc tcc      1488
   Gln Gln Gln Gly Lys Cys Leu Ala Ala Thr Ser Thr Leu Met Ser Ser
                   485                 490                 495
   cct gga tcc cca gtc ata ctg cag atg tgc aac cct aga gaa ggc aag      1536
   Pro Gly Ser Pro Val Ile Leu Gln Met Cys Asn Pro Arg Glu Gly Lys
               500                 505                 510
   cag aaa tgg agg aga aaa gga tct ttc atc cag cat tca gtc agt ggc      1584
   Gln Lys Trp Arg Arg Lys Gly Ser Phe Ile Gln His Ser Val Ser Gly
           515                 520                 525
   ctc tgc ctg gag aca aag cct gcc cag ctg gtg acc agc aag tgt cag      1632
   Leu Cys Leu Glu Thr Lys Pro Ala Gln Leu Val Thr Ser Lys Cys Gln
       530                 535                 540
   gct gac gcc cag gcc cag cag tgg cag ctg ttg cca cac aca tga          1677
   Ala Asp Ala Gln Ala Gln Gln Trp Gln Leu Leu Pro His Thr
   545                 550                 555
<210> 74
   <211> 558
   <212> PRT
   <213> Homo sapiens
<400> 74
   Met Arg Lys Ile Arg Ala Asn Ala Ile Ala Ile Leu Thr Val Ala Trp
   1 5 10 15
   Ile Leu Gly Thr Phe Tyr Tyr Leu Trp Gln Asp Asn Arg Ala His Ala
   20 25 30
   Ala Ser Ser Gly Gly Arg Gly Ala Gln Arg Ala Gly Arg Arg Ser Glu
           35                  40                  45
   Gln Leu Arg Glu Asp Arg Thr Ile Pro Leu Ile Val Thr Gly Thr Pro
       50                  55                  60
   Ser Lys Gly Phe Asp Glu Lys Ala Tyr Leu Ser Ala Lys Gln Leu Lys
   65                  70                  75                  80
   Ala Gly Glu Asp Pro Tyr Arg Gln His Ala Phe Asn Gln Leu Glu Ser
                   85                  90                  95
   Asp Lys Leu Ser Pro Asp Arg Pro Ile Arg Asp Thr Arg His Tyr Ser
               100                 105                 110
   Cys Pro Ser Val Ser Tyr Ser Ser Asp Leu Pro Ala Thr Ser Val Ile
           115                 120                 125
   Ile Thr Phe His Asn Glu Ala Arg Ser Thr Leu Leu Arg Thr Val Lys
       130                 135                 140
   Ser Val Leu Asn Arg Thr Pro Ala Asn Leu Ile Gln Glu Ile Ile Leu
   145                 150                 155                 160
   Val Asp Asp Phe Ser Ser Asp Pro Glu Asp Cys Leu Leu Leu Thr Arg
                   165                 170                 175
   Ile Pro Lys Val Lys Cys Leu Arg Asn Asp Arg Arg Glu Gly Leu Ile
               180                 185                 190
   Arg Ser Arg Val Arg Gly Ala Asp Val Ala Ala Ala Thr Val Leu Thr
           195                 200                 205
   Phe Leu Asp Ser His Cys Glu Val Asn Thr Glu Trp Leu Pro Pro Met
       210                 215                 220
   Leu Gln Arg Val Lys Glu Asp His Thr Arg Val Val Ser Pro Ile Ile
   225                 230                 235                 240
   Asp Val Ile Ser Leu Asp Asn Phe Ala Tyr Leu Ala Ala Ser Ala Asp
                   245                 250                 255
   Leu Arg Gly Gly Phe Asp Trp Ser Leu His Phe Lys Trp Glu Gln Ile
               260                 265                 270
   Pro Leu Glu Gln Lys Met Thr Arg Thr Asp Pro Thr Arg Pro Ile Arg
           275                 280                 285
   Thr Pro Val Ile Ala Gly Gly Ile Phe Val Ile Asp Lys Ser Trp Phe
       290                 295                 300
   Asn His Leu Gly Lys Tyr Asp Ala Gln Met Asp Ile Trp Gly Gly Glu
   305 310 315 320
   Asn Phe Glu Leu Ser Phe Arg Val Trp Met Cys Gly Gly Ser Leu Glu
   325 330 335
   Ile Val Pro Cys Ser Arg Val Gly His Val Phe Arg Lys Arg His Pro
   340 345 350
   Tyr Asn Phe Pro Glu Gly Asn Ala Leu Thr Tyr Ile Arg Asn Thr Lys
   355 360 365
   Arg Thr Ala Glu Val Trp Met Asp Glu Tyr Lys Gln Tyr Tyr Tyr Glu
   370 375 380
   Ala Arg Pro Ser Ala Ile Gly Lys Ala Phe Gly Ser Val Ala Thr Arg
   385 390 395 400
   Ile Glu Gln Arg Lys Lys Met Asn Cys Lys Ser Phe Arg Trp Tyr Leu
   405 410 415
   Glu Asn Val Tyr Pro Glu Leu Thr Val Pro Val Lys Glu Ala Leu Pro
   420 425 430
   Gly Ile Ile Lys Gln Gly Val Asn Cys Leu Glu Ser Gln Gly Gln Asn
   435 440 445
   Thr Ala Gly Asp Phe Leu Leu Gly Met Gly Ile Cys Arg Gly Ser Ala
   450 455 460
   Lys Asn Pro Gln Pro Ala Gln Ala Trp Leu Phe Ser Asp His Leu Ile
   465 470 475 480
   Gln Gln Gln Gly Lys Cys Leu Ala Ala Thr Ser Thr Leu Met Ser Ser
   485 490 495
   Pro Gly Ser Pro Val Ile Leu Gln Met Cys Asn Pro Arg Glu Gly Lys
   500 505 510
   Gln Lys Trp Arg Arg Lys Gly Ser Phe Ile Gln His Ser Val Ser Gly
   515 520 525
   Leu Cys Leu Glu Thr Lys Pro Ala Gln Leu Val Thr Ser Lys Cys Gln
   530 535 540
   Ala Asp Ala Gln Ala Gln Gln Trp Gln Leu Leu Pro His Thr
   545 550 555
<210> 75
   <211> 157
   <212> DNA
   <213> synthetic
<220>
   <221> misc_feature
   <222> (1)..(157)
   <223> pBKS-Histag-I modified sequence
<400> 75
   gcggccgctc tagaactagt ggatccagca gccatcatca tcatcatcac agcagcggcc 60
   tggtgccgcg cggcagccat atggctagca tgactggtgg acagcaaatg ggtcgcggaa 120
   ttccgatatc aagcttatcg ataccgtcga cctcgag 157
<210> 76
   <211> 123
   <212> DNA
   <213> synthetic
<220>
   <221> misc_feature
   <222> (1)..(123)
   <223> pBKS-Histag-II modified sequence
<400> 76
   gaattcgcgg ccgcagcagc catcatcatc atcatcacag cagcggcctg gtgccgcgcg 60
   gcagccatat ggctagcatg actggtggac agcaaatgga tccactagtt ctagagcggc 120
   cgc 123
<210> 77
   <211> 29
   <212> DNA
   <213> Homo sapiens
<220>
   <221> misc_binding
   <222> (1)..(29)
   <223> PCR primer for soluble secreted expression construct of human GalNAc-T1 [EBHC121H]
<400> 77
   gcgggatcca ggacttcctg ctggagatg 29
<210> 78
   <211> 28
   <212> DNA
   <213> Homo sapiens
<220>
   <221> misc_binding
   <222> (1)..(28)
   <223> PCR primer for soluble secreted expression construct of human GalNAc-T1 [EBHC107B]
<400> 78
   gcggatcctc agaatatttc tggaaggg 28
<210> 79
   <211> 35
   <212> DNA
   <213> Homo sapiens
<220>
   <221> misc_binding
   <222> (1)..(35)
   <223> PCR primer for soluble secreted expression construct of human GalNAc-T2 [EBHC75D]
<400> 79
   gcggaattct taaaaagaaa gaccttcatc acagc 35
<210> 80
   <211> 28
   <212> DNA
   <213> Homo sapiens
<220>
   <221> misc_binding
   <222> (1)..(28)
   <223> PCR primer for soluble secreted expression construct of human GalNAc-T2 [EBHC68]
<400> 80
   gcggaattcc tactgctgca ggttgagc 28
<210> 81
   <211> 28
   <212> DNA
   <213> Homo sapiens
<220>
   <221> misc_binding
   <222> (1)..(28)
   <223> PCR primer for soluble secreted expression construct of human GalNAc-T3 [EBHC219H]
<400> 81
   gcgggatcca acgatggaaa ggaacatg 28
<210> 82
   <211> 31
   <212> DNA
   <213> Homo sapiens
<220>
   <221> misc_binding
   <222> (1)..(31)
   <223> PCR primer for soluble secreted expression construct of human GalNAc-T3 [EBHC215]
<400> 82
   agcggatcca ggaacactta atcattttgg c 31
<210> 83
   <211> 31
   <212> DNA
   <213> Homo sapiens
<220>
   <221> misc_binding
   <222> (1)..(31)
   <223> PCR primer for soluble secreted expression construct of human GalNAC-T4 [EBHC318]
<400> 83
   gcgggatcct tttcatgcct ccgcaggagc c 31
<210> 84
   <211> 30
   <212> DNA
   <213> Homo sapiens
<220>
   <221> misc_binding
   <222> (1)..(30)
   <223> PCR primer fpr soluble secreted expression construct of human GalNAc-T4 [EBHC307]
<400> 84
   gcgggatccg acgaaagtgc tgttgtgctc 30
<210> 85
   <211> 32
   <212> DNA
   <213> Homo sapiens
<220>
   <221> misc_binding
   <222> (1)..(32)
   <223> PCR primer for soluble secreted expression construct of human GalNAC-T5 [EBHC909]
<400> 85
   gcgggatcct gctttaactg gagggctaga gc 32
<210> 86
   <211> 30
   <212> DNA
   <213> Homo sapiens
<220>
   <221> misc_binding
   <222> (1)..(30)
   <223> PCR primer for soluble secreted expression construct of human GalNAC-T5 [EBHC907]
<400> 86
   gcgggatcca tcagttacac ttcaggcttc 30
<210> 87
   <211> 34
   <212> DNA
   <213> Homo sapiens
<220>
   <221> misc_binding
   <222> (1)..(34)
   <223> PCR primer for soluble secreted expression construct of human GalNAc-T6 [EBHC514H]
<400> 87
   gcgggatccc ctggacctca tgctggaggc catg 34
<210> 88
   <211> 32
   <212> DNA
   <213> Homo sapiens
<220>
   <221> misc_binding
   <222> (1)..(32)
   <223> PCR primer for soluble secreted expression construct of human GalNAc-T6 [EBHC511N]
<400> 88
   agcggatcct ggggatgatc tgggtcctag ac 32
<210> 89
   <211> 32
   <212> DNA
   <213> Homo sapiens
<220>
   <221> misc_binding
   <222> (1)..(32)
   <223> PCR primer for soluble secreted expression construct of human GalNAc-T7 [EBHC1122H]
<400> 89
   gcgaagcttc aggatgaggg aagacagaga tg 32
<210> 90
   <211> 35
   <212> DNA
   <213> Homo sapiens
<220>
   <221> misc_binding
   <222> (1)..(35)
   <223> PCR primer for soluble secreted expression construct of human GalNAc-T7 [EBHC1116H]
<400> 90
   gcgaagcttc tctctaaaca ctatggatct tattc 35
<210> 91
   <211> 33
   <212> DNA
   <213> Homo sapiens
<220>
   <221> misc_binding
   <222> (1)..(33)
   <223> PCR primer for soluble secreted expression construct of human GalNAc-T8 [EBHC1820]
<400> 91
   gcgggatcct ctgaaagaaa gtatgaaatt agc 33
<210> 92
   <211> 30
   <212> DNA
   <213> Homo sapiens
<220>
   <221> misc_binding
   <222> (1)..(30)
   <223> PCR primer for soluble secreted expression construct of human GalNAC-T8 [EBHC1821]
<400> 92
   gcgggatcct cactggctgt tggtctgacc 30
<210> 93
   <211> 34
   <212> DNA
   <213> Homo sapiens
<220>
   <221> misc_binding
   <222> (1)..(34)
   <223> PCR primer for soluble secreted expression construct of human GalNAc-T9 [EBHC1320]
<400> 93
   gcgggatccc tgccgcctgc agggccgctc ccag 34
<210> 94
   <211> 32
   <212> DNA
   <213> Homo sapiens
<220>
   <221> misc_binding
   <222> (1)..(32)
   <223> PCR primer for soluble secreted expression construct of human GalNAC-T9 [EBHC1321]
<400> 94
   gcgggatcct cagtgccgtc ggtgtttgat cc 32
<210> 95
   <211> 31
   <212> DNA
   <213> Homo sapiens
<220>
   <221> misc_binding
   <222> (1)..(31)
   <223> PCR primer for soluble secreted expression construct of human GalNAc-T10 [EBHC2520]
<400> 95
   gcgggatccc cgcgagcggc agcccgacgg c 31
<210> 96
   <211> 30
   <212> DNA
   <213> Homo sapiens
<220>
   <221> misc_binding
   <222> (1)..(30)
   <223> PCR primer for soluble secreted expression construct of human GalNAc-T10 [EBHC2521]
<400> 96
   gcgggatcct cagttcctat tgaatttttc 30
<210> 97
   <211> 31
   <212> DNA
   <213> Homo sapiens
<220>
   <221> misc_binding
   <222> (1)..(31)
   <223> PCR primer for soluble secreted expression construct of human GalNAc-T11 [EBHC629]
<400> 97
   gcgaattcgt gaagtgactc agccacttaa g 31
<210> 98
   <211> 28
   <212> DNA
   <213> Homo sapiens
<220>
   <221> misc_binding
   <222> (1)..(28)
   <223> PCR primer for soluble secreted expression construct of human GalNAc-T11 [EBHC614]
<400> 98
   gcgaattcgt ctctgtcaga cacgtgtc 28
<210> 99
   <211> 32
   <212> DNA
   <213> Homo sapiens
<220>
   <221> misc_binding
   <222> (1)..(32)
   <223> PCR primer for soluble secreted expression construct of human GalNAc-T12 [EBHC1051]
<400> 99
   gcgggatccg gctcggtgct gcgggcgcag cg 32
<210> 100
   <211> 35
   <212> DNA
   <213> Homo sapiens
<220>
   <221> misc_binding
   <222> (1)..(35)
   <223> PCR primer for soluble secreted expression construct of human GalNAC-T12 [EBHC1032]
<400> 100
   gcgggatcct cataacatgc gctctttgaa gaacc 35
<210> 101
   <211> 34
   <212> DNA
   <213> Homo sapiens
<220>
   <221> misc_binding
   <222> (1)..(34)
   <223> PCR primer for soluble secreted expression construct of human GalNAc-T13 [EBHC2000]
<400> 101
   gcgggatccg atgttgcacv vtccccacca cacc 34
<210> 102
   <211> 31
   <212> DNA
   <213> Homo sapiens
<220>
   <221> misc_binding
   <222> (1)..(31)
   <223> PCR primer for soluble secreted expression construct of human GalNAc-T13 [EBHC2002]
<400> 102
   gcgggatcct catcgttcat ccacagcatt g 31
<210> 103
   <211> 32
   <212> DNA
   <213> Homo sapiens
<220>
   <221> misc_binding
   <222> (1)..(32)
   <223> PCR primer for soluble secreted expression construct of Human GalNAc-T14 [EBHC1720]
<400> 103
   gcgggatcct ctgctgcctg cattgagggc tg 32
<210> 104
   <211> 32
   <212> DNA
   <213> Homo sapiens
<220>
   <221> misc_binding
   <222> (1)..(32)
   <223> PCR primer for soluble secreted expression construct of human GalNAC-T14 [EBH21721]
<400> 104
   gcgggatcct catgtgccca aggtcatgtt cc 32
<210> 105
   <211> 31
   <212> DNA
   <213> Homo sapiens
<220>
   <221> misc_binding
   <222> (1)..(31)
   <223> PCR primer for soluble secreted expression construct of human GalNAc-T15 [EBHC412]
<400> 105
   gcgggatccc aagaggaagt tggaggtgcc g 31
<210> 106
   <211> 31
   <212> DNA
   <213> Homo sapiens
<220>
   <221> misc_binding
   <222> (1)..(31)
   <223> PCR primer for soluble secreted expression construct of human GalNAc-T15 [EBHC438]
<400> 106
   gcgggatccc aggggtcctc aagagctcac c 31
<210> 107
   <211> 33
   <212> DNA
   <213> Homo sapiens
<220>
   <221> misc_binding
   <222> (1)..(33)
   <223> PCR primer for soluble secreted expression construct of human GalNAc-T16 [EBHC1913]
<400> 107
   gcgggatccc tactacttat ggcaggacaa ccg 33
<210> 108
   <211> 30
   <212> DNA
   <213> Homo sapiens
<220>
   <221> misc_binding
   <222> (1)..(30)
   <223> PCR primer for soluble secreted expression construct of human GalNAC-T16 [EBHC1912]
<400> 108
   gcgtcatgtg tgtggcaaca gctgccactg 30

## Description

### TECHNICAL FIELD

The present invention relates generally to the biosynthesis, sorting and secretion of mucins, O-glycosylated glycoproteins, and glycoproteins. More specifically, it relates to modulation of the functions of a homologous family of UDP-*N*-acetyl-α-D-galactosamine: polypeptide *N*-acetylgalactosaminyltransferases (GalNAc-transferases), which are generally characterized by the enzyme activity to add *N*-acetylgalactosamine (GalNAc) to the hydroxy group of serine and threonine amino acid residues in peptides, glycopeptides, proteins, and glycoproteins.

In particular, this invention is related to a lectin domain found in the C-terminal region of most GalNAc-transferases, which is structurally and functionally distinct from the catalytic domain of the enzymes and can be expressed in a functional form as a truncated or mutated protein that is enzymatically inactive. Still more particularly, this invention is related to the lectin domains of more than 16 GalNAc-transferases, designated GalNAc-T1 to GalNAc-T16.

Further, this invention concerns a method of screening one or more test substances for the ability to inhibit or modulate polypeptide GalNAc-transferase lectins in a cell-free or cell-based assay, in order to find agents which are effective in binding to one or more lectin domains of polypeptide GalNAc-transferases, for example, selective inhibitors of the binding properties of the above mentioned lectin domains and selective inhibitors of the effects that these lectin domains exert on intracellular transport, transport to cell surface, and secretion of mucins, glycoproteins, and proteins.

Even further, is provided a preferable inhibitor, GalNAcβ-benzyl, which is a novel inhibitor and representative of a novel group of inhibitors which display the common characteristic of selectively inhibiting lectins of polypeptide GalNAc-transferases in direct binding assays and not serve as substrates for other glycosyltransferases involved in O-glycan biosyntheses, while exhibiting inhibitory activity of secretion and intracellular transport of mucins and glycoproteins in cells. GalNAcβ-benzyl and related inhibitors with the same biological functions represent preferable selective inhibitor compared to GalNAcα-benzyl because these do not serve as substrates for glycosyltransferases extending O-glycans and do not provide a general inhibition of the O-glycosylation process in cells.

### BACKGROUND OF THE INVENTION

Mucin-type O-glycosylation, one of the most abundant forms of protein glycosylation, is found on secreted and cell surface associated glycoproteins of all eukaryotic cells except yeast. Mucin-type O-glycans contribute to a number of important molecular functions, including: direct effects on protein conformation, solubility, and stability; specific receptor functions that regulate cell trafficking and cell-cell interactions; and microbial clearance. Mucin-type O-glycans are synthesised in the Golgi through the sequential addition of saccharide residues, first to hydroxyl groups on serines and threonines of a protein core and subsequently to hydroxyl groups on the growing saccharide chains that extend from the protein core. There is great diversity in the structures created by O-glycosylation (hundreds of potential structures), which are produced by the catalytic activity of hundreds of glycosyltransferase enzymes that are resident in the Golgi complex. Diversity exists at the level of the glycan structure and in positions of attachment of O-glycans to protein backbones. Despite the high degree of potential diversity, it is clear that O-glycosylation is a highly regulated process that shows a high degree of conservation among multicellular organisms.

The factors that regulate the attachment of O-glycans to particular protein sites and their extension into specific structures are poorly understood. Longstanding hypotheses in this area propose that mucin-type O-glycosylation occurs in a stochastic manner where structure of acceptor proteins combined with topology and kinetic properties of resident Golgi glycosyltransferases determine the order and degree of glycosylation ¹. This concept does not fully explain the high degree of regulation and specialisation that governs the O-glycosylation process. In particular it is difficult to envision how large mucin molecules with high densities of O-glycans are glycosylated in the Golgi by stochastic mechanisms that also create other sparsely glycosylated proteins.

The first step in mucin-type O-glycosylation is catalysed by one or more members of a large family of UDP-GalNAc: polypeptide *N*-acetylgalactosaminyltransferases (GalNAc-transferases) (EC 2.4.1.41), which transfer GalNAc to serine and threonine acceptor sites ². To date twelve members of the mammalian GalNAc-transferase family have been identified and characterized ³, and several additional putative members of this gene family have been predicted from analysis of genome databases. The GalNAc-transferase isoforms have different kinetic properties and show differential expression patterns temporally and spatially, suggesting that they have distinct biological functions ². Sequence analysis of GalNAc-transferases have led to the hypothesis that these enzymes contain two distinct subunits: a central catalytic unit, and a C-terminal unit with sequence similarity to the plant lectin ricin, designated the "lectin domain" ⁴⁻⁷. Previous experiments involving site-specific mutagenesis of selected conserved residues confirmed that mutations in the catalytic domain eliminated catalytic activity. In contrast, mutations in the "lectin domain" had no significant effects on catalytic activity of the GalNAc-transferase isoform, GalNAc-T1 ⁴. Thus, the C-terminal "lectin domain" was believed not to be functional and not to play roles for the enzymatic functions of GalNAc-transferases ⁴.

However, recent evidence demonstrates that some GalNAc-transferases exhibit unique activities with partially GalNAc-glycosylated glycopeptides. The catalytic actions of at least three GalNAc-transferase isoforms, GalNAc-T4, -T7, and -T10, selectively act on glycopeptides corresponding to mucin tandem repeat domains where only some of the clustered potential glycosylation sites have been GalNAc glycosylated by other GalNAc-transferases ⁸⁻¹¹. GalNAc-T4 and -T7 recognize different GalNAc-glycosylated peptides and catalyse transfer of GalNAc to acceptor substrate sites in addition to those that were previously utilized. One of the functions of such GalNAc-transferase activities is predicted to represent a control step of the density of O-glycan occupancy in mucins and mucin-like glycoproteins with high density of O-glycosylation. It was hypothesized that such sequential actions of multiple GalNAc-transferase isoforms may be required to complete O-glycan attachments to some mucin peptide sequences allowing for detailed control of density.

One example of this is the glycosylation of the cancer-associated mucin MUC1. MUC1 contains a tandem repeat O-glycosylated region of 20 residues (HGVTSAPDTRPAPGSTAPPA) with five potential O-glycosylation sites. GalNAc-T1, -T2, and -T3 can initiate glycosylation of the MUC1 tandem repeat and incorporate at only three sites (HGVTSAPDTRPAPGSTAPPA, GalNAc attachment sites underlined). GalNAc-T4 is unique in that it is the only GalNAc-transferase isoform identified so far that can complete the O-glycan attachment to all five acceptor sites in the 20 amino acid tandem repeat sequence of the breast cancer associated mucin, MUC1. GalNAc-T4 transfers GalNAc to at least two sites not used by other GalNAc-transferase isoforms on the GalNAc₄TAP24 glycopeptide (TAPPAHGVTSAPDTRPAPGSTAPP, GalNAc attachment sites underlined) ¹⁰. An activity such as that exhibited by GalNAc-T4 appears to be required for production of the glycoform of MUC1 expressed by cancer cells where all potential sites are glycosylated ¹². Normal MUC1 from lactating mammary glands has approximately 2.6 O-glycans per repeat ¹³ and MUC1 derived from the cancer cell line T47D has 4.8 O-glycans per repeat ¹². The cancer-associated form of MUC1 is therefore associated with higher density of O-glycan occupancy and this is accomplished by a GalNAc-transferase activity identical to or similar to that of GalNAc-T4.

The specific mechanism by which GalNAc-T4, -T7, and -T10 recognize and function with GalNAc-glycosylated glycopeptides is not known. However, it was originally demonstrated that the GalNAc-glycopeptide specificity exerted by GalNAc-T4 is directed or at least dependent on its lectin domain. A single amino acid substitution in the T4 lectin domain predicted to inactivate its function abolished the GalNAc-glycopeptide specificity of T4 without adversely affecting the basic catalytic mechanism of the transferase ². This suggests that the lectin domain interacts with GalNAc-glycopeptides and confers a novel catalytic function to the enzyme protein. Despite extensive attempts it has in the past not been possible to demonstrate actual binding of the transferase and lectin to sugars and glycopeptides, but it was possible to demonstrate selective inhibition of the GalNAc-glycopeptide activity of GalNAc-T4 using 230 mM concentration of GalNAc ². The present inventors and coworkers demonstrated that mM concentrations of GalNAcα-benzyl can inhibit the lectin mediated GalNAc-glycopeptide substrate specificity of GalNAc-T4 as well as -T7 (PCT WO 01/85215 A2).Polypeptide GalNAc-transferases, which have not displayed apparent GalNAc-glycopeptide specificities, also appear to be modulated by their putative lectin domains (PCT WO 01/85215 A2). Recently, it was found that mutations in the GalNAc-T1 putative lectin domain, similarly to those previously analysed in GalNAc-T4 ², modified the activity of the enzyme in a similar fashion as GalNAc-T4. Thus, while wild type GalNAc-T1 added multiple consequtive GalNAc residues to a peptide substrate with multiple acceptor sites, mutated GalNAc-T1 failed to add more than one GalNAc residue to the same substrate ¹⁴. The mechanism is however not understood.

Glycosylation confers physico-chemical properties including protease resistance, solubility, and stability to proteins ¹⁵⁻¹⁷. Glycosylation furthermore confers changes in immunological responses to proteins and glycoproteins. O-glycosylation on mucins and mucin-like glycoproteins protect these molecules found in the extracellular space and body fluids from degradation. Control of O-glycosylation with respect to sites and number (density) of O-glycan attachments to proteins as well as control of the O-glycan structures made at specific sites or in general on glycoproteins, is of interest for several purposes. Diseased cells e.g. cancer cells often dramatically change their O-glycosylation and the altered glycans and glycoproteins may constitute targets for therapeutic and diagnostic measures ^{18.19}. Mucins functioning in body fluids may have different properties depending on density and structure of O-glycans attached in protec-' tion against disease, including infections by microorganisms. Furthermore, mucins with different glycosylation may change physico-chemical properties including stability and solubility properties that may influence turnover and removal of mucous. A number of lung diseases, e.g. cystic fibrosis, asthma, chronic bronchitis, smokers lungs, are associated with symptomatic mucous accumulation ²⁰⁻²², and it is likely that the nature and structure of mucins play a role in the pathogenesis of such diseases.

Partial inhibitors of O-glycosylation in cells have been reported. Aryl-*N*-acetyl-α-galactosaminides such as benzyl-, phenyl-, and *p*-nitrophenyl-GalNAc were originally found to inhibit the second step in O-glycosylation, the O-glycan processing step, by inhibiting synthesis of core 1 (Galβ1-3GalNAcα1-R) and more complex structures ²³. Benzyl-αGalNAc was also found to inhibit sialylation. It is generally believed that the downstream effects of benzyl-αGalNAc treatment are mediated by substrate competition of biosynthetic glycosylation products of benzyl-αGalNAc. Thus, e.g. the immediate glycosylation product of benzyl-αGalNAc is Galβ1-3GalNAcα-benzyl and this serves as an efficient substrate for the core 1 α2-3sialyltransferase ST3Gal-I ^{24,25}. GalNAcα-benzyl has been the most widely used inhibitor of O-glycosylation, but it has only been used in cell culture as effective treatment concentrations lead to intracellular build-up of vesicles with GalNAca-benzyl products and treated cells change morphology and growth characteristics ²⁶.

Treatment of cells with benzyl-αGalNAc inhibit O-glycan processing and affect apical sorting of some O-glycosylated proteins ²⁷⁻²⁹. The mechanism for this is generally believed to be through inhibition of sialylation ²⁶. Inhibition of mucin secretion has also been observed in culture cells, more specifically HT29 MTX cells, but this effect is not generally found in mucin secreting cells ²⁶.

True inhibitors of O-glycosylation, i.e. inhibitors of the initial O-glycan attachment process governed by polypeptide GalNAc-transferases have not been identified. Benzyl-αGalNAc has been shown to exhibit inhibitory effect on the GalNAc-glycopeptide substrate specificity of some polypeptide GalNAc-transferases in test tube enzyme assays (PCT WO 01/85215 A2), however the effect of this compound *in vivo* on density of O-glycosylation is unknown.

Inhibitors of the initiating step in O-glycosylation could completely or selectively block attachment of O-glycans to O-glycosylation sites in proteins. Compounds inhibiting the catalytic function of a selected subset of the polypeptide GalNAc-transferase family may be predicted to only lead to partial inhibition of O-glycosylation capacity of cells. Proteins with no or little O-glycosylation may have entirely different biological properties than their normal glycosylated counterparts. Complete inhibition of O-glycosylation is not desirable because of the many diverse functions of O-glycans, and it is expected to result in cell death. Selective inhibition of O-glycosylation on the other hand is desirable in many cases such as cancer cells producing glycoproteins and mucins with denser O-glycosylation than normal cells. For example breast cancer cells appear to hyperglycosylate the cancer-associated cell surface mucin MUC1 compared to glycosylation in normal cells ^{12,30}. The overexpression of MUC and hyperglycosylation found in cancer cells are likely to be important for the pathobiology of cancers. Methods of inhibiting the hyperglycosylation of mucins in cancer cells are desirable.

It is apparent from the above that inhibitors in the prior art interfere with O-glycan processing, i.e. the glycosylation process that extend GalNAc residues directly attached to proteins at serine and threonine residues. Existing inhibitors of O-glycosylation are not suitable for therapeutic treatment in mammals including man as they profoundly affect O-glycosylation processing as well as lead to undesired morphological and growth effects on culture cells.

Consequently, there exists a need in the art for therapeutic compounds that display selectively and limited inhibition of O-glycosylation without generally affecting the process of O-glycosylation. The present invention meets these needs, and further presents other related advantages.

### SUMMARY OF THE INVENTION

The present invention provides for isolated polypeptide lectin domains of GalNAc-transferases. The present invention also provides for mammalian GalNAc-transferase lectin domains, lectin-functional variants and fragments thereof. The present invention also provides for human GalNAc-T1-T16 lectin domains having the sequences disclosed in Table III herein.

The present invention also provides for isolated nucleic acids encoding lectin domains of GalNAc-transferases. These nucleic acid sequences can encode for mammalian GalNAc-transferase lectin domains, lectin-functional variants and fragments thereof. The present invention also provides for isolated nucleic acids encoding human GalNAc-T1-T16 (these sequences are disclosed in Table III, herein).

A method of inhibiting or modulating specific functions mediated by lectin domains of polypeptide GalNAc-transferases is disclosed comprising administering an effective amount of an appropriate agent which is effective in binding to one or more lectin domains of polypeptide GalNAc-trmsferases and inhibiting functions mediated by said lectin domains.

A novel selective inhibitor of polypeptide GalNAc-transferase lectin domains is disclosed, which provides a major advancement in that this inhibitor and related inhibitors sharing common characteristics of activity binds lectin domains without serving as acceptor substrate for glycosyltransferases involved in synthesis of O-glycans. For this reason treatment of cells with this and related compounds is not expected to affect O-glycosylation extension including O-glycan branching and sialylation.

A selective GalNAc-transferase lectin inhibitor is represented by the β-anomeric configuration of GalNAc-benzyl, GalNAcβ-benzyl. Additional preferred selective inhibitors including but not limited to the following: GalNAcβ1-R (R represents any aglycone such as benzyl, phenyl, p-nitrophenyl, or umbrelliferyl, without limitation), selective GalNAcβ-peptides, mimetics or compounds derived herefrom. The identifying characteristics of the preferred selective inhibitor is that it retains the binding activity for lectin domains similar to GalNAcβ-benzyl and GalNAcα-benzyl as determined in binding assays as disclosed in the present invention, and lacks the ability to serve as acceptor substrate for glycosyltransferases involved in O-glycosylation as determined by standard enzyme assays widely known by the skilled in the art.

The lectin domains confer unique properties to the GalNAc-transferases including but not limited to selective GalNAc-glycopeptide substrate specificity, as well as binding properties for peptides and carbohydrates to enhance catalytic properties and other functions related to the O-glycosylation process. Methods of selectively blocking the lectin mediated acceptor substrate specificities of such GalNAc-transferase isoforms by βGalNAc and βGalNAc containing structures are disclosed.

The present invention provides a novel method for large scale screening of test substances for the ability to inhibit lectin-mediated activity of polypeptide GalNAc-transferases in a cell-free assay, which comprises:
(i) contacting an isolated polypeptide GalNAc-transferase, an isolated lectin domain from a polypeptide GalNAc-transferase, or a fragment of a polypeptide GalNAc-transferase capable of diplaying lectin-mediated binding on its substrate, with one or more test substances under assay conditions suitable for the detection of said binding ability; and
(ii) measuring whether said lectin-mediated activity is thereby inhibited or modulated by one or more of the substances. A method of screening one or more test substances for the ability to inhibit or modulate intracellular transport and/or cell surface expression of mucins, O-glycosylated glycoproteins, glycoproteins and proteins in a cell-based assay, comprises:

(i) contacting a cell that expresses mucins, O-glycosylated glycoproteins, glycoproteins and proteins, with one or more test substances under assay conditions suitable for the detection of inhibition or modulation of said expression; and
(ii) measuring whether intracellular transport and cell surface expression of said mucins, O-glycosylated glycoproteins, glycoproteins and proteins are thereby inhibited or modulated by one or more of the substances.

A method of screening one or more test substances for the ability to inhibit or modulate secretions of mucins, O-glycosylated glycoproteins, glycoproteins and proteins in a cell-based assay, comprises:
(i) contacting a cell that secretes mucins, O-glycosylated glycoproteins, glycoproteins with one or more test substances under assay conditions suitable for the detection of inhibition or modulation of said secretion; and
(ii) measuring whether secretion of said mucins, O-glycosylated glycoproteins, glycoproteins and proteins are thereby inhibited or modulated by one or more of the substances.

Substances identified as agents which are effective in binding to one or more lectin domains of polypeptide GalNAc-transferases using the above method may e.g. be selected from the group consisting of naturally or non-naturally occurring carbohydrates, peptides, glycopeptides, glycoconjugates and portions and fragments thereof. They may also be found among nucleic acids as well as small organic or inorganic molecules. They include but are not limited to peptides such as soluble peptides including Ig-tailed fusion peptides, members of random peptide libraries and combinatorial chemistry-derived molecular libraries made of D- and/or L-configuration amino acids, phosphopeptides (including members of random or partially degenerate, directed phosphopeptide libraries), antibodies [e.g. polyclonal, monoclonal, humanized, anti-idiotypic, chimeric, single chain antibodies, fragments, (e.g. Fab, F(ab)2, and Fab expression library fragments, and epitope-binding fragments thereof)], and polypeptides. A substance to be used as an agent according to the invention may be an endogenous physiological compound or it may be a natural or synthetic compound.

Agents are useful for changing the density and sites of O-glycan occupancy in mucins and O-linked glycoproteins. Further uses are in changing Golgi-transport and intracellular sorting events conferred by the lectin domains of GalNAc-transferases. For example, inhibitors of lectin domains of GalNAc-transferases may be useful in manipulating disease associated O-glycosylation to augment immunity and to prepare vaccines. Further use may be found in manipulating mucin secretion and O-glycan density in diseases associated with mucous accumulation to decrease secretion and enhance clearance of mucins. Further use may entail modulating O-glycosylation of recombinant glycoproteins by inhibition of polypeptide GalNAc-transferases in host expression cells. Aspects of the present invention will become evident upon reference to the following detailed description and drawings.

### BRIEF DESCRIPTION OF THE DRAWINGS

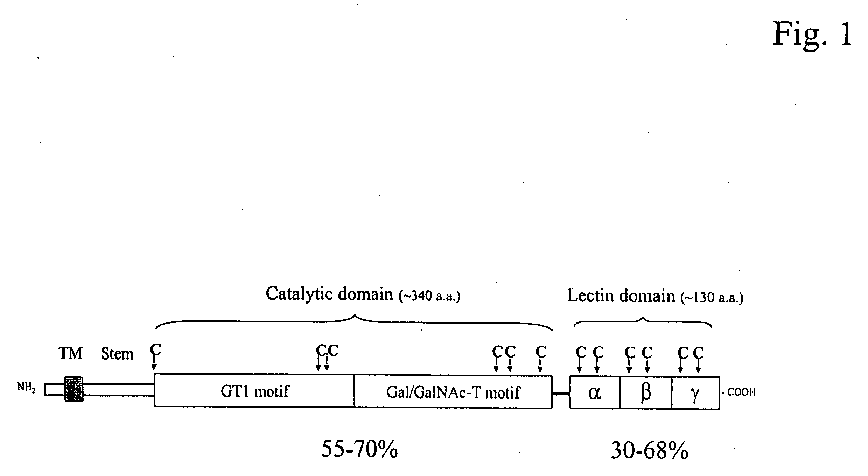

**Figure 1** is a schematic of the predicted domain structure of polypeptide GalNAc-transferases ³¹. Polypeptide GalNAc-transferases are predicted to be type II transmembrane proteins with a cytoplasmic N-terminal tail, a non-cleaved hydrophobic transmembrane retention signal (TM, grey box), a stem region of variable size, a well conserved catalytic unit of approximately 340 amino acid residues, and a poorly conserved C-terminal domain of approximately 130 amino acids which display structural similarity to the lectin ricin and have been designated the "lectin domain". The putative lectin domains consist of α-, β-, and γ- ricin-like repeats. C indicates conserved cysteine residues among sequences.

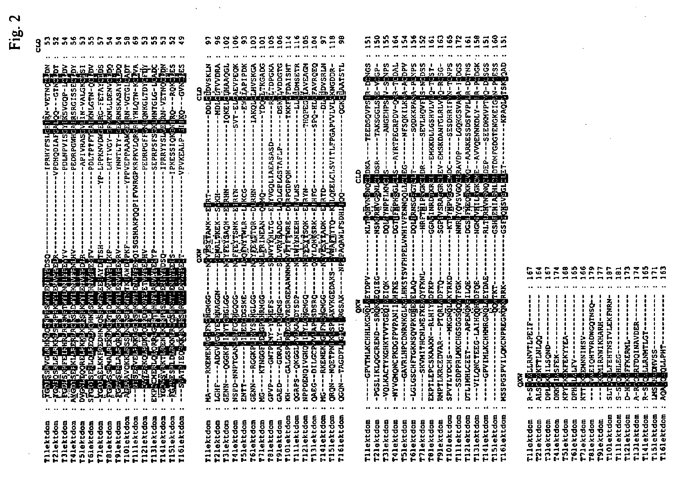

**Figure 2** is a multiple sequence alignment (ClustalW) of putative lectin domains derived from 16 human polypeptide GalNAc-transferases. Potitions of conserved motifs CLD and QxW in the α, β, and γ repeats are indicated. The numbering indicated in the margins reflects numbering of the analysed sequence region of each GalNAc-transferase. Conserved residues are indicated by black box'ing.

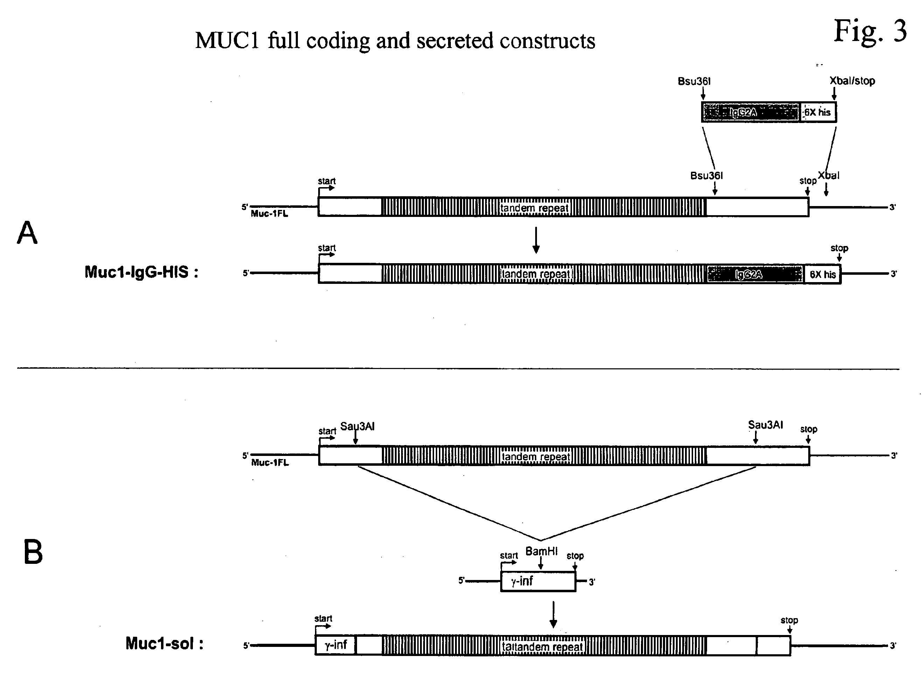

**Figures 3A and B** are schematic representations of human soluble secreted MUC1 expression constructs used for stable transfectants of CHO cells. **Panel A:** IgG2A His-tag was inserted into Bsu36I/Xbal site of MUC1FL, generating a His-tagged MUC1 construct containing the endogenous MUC1 secretion signal peptide. **Panel B:** Muc1FL Sau3AI insert was inserted into the BamHI site of pcDNA-inf., generating a non-tagged MUC1 construct containing the γ-interferon secretion signal peptide.

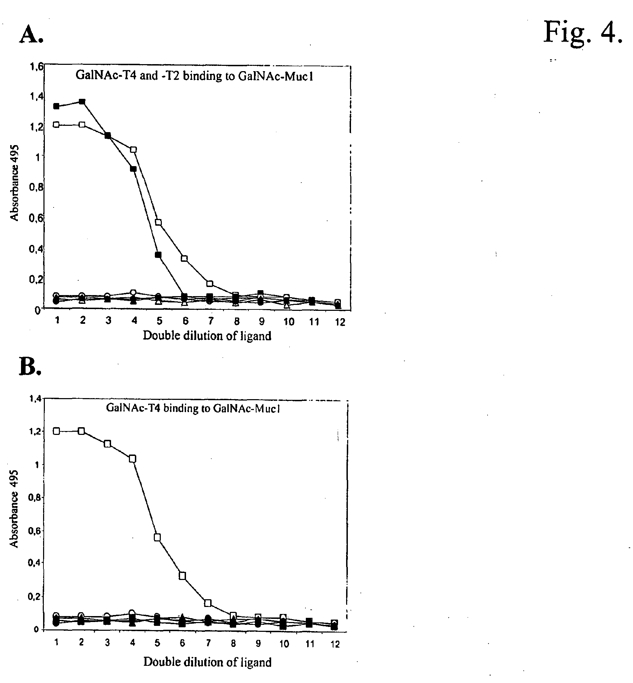

**Figure 4** is a plot of absorbance v. ligand dilution showing GalNAc-transferase binding to GalNAc-MUC1 glycopeptide. A direct binding assay (ELISA) mediated by the lectin domain was developed and validated with soluble secreted GalNAc-T4 and - T2 enzyme proteins. ELISA plates were coated with peptides or glycopeptides at 1 µg/ml, blocked with BSA, and incubated with biotinylated enzymes. After washing, bound enzyme proteins were detected with HRP-Streptavidin as described in detail in *Example* 2. Secreted soluble constructs of GalNAc-transferases which are enzymatically active may bind to (glyco)peptide substrates through their catalytic units as originally described for GalNAc-T2 ³². However, GalNAc-transferase binding to substrates by the catalytic domain requires UDP and divalent cat-ions (binding destroyed by EDTA treatment), in accordance with previous experience ³². **Panel A:** GalNAc-T4 wild type enzyme proteins (□) and GalNAc-T2 (■) selectively bind GalNAc-MUC1 glycopeptides, with no significant binding observed to the unglycosylated peptide (GalNAc-T4 wt (Δ) and GalNAcT2 (A)). **Panel B:** Furthermore, the GalNAc-T4 lectin mutant did not bind to either glycosylated Muc1 (GalNAc-Muc1) (●) or non glycosylated Muc1 (Muc1) (o), whereas GalNAc-T4 wild type binds GalNAc-Muc1 (□) but not nonglcosylated Muc1 (■). Binding was not affected by 10 mM EDTA. Soluble secreted GalNAc-T4 mutant, GalNAc-T4^{459H 2}, in which the lectin domain has been selectively inactivated by a single amino acid substitution, showed no binding demonstrating that the binding observed with the wild type enzyme is mediated through the lectin domain.

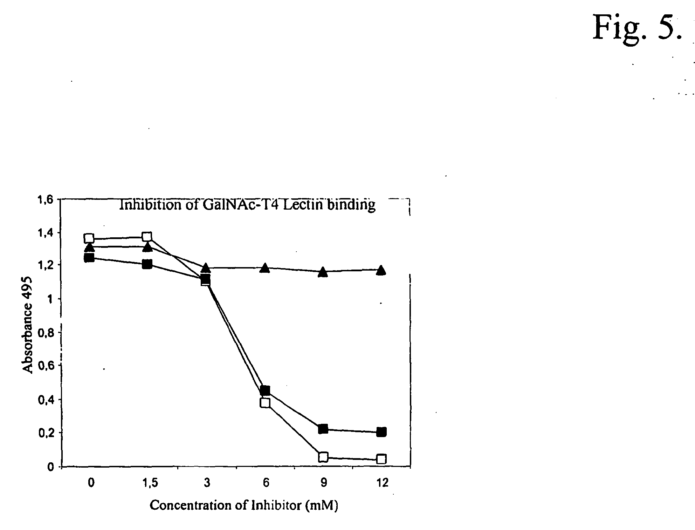

**Figure 5** is a plot (absorbance at 495 v. concentration of inhibitor) showing inhibition of GalNAc-T4 lectin binding. Direct binding assays were performed with preincubation of GalNAc-T4 with inhibitors followed by incubation of GalNAc-T4 in ELISA plates activated with GalNAc-Muc1 as described in detail in *Example* 2. Gal-NAcα-benzyl (■) as well as GalNAcβ-benzyl (□) inhibit at 3-6 mM, whereas the control GlcNAcα-benzyl (▲) showed no inhibition. This demonstrates that the GalNAc-transferase lectin domains show no specificity for the anomeric configuration of GalNAc, and identifies a novel inhibitor, GalNAcβ-benzyl, of GalNAc-transferase lectins.

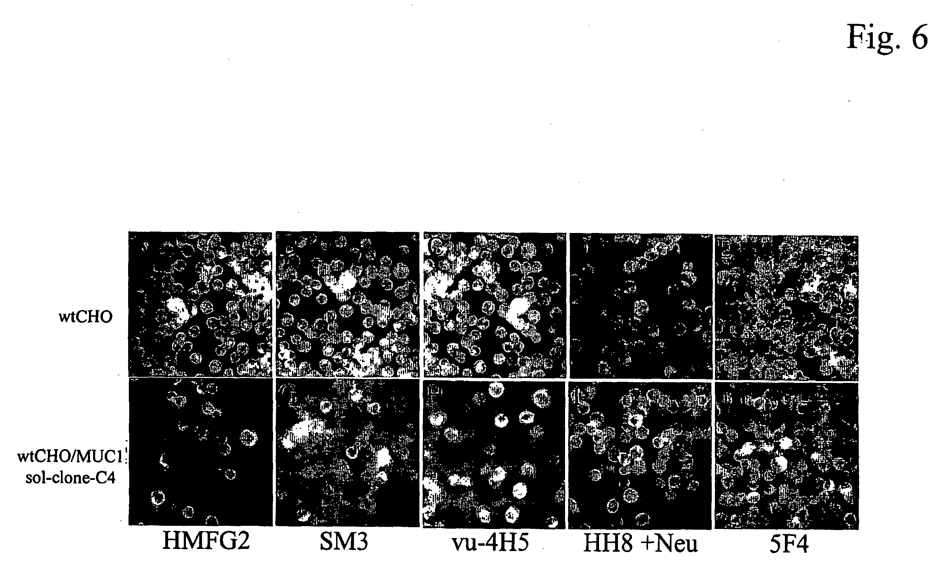

**Figures 6 A-J** are a series of photomicrographs showing immunostaining of wild type CHO and transfected wild type CHO cells with a secreted MUC1 construct. CHO1d1D/MUCsol-cloneD5 was established from this population. MUC1 expression in the cytoplasm of 10-20 % cells is visualized by HMFG2, SM3, and vu-4H5 antibodies. Anti-T antibody HH8 reacted only after neuraminidase pretreatment and the anti-Tn antibody reacted similarly before and after neuraminidase treatment. This suggests that cells grown in GalNAc alone produce mainly the Tn glycoform of MUC1, while cells grown in Gal and GalNAc produce mainly the sialylated T (core 1) glycoforms.

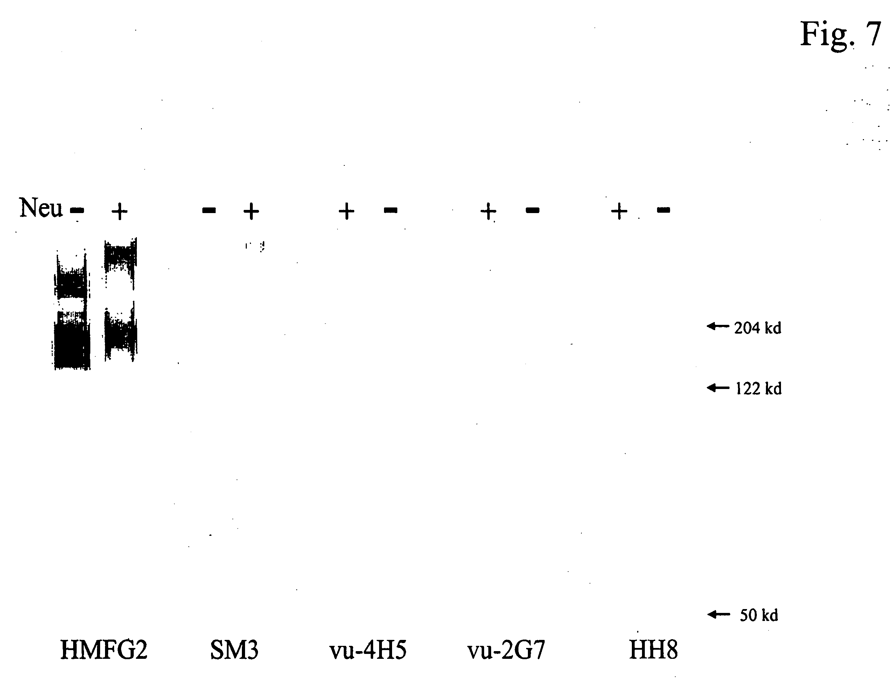

**Figure 7** is a series of SDS-PAGE Western analysis of MUC1 secreted from wild type CHO cells stably transfected cells with a secreted MUC1 construct (CHO1d1D/MUCsol-cloneD5). Neu + indicates pretreatment of samples with neuraminidase as described in *Examples.* Cells were grown in culture medium after 24 or 48 hours analysed directly or after neuraminidase treatment.

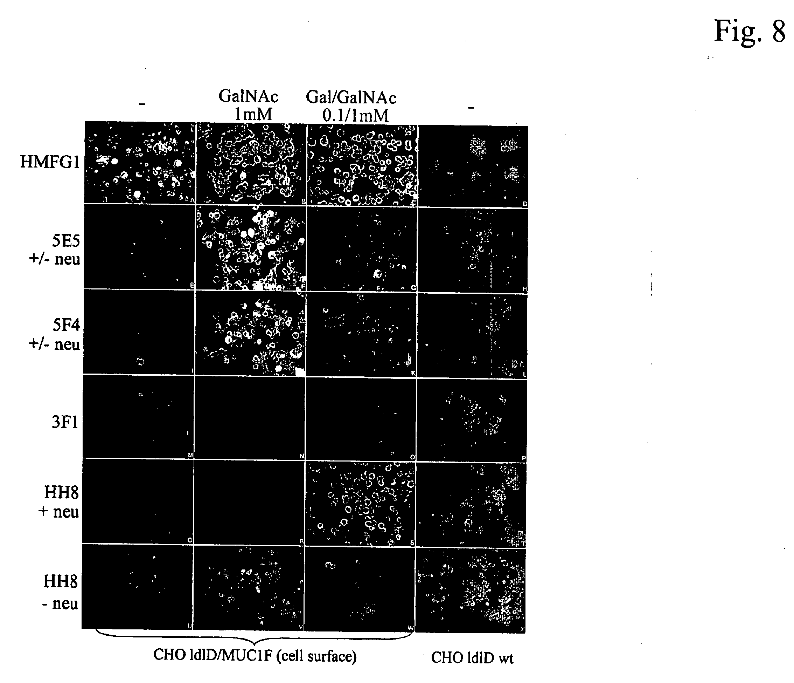

**Figure 8** A-X (left to right from top to bottom) is a series of photomicrographs showing immunostaining of CHO 1d1D cells stably transfected cells with a full coding cell surface secreted MUC1 construct. CHO1d1D/MUC1F-clone2 cells were grown in Optimem medium without and with 1 mM GalNAc, and 1 mM GalNAc plus 0.1 mM Gal for 24-48 hours. Cells were trypsinized, washed, air-dried on cover slides, and immunostained as described in *Examples* with antibodies to MUC1 and T and Tn carbohydrates. Reactivity was evaluated before and after neuraminidase treatment of dried acetone fixed cells. +/- neu indicates that the staining was identical with or without neuraminidase pretreatment.

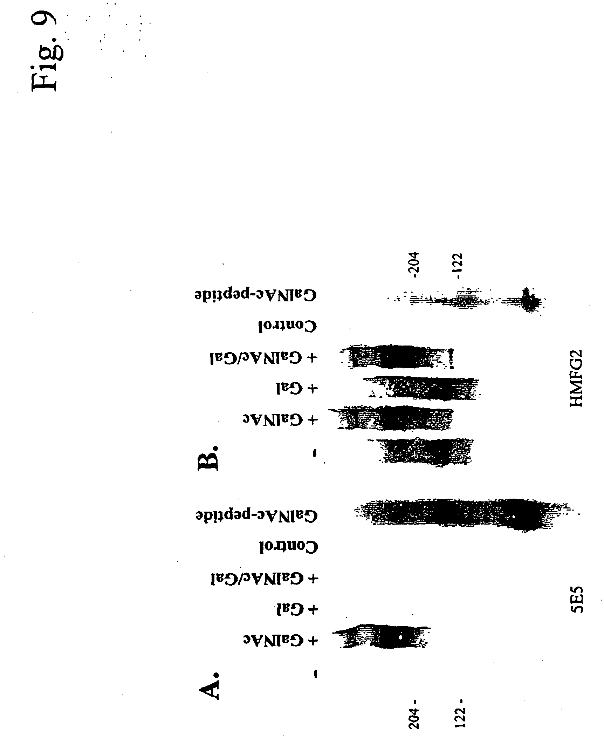

**Figures 9A and B** are SDS-PAGE Western analysis of MUC1 secreted from CHO 1d1D cells stably transfected cells with a secreted MUC1 construct. CHO1d1D/MUCsol-cloneD5 cells were grown in the presence or absence of sugars indicated, and samples of the culture medium analysed directly after 24-48 hours. Positive control (GalNAc-peptide) is a 60-mer MUC1 tandem repeat GalNAc-glycopeptide glycosylated with human polypeptide GalNAc-transferase GalNAc-T2. Lane labeled control includes medium from CHO 1d1D cells. Anti-MUC1 monoclonal antibodies 5E5 and HMFG2 were used.

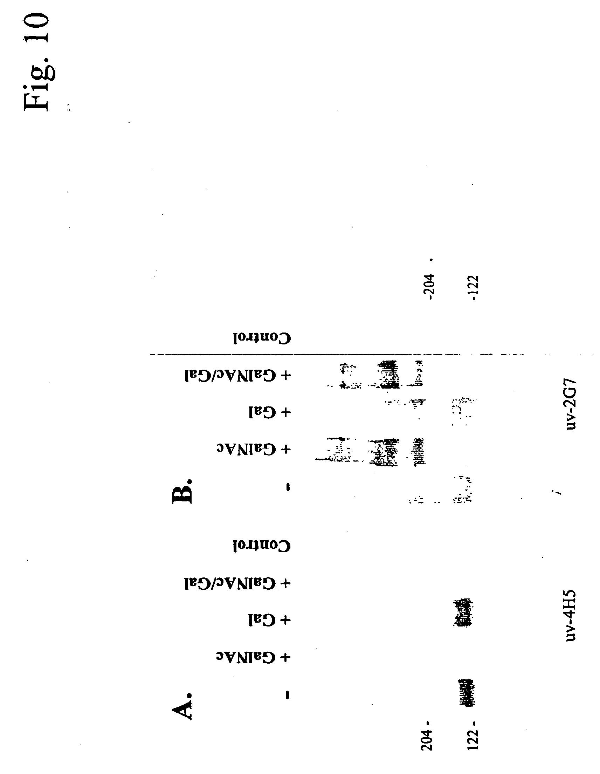

**Figures 10A and B** are SDS-PAGE Western analysis of MUC1 secreted from CHO 1d1D cells stably transfected cells with a secreted MUC1 construct. Same experiment as Figure 9 using anti-MUC1 monoclonal antibodies VU-4H5 and VU-2G7.

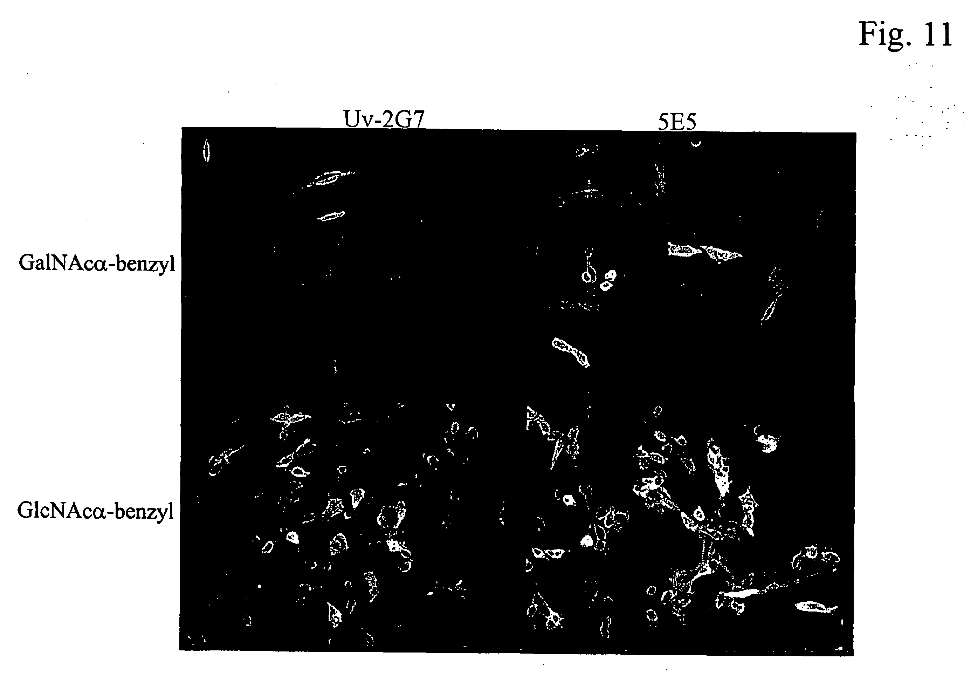

**Figures 11A-D** are a series of photomicrographs (left to right from top to bottom of) anti-MUC1 antibody immunofluorescense staining of CHO 1d1D cells stably transfected with a full coding cell surface MUC1 (CHO1d1D/MUC1F-clone2). Cell grown in the presence of GalNAc were treated with the O-glycosylation inhibitor GalNAcα-benzyl or control GlcNAcα-benzyl. Cells were grown in plates and stained without permeabilization as described in *Example 4.*

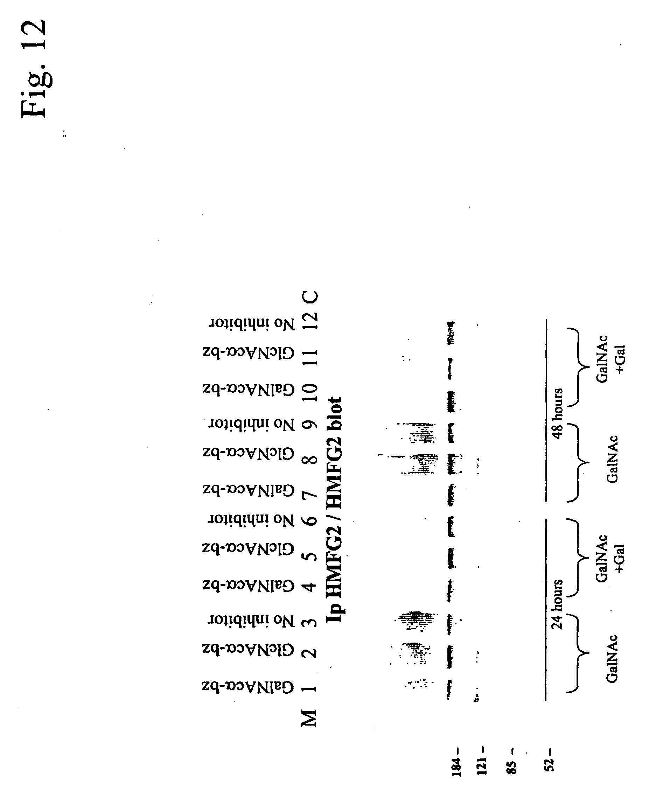

**Figure 12** is an SDS-PAGE Western analysis of GalNAcα-benzyl inhibition of MUC1 expression in CHO 1d1D cells stably transfected with a full coding MUC1 construct. Cells were grown for 24 hours (lanes 1-6) or 48 hours (lanes 7-12) in the presence of 1 mM GalNAc (lanes 1-3 and 7-9) or 1 mM GalNAc and 0.1 mM Gal (lanes 4-6 and 10-12) to limit core O-glycosylation to GalNAcα1-O-Ser/Thr and Galβ1-3GalNAcα1-O-Ser/Thr, respectively. Cells were further treated with 2 mM GalNAcα-benzyl (lanes 1, 4, 7, 10), 2 mM GlcNAcα-benzyl (lanes 2, 5, 8, 11) or no inhibitor (lanes 3, 6, 9, 12). Cells were washed and lysed at 24 or 48 hours and the lysates subjected to immunoprecipitation with monoclonal antibody HMFG2, which broadly recognize MUC1 glycoforms. Immunoprecipitates were analysed by SDS-PAGE and western blot using HMFG2 antibody to detect MUC1 expression. Lane M indicates molecular markers with assigned mw. Lane C includes a control MUC1 180-mer tandem repeat peptide which has been GalNAc-glycosylated with 3 moles GalNAc per repeat using GalNAc-T2. The sharp bands migrating at 100-200 kd are immunoglobulins indicated by IgG. At 24 hours the MUC1 glycoforms expressed by cells grown in GalNAc or Gal and GalNAc migrated similarly, indicating that synthesis of sialylated core 1 O-glycans were time limited (lanes 1-6). At 48 hours, MUC1 glycoforms migrating as higher molecular weight species were expressed by cells grown in Gal and GalNAc (lanes 11-12). Treatment with GalNAcα-benzyl had no significant effect at 24 hours (lanes 1 and 4), but after 48 hours a significant reduction in MUC1 expression was found in cells grown in GalNAc as well as in Gal and GalNAc (lanes 7 and 10). In the latter case a significant shift in migration further confirmed that GalNAcα-benzyl also serves as an inhibitor of O-glycan extension and reduces O-glycosylation to Gal-NAcal-O-Ser/Thr. GlcNAcα-benzyl served as a control and had no effect on MUC1 expression and O-glycosylation compared to untreated cells (lanes 8 and 11).

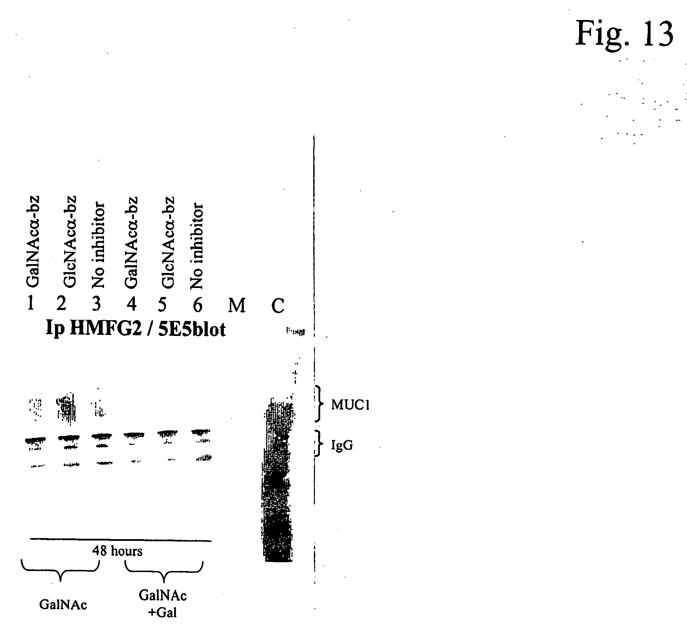

**Figure 13** is a SDS-PAGE Western analysis resulting from the same experiment as in Figure 12, but using a novel monoclonal antibody, 5E5, to visualize MUC1 expression. Cells were grown for 48 hours in the presence of 1 mM GalNAc (lanes 1-3) or 1 mM GalNAc and 0.1 mM Gal (lanes 4-6) to limit core O-glycosylation to GalNAcα1-O-Ser/Thr and Galβ1-3GalNAcα1-O-Ser/Thr, respectively. Cells were further treated with 2 mM GalNAcα-benzyl (lanes 1, 4), 2 mM GlcNAcα-benzyl (lanes 2,5) or no inhibitor (lanes 3, 6). Cells were washed and lysed at 48 hours and the lysates subjected to immunoprecipitation with monoclonal antibody HMFG2, which broadly recognize MUC1 glycoforms. Immunoprecipitates were analysed by SDS-PAGE and western blot using 5E5 antibody, which selectively recognize GalNAc-glycosylated MUC1 expression and show no reactivity with unglycosylated MUC1 peptides. Lanes M and C as described in legend to Figure 12. Treatment with GalNAcα-benzyl produced a significant reduction in MUC1 expression in cells grown in GalNAc as well as in Gal and GalNAc (lanes 1 and 4). In cells grown in Gal and GalNAc (lanes 4-6) only weak expression of MUC1 was detected, but treatment of cells with GalNAcα-benzyl still produced a marked shift in migration to lower molecular weight migrating species

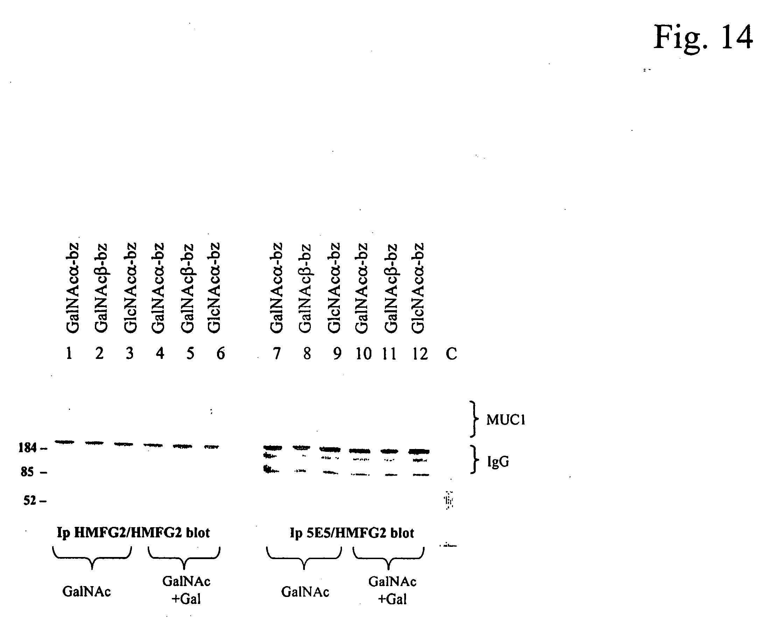

**Figure 14** is an SDS-PAGE Western analysis showing the identification of a novel inhibitor, GalNAcβ-benzyl, which exhibits the same effect on mucin transport as GalNAcα-benzyl, but does not affect O-glycan extension and O-glycosylation in general. CHO 1d1D cells stably transfected with a full coding MUC1 construct were grown for 36 hours in the presence of 1 mM GalNAc (lanes 1-3 and 7-9) or 1 mM GalNAc and 0.1 mM Gal (lanes 4-6 and 10-12) to limit core O-glycosylation to GalNAcα1-O-Ser/Thr and Galβ1-3GalNAcα1-O-Ser/Thr, respectively. Cells were further treated with 2 mM GalNAcα-benzyl (lanes 1, 4, 7, 10), 2 mM GalNAcβ-benzyl (lanes 2, 5, 8, 11) or 2 mM GlcNAcα-benzyl (lanes 3, 6, 9, 12). Cells were washed and lysed at 36 hours and the lysates subjected to immunoprecipitation with monoclonal antibodies HMFG2 (lanes 1-6) or 5E5 (lanes 7-12). Immunoprecipitates were analysed by SDS-PAGE and western blot using HMFG2 antibody. Lanes M and C as described in legend to Figure 7. Treatment with GalNAcβ-benzyl produced the same or better reduction in MUC1 expression as treatment with GalNAcα-benzyl in cells grown in GalNAc as well as in Gal and GalNAc (lanes 2, 5, 8). In cells grown in Gal and GalNAc (lanes 4-6) MUC1 expression was reduced with GalNAcβ-benzyl treatment (lane 5), but in contrast to cells treated with GalNAcα-benzyl (lane 4), GalNAcβ-benzyl produced no change in the migration of MUC1 demonstrating that this inhibitor does not affect O-glycosylation. The lack of immunoprecipitation of MUC1 by 5E5 in cells grown in Gal and GalNAc (lanes 10-12) indicates that MUC1 is glycosylated with more complex structures than Gal-NAcα1-O-Ser/Thr as recognized by this antibody.

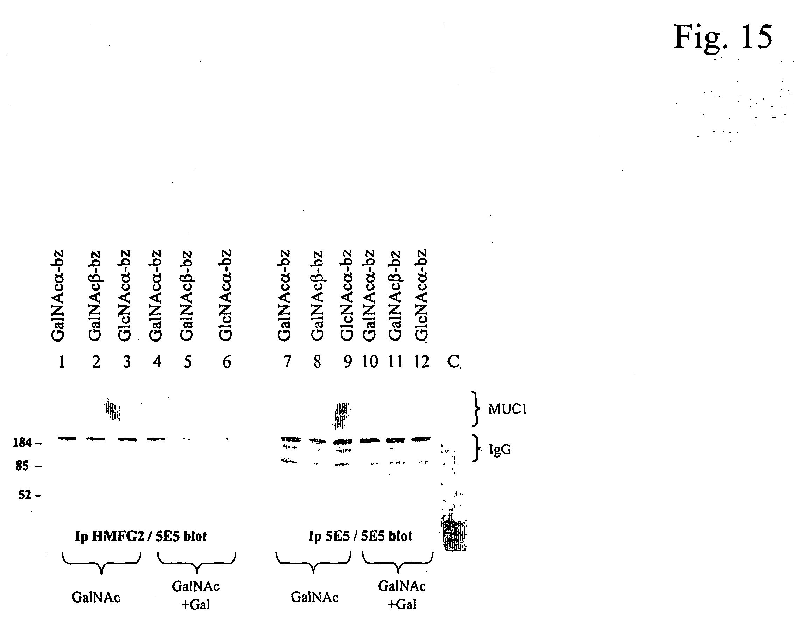

**Figure 15** is an SDS-PAGE Western analysis resulting from the same experiment as Figure 14, except that expression is visualized by the monoclonal antibody 5E5. Cells were grown for 36 hours in the presence of 1 mM GalNAc (lanes 1-3 and 7-9) or 1 mM GalNAc and 0.1 mM Gal (lanes 4-6 and 10-12) to limit core O-glycosylation to GalNAcα1-O-Ser/Thr and Galβ1-3GalNAcα1-O-Ser/Thr, respectively. Cells were further treated with 2 mM GalNAcα-benzyl (lanes 1, 4, 7, 10), 2 mM GalNAcβ-benzyl (lanes 2, 5, 8, 11) or 2 mM GlcNAcα-benzyl (lanes 3, 6, 9, 12). Cells were washed and lysed at 36 hours and the lysates subjected to immunoprecipitation with monoclonal antibodies HMFG2 (lanes 1-6) or 5E5 (lanes 7-12). Immunoprecipitates were analysed by SDS-PAGE and western blot using 5E5 antibody. Lanes M and C as described in legend to Figure 12. Treatment with GalNAcβ-benzyl produced the same or better reduction in MUC1 expression as treatment with GalNAcα-benzyl in cells grown in GalNAc (lanes 2 and 8). The lack of immunostaining of MUC1 by 5E5 in cells grown in Gal and GalNAc indicates that MUC1 is glycosylated with more complex structures than GalNAcα1-O-Ser/Thr as recognized by this antibody.

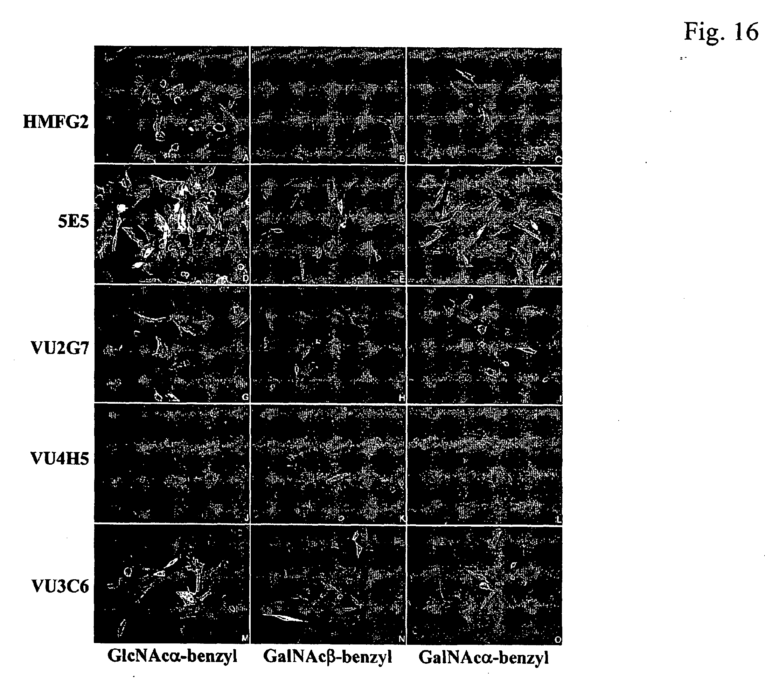

**Figures 16A-O** (left to right from top to bottom) is a series of photomicrographs showing that the main O-glycan phenotype of CHO 1d1D cells grown in Gal and GalNAc is sialylated T and that the 5E5 antibody does not react with MUC1, with T or silaylated T glycoforms of MUC1.

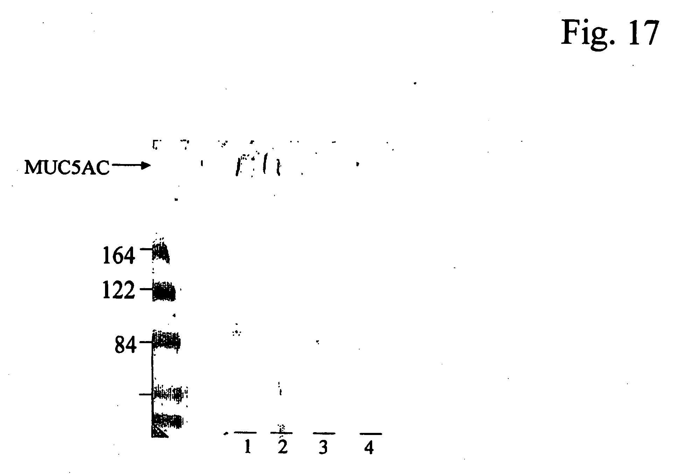

**Figure 17** illustrates SDS-PAGE Western analysis of MUC5AC secretion from HT29MTX cells using polyclonal anti-MUC5AC antibody LUMS-1. In order to evaluate GalNAcβ-benzyl as an inhibitor of mucin glycosylation and secretion we used the HT-29 metotrexate (MTX) cultured colon carcinoma cells of mucin secreting phenotype. HT-29 MTX cells have a goblet cell like phenotype with constitutive production of MUC5AC. Cells were continuously grown up to 21 days in the presence of 5 mM GalNAcα-benzyl, 5 mM GalNAcβ-benzyl, 5 mM GlcNAcα-benzyl and without inhibitor. SDS-PAGE Western blotting of media (10 µL) using the polyclonal anti-MUC5AC antibody LUM5-1 detected MUC5AC in media from untreated cells (Lane 1) and cells treated with GlcNAcα-benzyl (Lane 2). In striking contrast, MUC5AC was not detected in the media from cells treated with GalNAcα-benzyl (Lane 3) or GalNAcβ-benzyl (Lane 4). Results shown are from media collected at 14 days.

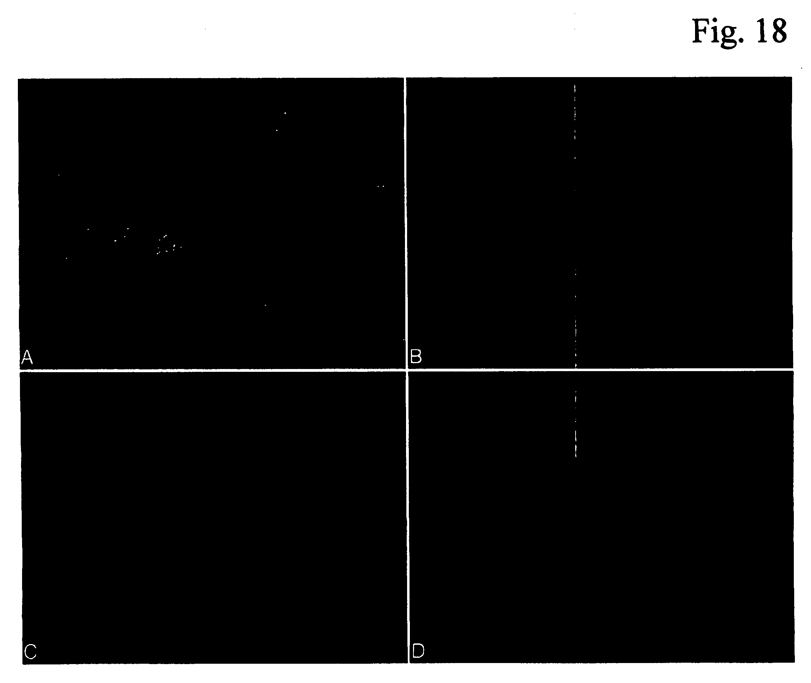

**Figure 18** illustrates immuno-fluorescence staining of HT29MTX cells treated with inhibitors as indicated and stained with the anti-MUC5AC monoclonal antibody, CLH2. Treatment of cells with GalNAcα-benzyl (Panel A) induced a storage phenotype with increased intracellular staining of MUC5AC. Cells treated with GalNAcβ-benzyl (Panel B) did not lead to a storage disease phenotype, and furthermore a clearly diminished intracellular staining of MUC5AC was found compared to cells treated with GlcNAcα-benzyl (Panel C) and untreated cells (Panel D).

### DETAILED DESCRIPTION OF THE INVENTION

### 1. Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

As used herein, the term "about" or "approximately" means within 50% of a given value, preferably within 20%, more preferably within 10%, more preferably still within 5%, and most preferably within 1% of a given value. Alternatively, the term "about" or "approximately" means that a value can fall within a scientifically acceptable error range for that type of value, which will depend on how qualitative a measurement can be given the available tools. "About" or "approximately" may define a distribution around a mean value, rather than a single value.

***Molecular Biology Definitions.*** In accordance with the present invention, there may be employed conventional molecular biology, microbiology and recombinant DNA techniques within the skill of the art. Such techniques are explained fully in the literature. See, for example, Sambrook, Fitsch & Maniatis, Molecular Cloning: A Laboratory Manual, Second Edition (1989) Cold Spring Harbor Laboratory Press, Cold Spring Harbor, New York (referred to herein as "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization (B.D. Hames & S.J. Higgins, eds. 1984); Animal Cell Culture (R.I. Freshney, ed. 1986); Immobilized Cells and Enzymes (IRL Press, 1986); B.E. Perbal, A Practical Guide to Molecular Cloning (1984); F.M. Ausubel et al. (eds.), Current Protocols in Molecular Biology, John Wiley & Sons, Inc.(1994).

"Nucleic acid" or "polynucleotide" as used herein refers to purine- and pyrimidine-containing polymers of any length, either polyribonucleotides or polydeoxy-ribonucleotides or mixed polyribo-polydeoxyribo nucleotides. This includes single-and double-stranded molecules, i.e., DNA-DNA, DNA-RNA and RNA-RNA hybrids, as well as "protein nucleic acids" (PNA) formed by conjugating bases to an amino acid backbone. This also includes nucleic acids containing modified bases (see below).

"Complementary DNA or cDNA" as used herein refers to a DNA molecule or sequence that has been enzymatically synthesised from the sequences present in an mRNA template, or a clone of such a DNA molecule. A "DNA Construct" is a DNA molecule or a clone of such a molecule, either single- or double-stranded, which has been modified to contain segments of DNA that are combined and juxtaposed in a manner that would not otherwise exist in nature. By way of non-limiting example, a cDNA or DNA which has no introns are inserted adjacent to, or within, exogenous DNA sequences.

A plasmid or, more generally, a vector, is a DNA construct containing genetic information that may provide for its replication when inserted into a host cell. A plasmid generally contains at least one gene sequence to be expressed in the host cell, as well as sequences that facilitate such gene expression, including promoters and transcription initiation sites. It may be a linear or closed circular molecule.

Nucleic acids are "hybridizable" to each other when at least one strand of one nucleic acid can anneal to another nucleic acid under defined stringency conditions. Stringency of hybridization is determined, e.g., by a) the temperature at which hybridization and/or washing is performed, and b) the ionic strength and polarity (e.g., formamide) of the hybridization and washing solutions, as well as other parameters. Hybridization requires that the two nucleic acids contain substantially complementary sequences; depending on the stringency of hybridization, however, mismatches may be tolerated. Typically, hybridization of two sequences at high stringency (such as, for example, in an aqueous solution of 0.5X SSC, at 65°C) requires that the sequences exhibit some high degree of complementarity over their entire sequence. Conditions of intermediate stringency (such as, for example, an aqueous solution of 2X SSC at 65°C) and low stringency (such as, for example, an aqueous solution of 2X SSC at 55°C), require correspondingly less overall complementarily between the hybridising sequences. Hybridization stringency has been defined in numerous publication known to the skilled in the art (Meinkoth and Wahl, Anal.Biochem. 138,267-284, 1984; Maniatis et al., Molecular cloning: a laboratory manual. Cold Spring Harbor Laboratory Press, 1989; J.Q.Zhang, Eur.J.Biochem. 239,835-841:1996; M.Friedman-Einat, General and Comparative Endocrinology 115,354-363:1999: M.Szabo, J.Bacteriology, 1544-1553:1995; S.Sau, J.Bacteriology, 21182126, 1996). Nucleic acids are "hybridizable" to each other when at least one strand can anneal to another nucleic acid under defined stringency conditions. High stringency hybridization is defined as 42°C over night hybridization under standard conditions (Martiatis et al., Molecular cloning: a laboratory manual. Cold Spring Harbor Laboratory Press, 1989), followed by 5 washes with 2XSSC, 0.1%SDS at 42°C, once with 0.5XSSC, 0.1%SDS at 55°C and once with 0.1XSSC, 0.1%SDS at 55°C. (1XSSC is 0.15M NaCl, 0.015M Na citrate). Northern and Southern nucleic acid blotting hybridization techniques, especially for the purpose of investigating hybridization specificity, is well known to those skilled in the field of the invention.

An "isolated" nucleic acid or polypeptide as used herein refers to a component that is removed from its original environment (for example, its natural environment if it is naturally occutring). An isolated nucleic acid or polypeptide contains less than about 50%, preferably less than about 75%, and most preferably less than about 90%, of the cellular components with which it was originally associated.

A "probe" refers to a nucleic acid that forms a hybrid structure with a sequence in a target region due to complementarily of at least one sequence in the probe with a sequence in the target region.

A nucleic acid that is "derived from" a designated sequence refers to a nucleic acid sequence that corresponds to a region of the designated sequence. This encompasses sequences that are homologous or complementary to the sequence, as well as "sequence-conservative variants" and "function-conservative variants". Sequence-conservative variants are those in which a change of one or more nucleotides in a given codon position results in no alteration in the amino acid encoded at that position.

Function-conservative variants of polypeptide GalNAc-transferases are those in which a given amino acid residue in the polypeptide has been changed without altering the overall conformation and enzymatic activity (including substrate specificity) of the native polypeptide; these changes include, but are not limited to, replacement of an amino acid with one having similar physico-chemical properties.This includes but is not limited to, replacement of an amino acid with one having similar structural or physical properties, including polar or non-polar character, size, shape and charge (see, *e.g.*, **Table A**).

A "polypeptide" is a chain of chemical building blocks called amino acids that are linked together by chemical bonds called "peptide bonds". The term "protein" refers to polypeptides that contain the amino acid residues encoded by a gene or by a nucleic acid molecule (e.g., an mRNA or a cDNA) transcribed from that gene either directly or indirectly. Optionally, a protein may lack certain amino acid residues that are encoded by a gene or by an mRNA. For example, a gene or mRNA molecule may encode a sequence of amino acid residues on the N-terminus of a protein (i.e., a signal sequence) that is cleaved from, and therefore may not be part of, the final protein. A protein or polypeptide, including an enzyme, may be a "native" or "wild-type", meaning that it occurs in nature; or it may be a "mutant", "variant" or "modified", meaning that it has been made, altered, derived, or is in some way different or changed from a native protein or from another mutant.

A "mutation" means any process or mechanism resulting in a mutant protein, enzyme, polypeptide, polynucleotide, gene, or cell. This includes any mutation in which a protein, enzyme, polynucleotide, or gene sequence is altered, and any detectable change in a cell arising from such a mutation. The altered protein, enzyme, polypeptide or polynucleotide is a Amutant@, also called a Avariant.@ Typically, a mutation occurs in a polynucleotide or gene sequence, by point mutations (substitutions), deletions, or insertions of single or multiple nucleotide residues. A mutation includes polynucleotide alterations arising within a protein-encoding region of a gene as well as alterations in regions outside of a protein-encoding sequence, such as, but not limited to, regulatory or promoter sequences. A mutation in a gene can be "silent", *i.e.,* not reflected in an amino acid alteration upon expression, leading to a "sequence-conservative" variant of the gene. This generally arises when one amino acid corresponds to more than one codon. Table A outlines which amino acids correspond to which codon(s).

Thus, due to the degeneracy of the genetic code, any three-nucleotide codon that encodes the GalNAc-transferase lectin domain polypeptides described herein is within the scope of the invention.

The terms Amutant@ and Avariant@ may also be used to indicate a modified or altered gene, DNA or RNA sequence, enzyme, cell, *etc., i.e.,* any kind of mutant. Such changes also include changes in the promoter, ribosome binding site, etc.

As outlined above, amino acid substitutions are generally based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

In addition, modifications, which do not normally alter the primary sequence of the GalNAc-transferase lectin domain polypeptides, include in vivo or in vitro chemical derivatization of polypeptides, e.g., acetylation, methylation, or carboxylation. Also included as variant polypeptides of this invention are these polypeptides modified by glycosylation, e.g., those made by modifying the glycosylation patterns of a polypeptide during its synthesis and processing or in further processing steps; or by exposing the polypeptide to enzymes which affect glycosylation, such as mammalian glycosylating or deglycosylating enzymes. Also embraced as variant polypeptides are the above-identified mutagenized sequences, which have phosphorylated amino acid residues, e.g., phosphotyrosine, phosphoserine, or phosphothreonine.

**TABLE A**

| Amino Acids, Corresponding Codons, and Functionality/Property | | | |
|---|---|---|---|
| Amino Acid | SLC | DNA codons | Side Chain Property |
| Isoleucine | I | ATT,ATC,ATA | Hydrophobic |
| Leucine | L | CTT, CTC, CTA, CTG, TTA, TTG | Hydrophobic |
| Valine | V | GTT, GTC, GTA, GTG | Hydrophobic |
| Phenylalanine | F | TTT, TTC | Aromatic side chain |
| Methionine | M | ATG | Sulphur group |
| Cysteine | C | TGT, TGC | Sulphur group |
| Alanine | A | GCT, GCC, GCA, GCG | Hydrophobic |
| Glycine | G | GGT, GGC, GGA, GGG | Hydrophobic |
| Proline | P | CCT, CCC, CCA, CCG | Secondary amine |
| Threonine | T | ACT, ACC, ACA, ACG | Aliphatic hydroxyl |
| Serine | S | TCT, TCC, TCA, TCG, AGT, AGC | Aliphatic hydroxyl |
| Tyrosine | T | TAT, TAC | Aromatic side chain |
| Tryptophan | W | TGG | Aromatic side chain |
| Glutamine | Q | CAA, CAG | Amide group |
| Asparagine | N | AAT, AAC | Amide group |
| Histidine | H | CAT, CAC | Basic side chain |
| Glutamic acid | E | GAA, GAG | Acidic side chain |
| Aspartic Acid | D | GAT, GAC | Acidic side chain |
| Lysine | K | AAA, AAG | Basic side chain |
| Arginine | R | CGT, CGC, CGA, CGG, AGA, AGG | Basic side chain |
| Stop codons | Stop | TAA, TAG, TGA | - |

As referred to herein, "sequence similarity" means the extent to which nucleotide or protein sequences are related. The extent of similarity between two sequences can be based on percent sequence identity and/or conservation. Amino acids other than those indicated as conserved may differ in a protein or enzyme so that the percent protein or amino acid sequence similarity between any two proteins of similar function may vary and can be, for example, at least 70%, preferably 75%, more preferably 80%, even more preferably 85%, and most preferably at least 90%, as determined according to an alignment scheme.

"Sequence identity" herein means the extent to which two nucleotide or amino acid sequences are invariant.

"Sequence alignment" means the process of lining up two or more sequences to achieve maximal levels of sequence identity (and, in the case of amino acid sequences, conservation), e.g., for the purpose of assessing the degree of sequence similarity. Numerous methods for aligning sequences and assessing similarity and/or identity are known in the art such as, for example, the ClustalW method, the Cluster Method, wherein similarity is based on the MEGALIGN algorithm, as well as BLASTN, BLASTP, and FASTA (Lipman and Pearson, 1985; Pearson and Lipman, 1988). When using all of these programs, the preferred settings are those that result in the highest sequence similarity.

The term Ahost cell@ means any cell of any organism that is selected, modified, transformed, grown, or used or manipulated in any way, for the production of a substance by the cell, for example the expression by the cell of a gene, a DNA or RNA sequence, a protein or an enzyme.

A "donor substrate" is a molecule recognised by, e.g., a polypeptide GalNAc-transferees and that contributes a *N*-acetylgalactosamine moiety for the transferase reaction. For polypeptide GalNAc-transferases, a donor substrate is UDP-N-acetylgalactosamine or with some GalNAc-transferase isoforms UDP-galactose. An "acceptor substrate" is a molecule, preferably a peptide, protein, glycopeptide, and glycoprotein, that is recognised by, e.g., a polypeptide GalNAc-transferase and that is the target for the modification catalysed by the transferase, i.e., receives the carbohydrate moiety. For polypeptide GalNAc-transferases, acceptor substrates include without limitation peptides, proteins, glycopeptides, and glycoproteins.

The term "agonist" refers to a molecule that increases the amount of, or prolongs the duration of, the activity of the polypeptide. The term "enhancer" refers to a molecule that similarly increases the amount of, or prolongs the duration of, the activity of the polypeptide. The term "antagonist" refers to a molecule, which decreases the biological or immunological activity of the polypeptide. The term "inhibitor" similarly refers to a molecule, which decreases the biological or immunological activity of the polypeptide. Agonists, antagonists, and inhibitors may include proteins, nucleic acids, carbohydrates, or any other molecules that associate with a polypeptide GalNAc-transferase.

The term "agent" includes small molecules, peptide mimetics and polypeptides.

"Mimetics" of GalNAc-transferase lectin-domain inhibitors are molecules that functionally mimic the structure or function of a GalNAc-transferase lectin-domain inhibitor. Molecular mimetics include, but are not limited to: small organic compounds; nucleic acids and nucleic acid derivatives; saccharides or oligosaccharides; peptide mimetics including peptides, proteins, and derivatives thereof, such as peptides containing non-peptide organic moieties, synthetic peptides which may or may not contain amino acids and/or peptide bonds, but retain the structural and functional features of a peptide ligand; pyrrolidines; peptoids and oligopeptoids which are molecules comprising N-substituted glycine, such as those described by Simon et al. (1992) Proc. Natl. Acad. Sci. USA 89:9367.

The human N-acetylgalactosaminyltransferase T1 gene (*GALNT1*) has been described previously ³². The sequence of the *GALNT1* mRNA and the sequence of the GalNAc-T1 polypeptide have been submitted to GenBank/EBI Data Bank and assigned accession numbers X85018 and CAA59380, respectively.

The human N-acetylgalactosaminyltransferase T2 gene (*GALNT2*) has been described previously ³². The sequence of the *GALNT2* mRNA and the sequence of the GalNAc-T2 polypeptide have been submitted to GenBank/EBI Data Bank and assigned accession numbers X85019 and CAA59381, respectively.

The human N-acetylgalactosaminyltransferase T3 gene (*GALNT3*) has been described previously ³³. The sequence of the *GALNT3* mRNA and the sequence of the GalNAc-T3 polypeptide have been submitted to GenBank/EBI Data Bank and assigned accession numbers X92689 and CAA63371, respectively.

The human N-acetylgalactosaminyltransferase T4 gene (*GALNT4*) has been described previously ¹⁰. The sequence of the *GALNT4* mRNA and the sequence of the GalNAc-T4 polypeptide have been submitted to GenBank/EBI Data Bank and assigned accession numbers Y08564 and CAA69875, respectively.

The human N-acetylgalactosaminyltransferase T5 gene (*GALNT5*) has been described previously ⁸. The sequence of the *GALNT5* mRNA and the sequence of the GalNAc-T5 polypeptide have been submitted to GenBank/EBI Data Bank and assigned accession numbers AJ245539 and CAB65104, respectively.

The human N-acetylgalactosaminyltransferase T6 gene (*GALNT6*) has been described previously ³⁴. The sequence of the *GALNT6* mRNA and the sequence of the GalNAc-T6 polypeptide have been submitted to GenBank/EBI Data Bank and assigned accession numbers AJ133523 and CAB55325, respectively.The human N-acetylgalactosaminyltransferase T7 gene (*GALNT7*) has been described previously ⁸. The sequence of the *GALNT7* mRNA and the sequence of the GalNAc-T7 polypeptide have been submitted to GenBank/EB1 Data Bank and assigned accession numbers AJ002744 and CAB60270, respectively.

The human N-acetylgalactosaminyltransferase T8 gene (*GALNT8*) has been described previously ³⁵. The sequence of the *GALNT8* mRNA and the sequence of the GalNAc-T8 polypeptide have been submitted to GenBank/EBI Data Bank and assigned accession numbers AJ271385 and CAB89199, respectively.

The human N-acetylgalactosaminyltransferase T9 gene (*GALNT9*) has been described previously ³⁶. The sequence of the *GALNT9* mRNA and the sequence of the GalNAc-T8 polypeptide have been submitted to GenBank/EBI Data Bank and assigned accession numbers AB040672 and BAB 13699, respectively.

The human N-acetylgalactosaminyltransferase T10 nucleic acid sequence (*GALNT10*) and the sequence of the encoded GalNAc-T10 polypeptide have been submitted to GenBank/EBI Data Bank. The nucleic acid accession number is AJ505950.

The human N-acetylgalactosaminyltransferase T11 gene (*GALNT11*) has been described previously ³. The sequence of the *GALNT11* mRNA and the sequence of the GalNAc-T11 polypeptide have been submitted to GenBank/EBI Data Bank and assigned accession numbers Y12434 and CAC79625, respectively.

The human N-acetylgalactosaminyltransferase T12 nucleic acid sequence (*GALNT12*) and the sequence of the GalNAc-T12 polypeptide have been submitted to GenBank/EBI Data Bank. The nucleic acid accession number is AJ132365. This sequence is disclosed herewith.

The human N-acetylgalactosaminyltransferase T13 nucleic acid sequence *(GALNT13*) and the sequence of the GalNAc-T13 polypeptide have been submitted to GenBank/EBI Data Bank. The nucleic acid accession number is AR153422.

Expression to produce enzymatically-active polypeptide GalNAc-transferases can be carried out in any number of conventional expression systems familiar to those skilled in the art. In one embodiment, GalNAc-transferases are expressed in a secreted soluble form, which can be recovered from the culture medium. Such secreted enzymes lack the N-terminal cytoplasmic tail and transmembrane retention sequence, and have N-terminal sequence starting in the predicted stem region (depicted domain structures of polypeptide GalNAc-transferases shown in Figure 1). The boundaries of the stem is N-terminally defined by the hydrophobic signal sequence, while the C-terminal boundary is less clearly defined but limited to the conserved catalytic unit of the enzymes as defined by multiple sequence alignments. For some isoforms including GalNAc-T2 the N-terminal sequence have been determined in naturally occurring soluble proteins derived from proteolytic cleavage ³². In another embodiment, host cells (e.g. CHO cells) are engineered to express full coding GalNAc-transferases and glycosylate substrates in vivo in host cells.

Expression to produce functional lectin domains of polypeptide GalNAc-transferases without the catalytic unit (or activity) can be carried out in any number of conventional expression systems familiar to those skilled in the art. In one embodiment, GalNAc-transferase lectins are expressed in a secreted soluble form, which can be recovered from the culture medium. Such secreted soluble forms lack the N-terminal cytoplasmic tail, transmembrane retention sequence, stem region and the catalytic unit. The boundaries of the catalytic units and lectin domains are defined by multiple sequence alignments and experimentation of lectin binding activity (multiple sequence alignment analysis of the C-terminal sequences polypeptide GalNAc-transferases including the most C-terminal boundaries of the catalytic domains and the entire lectin domains shown in Figure 2). The boundaries cannot be clearly defined but the most C-terminal well-conserved sequence motif of the catalytic units (WYLENVYP) can be excluded from the lectin domains. Parts of or the entire catalytic domains may be included to produce functional lectin domains, and inclusion of inactivating mutations in the catalytic units (e.g. mutations in the DxH motif important for donor substrate binding, or residues important for acceptor substrate binding ⁴) may be used to avoid additional binding activity mediated through the catalytic units. In another embodiment, host cells (e.g. CHO cells) are engineered to express full coding polypeptide GalNAc-transferases with or without mutations in their catalytic units and binding mediated through lectin domains are detremiend in vivo in host cells.

Cells stably or transiently transfected with full coding or secreted expression constructs of mucins, mucin-like glycoproteins, O-glycosylated proteins, or proteins can be carried out by any number of conventional methods familiar to those skilled in the art. In one embodiment, the mucin MUC1 is expressed in a soluble form, which can be recovered from the culture medium (Figure 3 illustrates MUC1 expression constructs used in this invention; the DNA sequence is available from GenBank accession number M61170). In another embodiment, host cells (e.g. CHO or CHO 1d1D cells) are engineered to express MUC1 on the cell surface. In a preferred embodiment of the invention the cells are mammalian and more preferably, the cells are human.

Human cell lines expressing cell surface mucins or secreting mucins can be selected, cultured and treated by any number of conventional methods familiar to those skilled in the art. In one embodiment, mucins are expressed in a secreted soluble form without transmembrane retention sequence, which can be recovered from the culture medium. In another embodiment, host cells (e.g. CHO 1d1D cells) are engineered to express full coding mucins on the cell surface.2.

### 2. General Aspects of the Invention

The putative lectin domains of some GalNAc-transferases notably GalNAc-T4 and -T7 have previously been shown to be important for the GalNAc-glycopeptide substrate specificities exhibited by these GalNAc-transferase isoforms (PCT WO 01/82215). The mechanism by which the putative lectin domains exert this effect on the enzyme activities is unknown. However, because GalNAc and GalNAcα-benzyl were found to selectively inhibit these activities it was hypothesized that the putative lectin domains functioned by recognizing the sugar or glycopeptide in a lectin-like interaction². Considerable efforts have been applied to demonstrate actual binding without success in the past (Bennett *et al.* unpublished, personal communications).

In the present invention a direct binding assay was developed using secreted soluble GalNAc-T4 and -T2, and chemoenzymatically produced multimeric MUC1 tandem repeat GalNAc-glycopeptides (Figure 4). Short MUC1 glycopeptides of traditional length of 15-20 amino acids have failed to provide significant binding in the same assay system, and one improvement leading to the success was the application of extended multimeric MUC1 GalNAc-glycopeptides. Binding was also found to an enzymatically GalNAc-glycosylated fusion protein expressed in *E. coli* and containing 30 amino acids of the MUC2 tandem repeat. Another improvement was the use of biotinylation of the enzymes, which provided an improved signal compared to previous attempts with identifying retained or bound enzyme by measuring activity. The specific activities of GalNAc-transferases as measured in *in vitro* assays are relatively low, and in past attempts to use binding and elution of enzyme acitivity presumably the detection level was not sufficient to detect binding. The developed assay was validated to demonstrate binding through the lectin domains by several ways: i) binding was selective for GalNAc-glycosylated glycopeptides with no significant binding to unglycosylated peptides; ii) a single amino acid substitution in the lectin domains of GalNAc-T4 (and - T2), known to selectively destroy GalNAc-glycopeptide specificity of these enzymes without affecting the catalytic unit ² abolished binding; iii) binding was not affected by EDTA treatment which is known to destroy catalytic activity of GalNAc-transferases ^{32,37-39}; iv) binding was selectively inhibited by the monosacharide GalNAc and not by other sugars.

In order to minimize the size and functional complexity of GalNAc-transferase lectins to be used as probes for binding studies, we used multiple sequence alignment analysis to predict and design suitable expression constructs for isolated lectin domains (Figure 2). In the present invention a direct binding assay was developed using isolated lectin domains of GalNAc-T4 and -T2 with minimal size (Figure 5). Analysis of the fine specificity of the binding by inhibition studies showed that GalNAc-T2 and -T4 lectins exhibit restricted specificity for GalNAc structures, and surprisingly that the anomeric configuration of the GalNAc residue is not important. Thus, both GalNAcα-and GalNAcβ-benzyl inhibited binding to the same degree. The lectin Helix Pomatia (HP) was used as a control plant lectin with known binding specificity for GalNAcα-structures. HP lectin showed a very different highly preferred binding specificity for GalNAcα-structures.

Studies with GalNAcα-benzyl have shown that this compound is effectively taken up by cells and used in the Golgi compartments ²⁶. It is also well known in the art that sugar-aryl compounds are taken up by the cell and used in the Golgi compartments. Thus, the surprising finding that GalNAc-transferase lectins can be inhibited by βGalNAc (GalNAcβ-benzyl), provides a new tool to study the function of polypeptide GalNAc-transferases *in vivo*; GalNAcβ-benzyl, because it, too, will enter the cell and be used in the Golgi compartments.

Availability of a binding assay is a useful tool to identify and characterize inhibitors of GalNAc-transferase lectins. In accordance with the binding assay method of the invention, GalNAc-transferases are contacted with a glycopeptide, glycoprotein, fusionprotein, or other appropriate structure or polymer containing the sugar hapten structure recognized by the GalNAc-trmsferase lectins, preferably *N*-acetylgalactosamine, and the GalNAc-transferase protein or lectin bound is quantitatively measured. The GalNAc-transferases may be in the form of a secreted soluble construct as applied in this invention, and any extended or truncated construct of a GalNAc-transferase as well as hybrid fusion protein that maintains the binding properties of the lectin. The ligand may be in the form of a chemoenzymatically produced MUC1 GalNAc-glycopeptide as applied in this invention, and any glycopeptide, glycoprotein, fusionprotein of any size or sequence, or other appropriate structure or polymer containing the sugar hapten structure recognized by the GalNAc-transferase lectins. Synthesis and chemoenzymatic synthesis of glycopeptides are familiar to those in the art, and are described in the literature cited above and in the *Examples* provided below. The ligand sugar may be GalNAc, *N*-acetylgalactosamine, or any other sugar in any linkage and sequence recognized by a GalNAc-transferase lectin. The binding assay may be an enzyme-linked solid phase immunoadsorption assay (ELISA) as applied in the invention, and any variant assay hereof where binding to ligand can be detected including without limitation radioimmunoassay (RIA), surface plasmon resonance (SPR), chemoluminescense, nuclear magnetic resonance spectroscopy (¹H-NMR), and other methods know in the art. Binding may be detected by horse-radish-peroxidase HRP-Avidin biotin as applied in this invention, and any other detection system including without limitation enzyme reactions, fluorescence, radioactivity, spectroscopy, spectrometry and other methods. The GalNAc-transferases may be labelled by biotinylation as applied in this invention, and any other labelling including without limitation antibody tags, enzymes, fluorochromes, radioisotopes and other methods know in the art, as well as detected by antibodies, phage antibody fragments or other binding proteins. The assay may used to characterize binding specificities of GalNAc-transferase lectins, screen and identify inhibitors of GalNAc-transferase lectins, and screen and identify competitive binders such as different GalNAc-transferase lectins and other lectins and proteins with binding properties for carbohydrates.

An *in vivo* model system for secretion of mucins was developed. A truncated secreted expression construct of the human cell surface mucin MUC1 containing 32 tandem repeats (Figure 3), was stably transfected into CHO wild type and CHO 1d1D cells ⁴⁰. Figure 6 illustrates intracellur expression of soluble MUC1 in wild type CHO transfectant clone, wtCHO/MUC1 sol-clone-C4, visualized by multiple monoclonal anti-MUC1 antibodies. Analysis of glycosylation was performed with a panel of antibodies with well-defined specificities for carbohydrate structures, and reactivity was mainly found with anti-T after pre-treatment with neuraminidase to remove sialic acids. Weak staining with anti-Tn was also found in some cells. Figure 7 illustrates western blot analysis of secreted MUC1 from the same cells. High molecular weight MUC1 migrating with apparent mw higher than 300 Kd is labelled by HMFG2, SM3, and VU-2G7, while all antibodies including VU-4H5 label a low molecular weight MUC1 migrating with apparent mw of 130 Kd and presumed to represent virtually unglycosylated MUC1. Pretreatment with neuraminidase decreased migration of the high molecular weight MUC1 bands, and anti-T antibody reactivity emerged. Stable MUC1 transfectants in CHO 1d1D showed similar patterns of reactivity when grown in Gal and GalNAc.

An *in vivo* model system for cell surface expression of mucins was developed. A full coding expression construct of the human cell surface mucin MUC1 containing 32 tandem repeats (Figure 3), was stably transfected into CHO wild type and CHO 1d1D cells. CHO 1d1D cells were originally established by Krieger *et al.* ⁴⁰ and found to have a defect in UDP-Gal/GalNAc epimerase that renders the cells incapable of producing UDP-Gal and UDP-GalNAc. Lack of UDP-Gal limits the synthesis of all types of glycoconjugates including glycosphingolipids, N-linked and O-linked glycoproteins. The synthesis.of O-linked glycoproteins will be arrested at GalNAcα1-O-Ser/Thr with or without addition of α2,6 linked sialic acid. In the absence UDP-GalNAc mainly O-linked mucin-type glycoconjugates are affected, and essentially no glycosylation occur, as the first sugar attached is GalNAc. The defect in CHO 1d1D cells can be selectively restored by addition of 1 mM GalNAc and or 0.1 mM Gal to the growth medium ⁴⁰. Addition of both sugars essentially restores normal glycosylation, whereas addition of GalNAc alone limits O-glycosylation to GalNAca1-O-Ser/Thr with or without addition of α2,6 linked sialic acid, and also affects galactosylation of N-linked glycosylation and glycolipid biosynthesis. Altschuler *et al.* ⁴¹ have previously shown that cell surface expression of MUC1 in CHO 1d1D cells requires addition of GalNAc.

Cell surface expression of MUC1 was established in stably transfected CHO wildtype and CHO 1d1D cells. MUC1 was detected at the surface of non-permeabilized cells using monoclonal anti-MUC1 antibodies (Figure 8). In accordance with Alschuler *et al.* ⁴¹ MUC1 surface expression in CHO 1d1D cells was only found in cells grown in GalNAc or Gal and GalNAc, whereas cells grown without sugars or only in Gal failed to express MUC1 at the surface. In agreement with the conclusion drawn by Altchuler *et al.* ⁴¹ surface expression of MUC1 was dependent only on the first step in O-glycosylation, the addition of GalNAc.

MUC1 produced in CHO 1d1D cells grown without GalNAc is not accumulated in Golgi, but degraded in lysosomes ⁴¹. This indicates that measuring total MUC1 in cell lysates rather than exclusively at the cell surface may be used as a measure of MUC1 expression. The experiments shown in Figures 9-10 use immunoprecipitation of total cell lysates with anti-MUC1 antibody followed by western blot analysis with the same or different anti-MUC1 antibody to quantify and characterize MUC1 expression in cells. MUC1 produced in cell grown without GalNAc or only in the presence of Gal migrate close to the predicted mass of the protein core. With the addition of GalNAc to the medium high molecular weight forms of MUC1 are found, and these react with all antibodies except VU-2G7. The antibody 5E5 only reacts with Tn glycoforms and lack of reactivity with MUC1 from cells grown in Gal and GalNAc indicate that the majority of MUC1 produced is glycosylated with sialyl-T structures.

GalNAcα-benzyl is a well-known inhibitor of O-glycosylation extension ²⁶. Treatment of cells with 1-2 mM GalNAcα-benzyl partially blocks core 1 O-glycosylation including α2,3 sialylation. Treatment of cells with GalNAcα-benryl is also known to affect surface expression of mucins and O-glycosylated glycoproteins, as well as in some cases secretion of mucins. A number of mammalian cell lines have been treated with 1-2 mM GalNAcα-benzyl in the past and the resulting effects on O-glycosylation as well as mucin secretion have varied with cell type (for a detailed review see ⁴²).

The effect of GalNAcα-benzyl on mucin transport and secretion has been concluded to be due to blockage of O-glycosylation extension ⁴². Figures 11-13 illustrate that CHO 1d1D cells grown in Gal and GalNAc (or wild type CHO cells), and treated with 2 mM GalNAcα-benzyl in agreement with this, exhibits reduced expression of MUC1 as well as altered O-glycosylation as judged by an altered SDS-PAGE migration pattern. Wildtype CHO cells as well as CHO 1d1D cells grown in Gal and GalNAc produce O-glycans of the mono- and disialylated core 1 structures (NeuAcα2-3Galβ1-3[NeuAca2-6]_{+/-}GalNAcα-O-Ser/Thr). Treatment with GalNAcα-benzyl results in some exposure of unsialylated core 1 as evaluated by staining with anti-T monoclonal antibody HH8, whereas only very little Tn is exposed as evaluated with anti-Tn monoclonal antibody 5F4. The altered SDS-PAGE migration of MUC1 produced in CHO 1d1D cells grown in Gal and GalNAc (or wild type CHO cells, not shown) shown in Figures 12-13 (lanes 4 and 10 when indicated) reflects mainly loss of sialic acids.

If the effect of GalNAcα-benzyl treatment on mucin transport and secretion is due to inhibition of sialylation, then treatment of CHO 1d1D cells grown only in GalNAc and hence producing only GalNAcα1-O-Ser/Thr O-glycosylation (neglible STn is produced as evidenced by lack of staining with anti-STn monoclonal antibodies 3F and TKH2, while cells stain very strongly with anti-Tn monoclonal antibodies 5F4 and 1E3), should have no effect on expression of MUC1 in these cells. Surprisingly as shown in Figures 12-13 (lanes 1 and 7 when indicated), GalNAcα-benzyl treatment does inhibit MUC1 expression in CHO 1d1D cells with O-glycosylation controlled and limited to Tn glycoforms. This result shows for the first time that mucin transport and secretion may be directly affected by treatment with GalNAcα-benzyl and not through a mechanism involving inhibtion of sialylation or the O-glycosylation extension pathways. Combined with the findings of Altchuler *et al.* ⁴¹, these results indicate that mucin transport and secretion requires some degree of GalNAc O-glycosylation, whereas O-glycan extension including sialylation seems to be of less importance for this process.

An appropriate control for GalNAcα-benzyl treatment has not previously been studied. Selection of a benzyl monosaccharide that is not involved in and does not affect glycosylation pathways in cells is problematic. We chose to use GlcNAcα-benzyl as such a control as this structure is not used in glycosylation pathways of mammalian glycoproteins and glycosphingolipids. As shown in Figures 12-13 (lanes 2,5, 8, and 11, when indicated) treatment of CHO 1d1D cells with 2 mM GlcNAcα-benzyl had no effect on MUC1 expression and glycosylation. GlcNAcα-benzyl thus serves as a control for treatment of cells with benzyl sugars, and this is important because benzyl sugars and their biosynthetic products appear to aggregate in cells and cause morphological changes with prolonged treatment ²⁶.

Since transport of mucin in cells was selectively inhibited by GalNAcα-benzyl (and not GlcNAcα-benzyl), even in cells limited to GalNAcα1-O-Ser/Thr O-glycosylation, we hypothesized that polypeptide GalNAc-transferases and in particular their lectin domains could be involved in ensuring mucin transport and preventing direction to lysosomes. On the one hand, GlcNAcα-benzyl does not in general inhibit GalNAc O-glycosylation and polypeptide GalNAc-transferase enzyme activity. On the other, we had previously discovered that the GalNAc-glycopeptide acceptor substrate specificities of some GalNAc-transferase isoforms including GalNAc-T4 and -T7 ²(PCT WO 01/85215), which suggested that these enzymes could be involved. These precepts thus give rise to plural hypothesis. One hypothesis would suggest that inhibition of the GalNAc-glycopeptide acceptor substrate specificity of GalNAc-transferases leads to mucin glycoforms with lower density of O-glycan occupancy (shown *in vitro* for e.g. MUC1 tandem repeats ²), and that this decrease in O-glycan density results in increased targeting to lysosomal degradation and hence decrease in expression. Another hypothesis would suggest that the lectin domains of GalNAc-transferases in general have the capacity to bind GalNAc and hence provide a lectin mediated chaperone-like function, which is required for Golgi transport of O-glycosylated proteins. Lectin chaperones are well known to function ER transport as well as in lysosomal targeting ⁴³, but the existence of such lectin chaperones for cell surface expression and secretion have not been demonstrated in the Golgi or trans-Golgi network.

As described above we found that GalNAc-transferase lectins in a binding assay to GalNAc-glycopeptides surprisingly showed similar inhibition with GalNAcα-benzyl and GalNAcβ-benzyl. This indicates that these lectins in contrast to many lectins including Helix Pomatia fall to distinguish the anomeric configuration of the monosaccharide hapten recognized. βGalNAc is a rare linkage in mammalian glycoproteins and is found only in N-linked glycoproteins in man associated with the hormone specific glycosylation pattern where it generally is sulphated. Although, βGalNAc is found in both ganglioseries (GalNAcβ1-4Galβ1-4Glcβ1-Cer) and globoseries (GalNAcβ1-3Galα1-4Galβ1-4Glcβ1-Cer) it is expected that treatment of cells with 2 mM GalNAcβ-benzyl will not interfere significantly with glycosylation. This is based on the findings that the β1,3galactosyltransferases (β3Gal-T4 and β3Gal-T5, respectively) involved in extending βGalNAc in these two glycolipid structures show no or very poor activity with GalNAcβ-benzyl ^{44,45}. The only βGalNAc containing structure in O-linked glycosylation is found in the blood group related Sda structure (GalNAcβ1-4(Neuα2-3)Galβ1-3GalNAcα1-O-Ser/Thr) which has very restricted expression ⁴⁶.

We therefore tested if GalNAcβ-benzyl treatment of cells showed the same effects as GalNAcα-benzyl treatment. As shown in Figures 14-15 (lane 5) GalNAcβ-benzyl treatment does not interfere with O-glycosylation in contrast to GalNAcα-benzyl (lane 4), as no difference in SDS-PAGE migration is observed. However, GalNAcα-benzyl, as well as GalNAcβ-benzyl treatment of cells, produces similar significant reduction in expression of MUC1 Figures 14-15. This shows surprisingly and for the first time that GalNAcβ-benzyl represents a selective inhibitor of mucin transport and secretion. GalNAcβ-benzyl is a novel preferred inhibitor of transport, surface expression and secretion of O-glycosylated proteins and mucins, because it does not interfere with the O-glycosylation extension process. GalNAcβ-benzyl is not expected to accumulate biosynthetic oligosaccharide products similar to those found with GalNAcα-benzyl treatment ²⁶. The finding that GalNAcβ-benzyl exerts these effects on mucin expression combined with the finding that it inhibits polypeptide GalNAc-transferases strongly indicate that the mechanism by which GalNAcα- and GalNAcβ-benzyl inhibits mucin expression is through inhibition of GalNAc-transferase lectins. This supports the second hypothesis articulated above. Polypeptide GalNAc-transferase lectins thus represent prime targets for intervention with mucin secretion and cell surface expression, and GalNAcβ-benzyl represents a novel selective prototype inhibitor for such intervention.

Preferred compounds for inhibition of GalNAc-transferase lectins are inactive as acceptor substrates for glycosyltransferases. In particular, the following glycosyltransferase activities: core 1 UDP-Gal:GalNAc-peptide β1,3galactosyltransferases; CMP-NeuAc:GalNAc-peptide α2,6sialyltransferases, and UDP-GlcNAc:β1,3N-acetylglucosaminyltransferases involved in O-glycosylation, are inactive with the preferred inhibitory compounds. Examples of such inhibitory compounds are GalNAcα1-O-benzyl with substitution of hydroxyl groups at C3 and/or C6 with methyl or acetyl groups to block acceptor sites.

The methods described herein are designed to identify substances and compounds that bind to and or modulate the biological activity of a polypeptide GalNAc-transferase lectin, including substances that interfere with or enhance the activity of a polypeptide GalNAc-transferase lectin.

GalNAc-transferase lectins may be used in the form of a truncated lectin domain as shown in *Example* 3, as a secreted GalNAc-transferase enzyme as shown in *Example* 2, or as a truncated protein or fusion protein with or without catalytic activity but with retained lectin domain and carbohydrate binding activity.

Agents that modulate a polypeptide GalNAc-transferase lectin can be identified based on their ability to associate with such a lectin. Therefore, the invention also provides a method of identifying agents that associate with a polypeptide GalNAc-transferase lectin. Agents identified using the method of the invention may be isolated, cloned and sequenced using conventional techniques. An agent that associates with a polypeptide GalNAc-transferase lectin may be an agonist or antagonist of the biological or immunological activity of the lectin.

Agents that can associate with a polypeptide GalNAc-transferase lectin may be identified by reacting such GalNAc-transferase lectin with a test substance, which potentially associates with a polypeptide GalNAc-transferase lectin under conditions which permit the association, and removing and/or detecting the associated GalNAc-transferase lectin and substance. The substance-lectin complex, free substance, or non-complexed lectin may be assayed. Conditions, which permit the formation of substance-lectin complexes, may be selected having regard to factors such as the nature and amounts of the substance and the lectin.

The substance-lectin complex, free substance or non-complexed lectin may be isolated by conventional isolation techniques, for example, salting out, chromatography, electrophoresis, gel filtration, fractionation, absorption, polyacrylamide gel electrophoresis, agglutination, or combinations thereof. To facilitate the assay of the components, a labelled antibody against the transferase or the substance or a labelled lectin or a labelled substance may be utilized. The antibodies, lectins, or test substances may be labelled with a detectable substance as described above.

A polypeptide GalNAc-transferase lectin, or a test substance used in the method of the invention may be insolubilized. For example, a lectin, or a test substance may be bound to a suitable carrier such as agarose, cellulose, dextran, Sephadex, Sepharose, carboxymethyl cellulose polystyrene, filter paper, ion-exchange resin, plastic film, plastic tube, glass beads, polyamine-methyl vinyl-ether-maleic acid copolymer, amino acid copolymer, ethylene-maleic acid copolymer, nylon, silk, etc. The carrier may be in the shape of, for example, a tube, test plate, beads, disc, sphere etc. The insolubilized lectin or substance may be prepared by reacting the material with a suitable insoluble carrier using known chemical or physical methods, for example, cyanogen bromide coupling.

The invention also contemplates a method for evaluating an agent for its ability to modulate the biological activity of a polypeptide GalNAc-transferase lectin by assaying for an agonist or antagonist (i.e. enhancer or inhibitor) of the association of the lectin with a substance that interacts with the polypeptide (e.g. carbohydrate binding site or parts thereof). The basic method for evaluating whether an agent is an agonist or antagonist of the association of a polypeptide GalNAc-transferase lectin and a substance that associates with the lectin is to prepare a reaction mixture containing the lectin and the substance under conditions which permit the formation of substance-lectin complexes, in the presence of a test agent. The test agent may be initially added to the mixture, or may be added subsequent to the addition of the lectin and substance. Control reaction mixtures without the test agent or with a placebo are also prepared. The formation of complexes is detected and the formation of complexes in the control reaction, but not in the reaction mixture, indicates that the test agent interferes with the interaction of the lectin and substance. The reactions may be carried out in the liquid phase or the lectin, substance, or test agent may be immobilized as described herein.

It will be understood that the agonists and antagonists, i.e. enhancers and inhibitors, that can be assayed using the methods of the invention may act on one or more of the interaction sites on the lectin or substance including agonist binding sites, competitive antagonist binding sites, non-competitive antagonist binding sites or allosteric sites. It will also be understood that competitive assays, in addition to direct assays, can be used to screen for and identify the agents of the present invention.

The invention also makes it possible to screen for antagonists that inhibit the effects of an agonist of the interaction of a polypeptide GalNAc-transferase lectin with a substance capable of associating with the lectin. Thus, the invention may be used to assay for an agent that competes for the same interacting site of a polypeptide GalNAc-transferase lectin.

Test compounds are screened from, for example, large libraries of synthetic or natural compounds. Numerous means are currently used for random and directed synthesis of saccharide, peptide, and nucleic acid based compounds. Examples of available libraries are synthetic compound libraries are commercially available from Maybridge Chemical Co. (Trevillet, Cornwall, UK), Comgenex (Princeton, NJ), Brandon Associates (Merrimack, NH), and Microsource (New Milford, CT).

Agents which are effective in modulating a polypeptide GalNAc-transferase lectin can be identified, based on their ability to interfere with or enhance the lectin mediated binding capacity of the GalNAc-transferase protein or fragment hereof containing the lectin region. Therefore, the invention provides a method for evaluating a test substance for its ability to modulate the binding capacity of a polypeptide GalNAc-transferase lectin comprising
(a) reacting a binding substrate with a GalNAc-transferase or lectin polypeptide or fragment hereof in the presence of a test substance;
(b) measuring the amount of binding substrate bound to the GalNAc-transferase polypeptide or fragment hereof, and
(c) carrying out steps (a) and (b) in the absence of the test substance to determine if the substance interferes with or enhances the binding by the polypeptide GalNAc-transferase.

Suitable binding substrates for use in the methods of the invention are polypeptides, glycopolypeptides, or glycoproteins, which are either synthetic or naturally occurring structures. The GalNAc-transferase lectin polypeptide may be obtained from natural sources or produced using recombinant methods as described and referenced herein.

The binding or modifying substrates may be labelled with a detectable substance as described herein, and the interaction of the polypeptide of the invention with the binding or modifying substrates will give rise to a detectable change. The detectable change may be colorimetric, photometric, radiometric, potentiometric, etc. The GalNAc-transferase lectin polypeptide is reacted with the binding or modifying substrates at a pH and temperature effective for the polypeptide to bind the substrates, and where preferably one of the components is labeled, to produce a detectable change. It is preferred to use a buffer with the substrates to maintain the pH within the pH range effective for the polypeptides. The buffer and substrates may be used as an assay composition. Other compounds such as EDTA and detergents may be added to the assay composition.

The reagents suitable for applying the methods of the invention to evaluate agents that modulate a polypeptide GalNAc-transferase lectin may be packaged into convenient kits providing the necessary materials packaged into suitable containers. The kits may also include suitable supports useful in performing the methods of the invention.

Agents that modulate polypeptide GalNAc-transferase lectin(s) can also be identified by treating immortalized cells which express the transferase(s) with a test substance, and comparing the intracellular transport, degradation, surface expression, or secretion of O-glycosylated proteins, mucins, and glycoproteins performed of the cells with those of the cells in the absence of the test substance and/or with immortalized cells which do not express the transferase(s). Examples of immortalized cells that can be used include human cell lines, Chinese hamster ovary (CHO) cells and mutant cells CHO 1d1D ⁴⁰, which express polypeptide GalNAc-transferase(s) or lectin(s) and produce cell membrane bound or secereted forms of the human mucin MUC1. In the absence of an inhibitor the cells will produce and transport MUC1 to the cell surface or secrete MUC1 into the growth medium. Substances that reduce the cell surface expression or the quantity of MUC1 in the medium may be considered an inhibitor.

The agents identified by the methods described herein, may be used for modulating the binding activity of a polypeptide GalNAc-transferase lectin, and they may be used as prototype drugs in the treatment of conditions mediated by a polypeptide GalNAc-transferase lectin and in designing further substances effective to treat such conditions. In particular, they may be used to alter density of O-glycosylation on glycoproteins and mucins produced by cells, the intracellular transport and surface expression of glycoproteins and mucins, the secretion of glycoproteins and mucins, and other functions governed by the polypeptide GalNAc-transferases and their lectins in transport and secretion of glycoproteins and mucins.

Therefore, the present disclosure has potential application in the treatment of various disorders associated with aberrant O-glycosylation and/or mucin production in mammals, preferably humans. Such disorders include the following: tumors and cancers, lungs diseases associated with mucous accumulation such as asthma, chronic bronchitis, smoker's lung, cystic fibrosis, diseases of exocrine glands associated with increased or decreased mucin secretion such as Sjøgrens syndrome, dry mouth etc. Other disorders include dysregulation of selectin-mediated leukocyte trafficking and would include but not be limited to disorders involving autoimmunity, arthritis, leukaemia's, lymphomas, immunosuppression, sepsis, wound healing, acute and chronic in action, cell mediated immunity, and the like.

The agents identified by the methods described herein, have potential application in treatment of tumors including inhibition of tumor metastasis and growth and/or regression of same. Tumor metastasis may be inhibited by inhibiting the adhesion of circulating cancer cells. The agents of the invention have particular potential application may be especially useful in the treatment of various forms of neoplasia such as leukaemias, lymphomas, melanomas, adenomas, sarcomas, and carcinomas of solid tissues in patients. In particular the composition may be used for treating malignant melanoma, pancreatic cancer, cervico-uterine cancer, cancer of the liver, kidney, stomach, lung, rectum, breast, bowel, gastric, thyroid, neck, cervix, salivary gland, bile duct, pelvis, mediastinum, urethra, bronchogenic, bladder, esophagus and colon, and Kaposi's Sarcoma which is a form of cancer associated with HIV-infected patients with Acquired Immune Deficiency Syndrome (AIDS). The substances etc. are particularly useful in the prevention and treatment of tumors of lining mucosa and glands and the metastases derived from these tumors.

Accordingly, the various agents may be formulated into pharmaceutical compositions for administration to subjects in a biologically compatible form suitable for administration in vivo. By biologically compatible form suitable for administration in vivo is meant a form of the agent to be administered in which any toxic effects are outweighed by the therapeutic effects. The agents may be administered to living organisms including humans, and animals. Administration of a therapeutically active amount of the pharmaceutical compositions of the present invention is defined as an amount effective, at dosages and for periods of time necessary to achieve the desired result. For example, a therapeutically active amount of an agent may vary according to factors such as the disease state, age, sex, and weight of the individual, and the ability of pharmaceutical composition or polypeptide to elicit a desired response in the individual. Dosage regima may be adjusted to provide the optimum therapeutic response. For example, several divided doses may be administered daily or the dose may be proportionally reduced as indicated by the exigencies of the therapeutic situation.

The active agent may be administered in a convenient manner such as by injection (subcutaneous, intravenous, etc.), oral administration, inhalation, transdermal application, or rectal administration. Depending on the route of administration, the active agent may be coated in a material to protect the agent from the action of enzymes, acids and other natural conditions that may inactivate it.

The compositions described herein can be prepared by methods known per se for the preparation of pharmaceutically acceptable compositions which can be administered to subjects, such that an effective quantity of the active agent is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the compositions include, albeit not exclusively, solutions of the agents in association with one or more pharmaceutically acceptable vehicles or diluents, and contained in buffered solutions with a suitable pH and iso-osmotic with the physiological fluids.

The phrase "pharmaceutically acceptable" refers to molecular entities and compositions that are physiologically tolerable and do not typically produce an allergic or similar untoward reaction (for example, gastric upset, dizziness and the like) when administered to an individual. Preferably, and particularly where a immunogenic composition is used in humans, the term "pharmaceutically acceptable" denotes approved by a regulatory agency (for example, the U.S. Food and Drug Agency) or listed in a generally recognized pharmacopeia for use in animals (for example, the U.S. Pharmacopeia).

Toxicity and therapeutic efficacy of compounds can be determined by standard pharmaceutical procedures, for example in cell culture assays or using experimental animals to determine the LD50 and the ED50. The parameters LD50 and ED50 are well known in the art, and refer to the doses of a compound that are lethal to 50% of a population and therapeutically effective in 50% of a population, respectively. The dose ratio between toxic and therapeutic effects is referred to as the therapeutic index and may be expressed as the ratio: LD50/ED50. Compounds that exhibit large therapeutic indices are preferred. While compounds that exhibit toxic side effects may be used. However, in such instances it is particularly preferable to use delivery systems that specifically target such compounds to the site of affected tissue so as to minimize potential damage to other cells, tissues or organs and to reduce side effects.

Data obtained from cell culture assay or animal studies may be used to formulate a range of dosages for use in humans. The dosage of compounds used in therapeutic methods of the present invention preferably lie within a range of circulating concentrations that includes the ED50 concentration but with little or no toxicity (e.g., below the LD50 concentration). The particular dosage used in any application may vary within this range, depending upon factors such as the particular dosage form employed, the route of administration utilized, the conditions of the individual (e.g., patient), and so forth.

Non-human animals include, without limitation, laboratory animals such as mice, rats, rabbits, hamsters, guinea pigs, etc.; domestic animals such as dogs and cats; farm animals such as sheep, goats, pigs, horses, and cows; and non-human primates.

A therapeutically effective dose may be initially estimated from cell culture assays and formulated in animal models to achieve a circulating concentration range that includes the IC50. The IC50 concentration of a compound is the concentration that achieves a half-maximal inhibition of symptoms (e.g., as determined from the cell culture assays). Appropriate dosages for use in a particular individual, for example in human patients, may then be more accurately determined using such information.

Measures of compounds in plasma may be routinely measured in an individual such as a patient by techniques such as high performance liquid chromatography (HPLC) or gas chromatography.

Pharmaceutical compositions for use in accordance with the present invention may be formulated in conventional manner using one or more physiologically acceptable carriers or excipients.

Thus, the compounds and their physiologically acceptable salts and solvates may be formulated for administration by the routes described above.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner. For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

After pharmaceutical compositions have been prepared, they can be placed in an appropriate container and labelled for treatment of an indicated condition. For administration of an inhibitor of a polypeptide GalNAc-transferase, such labelling would include amount, frequency, and method of administration.

The use of inhibitors of the lectin domain mediated activities of the above mentioned polypeptide GalNAc-transferase isoforms and other isoforms allows for unique selective inhibition of these functions *in vitro* and *in vivo* in cells and organisms. This is desirable in manipulating the density of O-glycans, e.g. changing high density O-glycosylated tumour-associated MUC1 to low density normal MUC1 in cells. Further this is desirable in inhibiting any adhesive role the lectin domains may play in Golgi transport and intracellular sorting.

### EXAMPLES

### 1. Cloning, Expression, and Purification of Soluble GalNAc-Transferase Proteins and Soluble GalNAc-Transferase Lectins.

Polypeptide GalNAc-transferases are highly conserved throughout evolution. Orthologous relationships can be defined from man to Drosophila, 48 and ortholgous members of all human polypetide GalNAc-transferase isoforms are clearly identifiable in mouse and rats, and likely all mammals.

Polypeptide GalNAc-transferases are predicted to be type II transmembrane Golgi-resident proteins with a domain structure depicted in Figure 1 2. The N-terminal cytoplasmic tail, the hydrophic transmembrane signal sequence, and the stem region may be involved in directing Golgi-localization 47. The catalytic unit of the enzymes is approximately 300-350 amino acid residues and highly conserved in primary sequence among isoforms and also throughout evolution of the gene family 3,48. The C-terminal region of approximately 130 amino acids exhibits similarity with the galactose binding lectin, ricin. This region show little sequence similarity among isoforms and is poorly conserved in evolution 3.

Soluble, secreted expression constructs of human GalNAc-transferases GalNAc-T1, -T2, -T3, -T4, -T6, -T7, and -T11 for baculo-virus mediated expression in insect cells have been described in detail previously ^{3,8,10,32-34}. His-tagged soluble expression constructs for all human ppGalNAc-transferases, including novel genes designated GalNAc-T12, -T13, -T14, -T15, and -T16, were prepared using PCR primers as listed in Table I below.

**TABLE I. Primers used for PCR of soluble secreted GalNAc-transferase expression constructs.**

| **CalNAc-T1:** | |
|---|---|
| EBHC121H: | 5'-GCGGGATCCAGGACTTCCTGCTGGAGATG-3' |
| EBHC107B: | 5'-GCGGATCCTCAGAATATTTCTGGAAGGG-3' |
| | |

| **GalNAc-T2:** | |
|---|---|
| EBHC75D: | 5'-GCGGAATTCTTAAAAAGAAAGACCTTCATCACAGC-3' |
| EBHC68: | 5'-GCGGAATTCCTACTGCTGCAGGTTGAGC-3' |
| | |

| **GalNAc-T3:** | |
|---|---|
| EBHC219H: | 5'-GCGGGATCCAACGATGGAAAGGAACATG-3' |
| EBHC215: | 5'-AGCGGATCCAGGAACACTTAATCATTTTGGC-3' |
| | |

| **GalNAc-T4:** | |
|---|---|
| EBHC318: | 5'-GCGGGATCCTTTTCATGCCTCCGCAGGAGCC-3' |
| EBHC307: | 5'-GCGGGATCCGACGAAAGTGCTGTTGTGCTC-3' |
| | |

| **GalNAc-T5:** | |
|---|---|
| EBHC909: | 5'-GCGGGATCCTGCTTTAACTGGAGGGCTAGAGC-3' |
| EBHC907: | 5'-GCGGGATCCATCAGTTACACTTCAGGCTTC-3' |
| | |

| **GalNAc-T6:** | |
|---|---|
| EBHC514H: | 5'-GCGGGATCCCCTGGACCTCATGCTGGAGGCCATG-3' |
| EBHC511N: | 5'-AGCGGATCCTGGGGATGATCTGGGTCCTAGAC-3' |
| | |

| **GalNAc-T7:** | |
|---|---|
| EBHC1122H: | 5'-GCGAAGCTTCAGGATGAGGGAAGACAGAGATG |
| EBHC1116H: | 5'-GCGAAGCTTCTCTCTAAACACTATGGATCTTATTC-3' |
| | |

| **GalNAc-T8:** | |
|---|---|
| EBHC1820: | 5'-GCGGGATCCTCTGAAAGAAAGTATGAAATTAGC-3' |
| EBHC1821: | 5'-GCGGGATCCTCACTGGCTGTTGGTCTGACC |
| | |

| **GalNAc-T9:** | |
|---|---|
| EBHC1320: | 5'-GCGGGATCCCTGCCGCCTGCAGGGCCGCTCCCAG-3' |
| EBHC1321: | 5'-GCGGGATCCTCAGTGCCGTCGGTGTTTGATCC-3' |
| | |

| **GalNAc-T10:** | |
|---|---|
| EBHC2520: | 5'-GCGGGATCCCCGCGAGCGGCAGCCCGACGGC-3' |
| EBHC2521: | 5'-GCGGGATCCTCAGTTCCTATTGAATTTTTC-3' |
| | |

| **GalNAc-T11:** | |
|---|---|
| EBHC629: | 5'-GCGAATTCGTGAAGTGACTCAGCCACTTAAG-3' |
| EBHC614: | 5'-GCGAATTCGTCTCTGTCAGACACGTGTC-3' |
| | |

| **GalNAc-T12:** | |
|---|---|
| EBHC1051: | 5'-GCGGGATCCGGCTCGGTGCTGCGGGCGCAGCG-3' |
| EBHC1032: | 5'-GCGGGATCCTCATAACATGCGCTCTTTGAAGAACC-3' |
| | |

| **CalNAc-T13:** | |
|---|---|
| EBHC2000: | 5'-GCGGGATCCGATGTTGCACWTCCCCACCACACC-3' |
| EBHC2002: | 5'-GCGGGATCCTCATCGTTCATCCACAGCATTG-3' |
| | |

| **GalNAc-T14:** | |
|---|---|
| EBHC1720: | 5'-GCGGGATCCTCTGCTGCCTGCATTGAGGGCTG-3' |
| EBH21721: | 5'-GCGGGATCCTCATGTGCCCAAGGTCATGTTCC-3' |
| | |

| **GalNAc-T15:** | |
|---|---|
| EBHC412: | 5'-GCGGGATCCCAAGAGGAAGTTGGAGGTGCCG-3' |
| EBHC438: | 5'-GCGGGATCCCAGGGGTCCTCAAGAGCTCACC-3' |
| | |

| **GalNAc-T16:** | |
|---|---|
| EBHC1913: | 5'-GCGGGATCCCTACTACTTATGGCAGGACAACCG-3' |
| EBHC1912: | 5'-GCGTCATGTGTGTGGCAACAGCTGCCACTG-3' |

Expression constructs were amplified by PCR using 20 ng plasmid DNA as template. Expand High Fidelity-kit (Roche) was used as recommended by the manufacturer using an ABI2700 thermocycler (Applied Biosystems). Products were digested with *Eco*RI (GalNAc-T2, -T11, -T12 and -T16), *Bam*HI (GalNAc-T1, -T3, -T4, -T5, - T6, -T8, -T9, -T10, -T13, -T14 and -T15) and *Hind*III (GalNAc-T7), and sub-cloned into the *Eco*RI or *Hind*III site of pBKS-HistagI or the BamHI site of pBKS-HistagII. PBKS-Histag-I and -II vectors were generated from pBluescrip (Stratagene), by inserting a fragment encoding 6xHis, a thrombin cleavage site, and a T7 antibody site. pBKS-Histag-I was modified with the sequence pBKS-Histag-II was modified with the sequence:

All construct were fully sequenced. His-tagged GalNAc-transferase pBKS-HIS-tag-I constructs were excised with NotI and XhoI (blunt-ended) or as for GalNAc-T11, with NotI and HindIII (blunt-ended) and sub-cloned into the NotI/BglII (blunt-ended) site of the pAcGP67A Baculo expression vector (Pharmingen). His-tagged GalNAc-transferase pBKS-HIS-tag-II constructs were excised with NotI and inserted into the NotI site of pAcGP67A Baculo expression vector.

The coding region for human polypeptide GalNAc-T12 has been submitted to GenBank/EBI Data Bank and assigned accession number AJ 132365:
Human GalNAc-T12 DNA sequence:
Human GalNAc-T12 amino acid sequence:

The coding region for human polypeptide GalNAc-T13 has been submitted to GenBank/EBI Data Bank and assigned accession number AR153422:
Human GalNAc-T13 DNA sequence:
Human GalNAc-T13 amino acid sequence:

The coding region for human polypeptide GalNAc-T14 has been submitted to GenBank/EBI Data Bank and assigned accession number AJ505991:
Human GalNAc-T14 DNA sequence:
Human GalNAc-T14 amino acid sequence:

The coding region for human polypeptide GalNAc-T15 has been submitted to GenBank/EBI Data Bank and assigned accession number Y09324:
Human GalNAc-T15 DNA sequence:
Human GalNAc-T15 amino acid sequence:

The coding region for human polypeptide GalNAc-T16 has been submitted to GenBank/EBI Data Bank and assigned accession number AJ505951:
Human GalNAc-T16 DNA sequence:
Human GalNAc-T16 amino acid sequence:

Additional homologous polypeptide GalNAc-transferase genes have been identified and cloning and expression are in progress, and it follows from the descriptions that similar methods as outlined above will yield soluble secreted proteins for study. Expression constructs may have immunoaffinity tags or purification tags at the *N-*terminal and/or *C*-terminal region. These may include myc, FLAG, HIS, GST, and other (Stratagene, Qiagen, Amersham Biosciences).

Soluble secreted expression constructs of GalNAc-transferase lectin domains were prepared from the GalNAc-transferase expression constructs described above by PCR using primer pairs as listed Table II below.

**Table II. Primers used for amplification of GalNAc-transferase lectin domains**

| **GalNAc-T1 lectin domain:** | |
|---|---|
| T1LECFOR: | 5'-CAAAGGAAGCTTATGGAGATATATCGTCAAGAG-3' |
| T1LECREV: | 5'-GCAAGCTCGAGGCGGCCGCTCAGAATATTTCTGGAAGGGTGAC-3' |
| | |

| **GalNAc-T2 lectin domain:** | |
|---|---|
| T2LECFOR: | 5'-CAAGGAAGCTTCTTATGGAAATATTCAGAGCAGATTG-3' |
| T2LECREV: | 5'-GCAAGCTCGAGGCGGCCGCCTACTGCTGCAGGTTGAGC-3' |
| | |

| **GalNAc-T3 lectin domain:** | |
|---|---|
| T3LECFOR: | 5'-CAAGGAAGCTTCATTTGGTGATCTTTCAAAAAGATTT-3' |
| T3LECREV: | 5'-GCAAGCTCGAGGCGGCCGCAGGAACACTTAATCATTTTGG-3' |
| | |

| **GalNAc-T4 lectin domain:** | |
|---|---|
| T4LECFOR: | 5'-AGAAAAGAAGCTTATGGTGATATTTCTG-3' |
| EBHC307: | 5'-AGCGGATCCGACGAAGTGCTGTTGTGCT -3' |
| | |

| **GalNAc-T5 lectin domain:** | |
|---|---|
| T5LECFOR: | 5'-CAAGGAAGCTTTAGATGTTGGCAACCTCACCCAGC-3' |
| T5LECREV: | 5'-GCAAGCTCGAGGCGGCCGCAAGCATCAGTTACACTTCAGGCTTC-3' |
| | |

| **GalNAc-T6 lectin domain:** | |
|---|---|
| T6LECFOR: | 5'-CAAGGAAGCTTCCTTCGGTGACATTTCGGAACG-3' |
| T6LECREV: | 5'-GCAAGCTCGAGGCGGCCGCTGGGTCCTAGACAAAGAGCC-3' |
| | |

| **GalNAc-T7 lectin domain:** | |
|---|---|
| T7LECFOR: | 5'-AGAAAAGAAGCTTATGGGGATATATCGGAGCTG-3' |
| T7LECREV: | 5'-GCAAGCTCGAGGCGGCCGCTCTCTAAACACTATGGATGTTATTC-3' |
| | |

| **GalNAc-T8 lectin domain:** | |
|---|---|
| T8LECFOR: | 5'-CAAGGAAGCTTTTGGAGACGTTTCTTCCAGAATG-3' |
| TBLECREV: | 5'-GCAAGCTCGAGGCGGCCGCTCACTGGCTGTTGGTCTGACCCC-3' |
| | |

| **GalNAc-T9 lectin domain:** | |
|---|---|
| T9LECFOR: | 5'-CAAGGAAGCTTTCGGGGACGTGTCTGAGAGGCTG-3' |
| T9LECREV: | 5'-GCAAGCTCGAGGCGGCCGCTCAGTGCCGTGCGTGTTTGATCC -3' |
| | |

| **GalNAc-T10 lectin domain:** | |
|---|---|
| T10LECFOR: | 5'-CAAGGAAGCTTCCGCTGGGGATGTCGCAGTCCAG-3' |
| T10LECREV: | 5'-GCAAGCTCGAGGCGGCCGCTCAGTTCCTATTGAATTTTTCC-3' |
| | |

| **GalNAc-T11 lectin domain:** | |
|---|---|
| T11LECFOR: | 5'-CAAGGAAGCTTGCAATATCAGTGAGCGTGTGG-3' |
| T11LECREV: | 5'-GCAAGCTCGAGGCGGCCGCCCACCTTAACCTTCCAAATGC-3' |
| | |

| **GalNAc-T12 lectin domain:** | |
|---|---|
| T12LECFOR: | 5'-CAAGGAAGCTTGGGATGTGACAGAGAGGAAG-3' |
| T12LECREV: | 5'-GCAAGCTCGAGGCGGCCGCTCATAACATGCGCTCTTTGAAGAACC-3' |
| | |

| **GalNAc-T13 lectin domain:** | |
|---|---|
| T13LECFOR: | 5'-CAAGGAAGCTTCTGAGAAGCCAGACTGCATGG-3' |
| T13LECREV: | 5,-GCAAGCTCGAGGCGGCCGCTCATCGTTCATCCACAGCATTG-3' |
| | |

| **GalNAc-T14 lectin domain:** | |
|---|---|
| T14LECFOR: | 5'-CAAGGAAGCTTATGGAGATGTGTCAGTCAGAAAAAC-3' |
| T14LECREV: | 5'-GCAAGCTCGAGGCGGCCGCTCATGTGCCCAAGGTCATGTTCC-3' |
| | |

| **GalNAc-T15 lectin domain:** | |
|---|---|
| T15LECFOR: | 5'-CAAGGAAGCTTTCGGGAATGTTGAGAGCAGATTG-3' |
| T15LECREV: | 5'-GCAAGCTCGAGGCGGCCGCTCAAGAACTCACCATGTCCCAGTG-3' |
| | |

| **GalNAc-T16 lectin domain:** | |
|---|---|
| T16LECFOR: | 5'-CAAGGAAGCTTGCAGTGTGGCTACGCGGATAGAGCAGAGG-3' |
| T16LECREV: | 5'-GCAAGCTCGAGGCGGCCGCTCATGTGTGTGGCAACAGCTGCC-3' |

PCR amplifications were performed with 10 ng GalNAc-transferase plasmid DNA as template and High Fidelity PCR kit (Roche) with conditions recommended by the manufacturer. Amplified products were digested with *Hind*III and *Xho*I and inserted into the HindIII/XhoI site of pBKS-HistagI. All constructs were fully sequenced. Tagged lectin domain constructs were excised with *Not*I and sub-cloned into the *Not*I site of pAcGP67-A Baculo expression vector.

The exact borders of the lectin domains and the catalytic units have not been defined, but multiple sequence alignment analysis (Fig. 2) was used to predict the most likely borders and these were used for design of PCR primers as listed in Table II. DNA and amino acid sequences of preferred constructs of GalNAc-transferase lectin domains and their construct design include the following (Table III):

**Table III: DNA and amino acid sequences of GalNAc-transferase lectin domains.**

| |
|---|
| **GalNAc-T1 lectin domain:** |
| The lectin domain polypeptide sequence comprises amino acid residues 393-559 of *GALNT1* (GALNT1 nucleotide sequence accession number is AJ505952) |
| |
| |
| **GalNAc-T2 lectin domain:** |
| The lectin domain polypeptide sequence comprises amino acid residues 408-571 of GALNT2 (GALNT2 nucleotide sequence accession number is X85019). |
| |
| |
| |
| **GalNAc-T3 lectin domain:** |
| The lectin domain polypeptide sequence comprises amino acid residues 467-633 of *GALNT3* (GALNT3 nucleotide sequence accession number is AJ505954). |
| |
| |
| **GalNAc-T4 lectin domain:** |
| The lectin domain polypeptide sequence comprises amino acid residues 405-578 of *GALNT4* (GALNT4 nucleotide sequence accession number is YO8564). |
| |
| |
| **GalNAc-T5 lectin domain:** |
| The lectin domain polypeptide sequence comprises amino acid residues 486-653 of GALTN5 (GALTN5 nucleotide sequence accession number is AJ505956). |
| |
| |
| **GalNAc-T6 lectin domain:** |
| The lectin polypeptide sequence comprises amino acid residues 458-622 of *GALNT6* (GALNT6 nucleotide sequence accession number is AJ133523) |
| |
| |
| **GalNAc-T7 lectin domain:** |
| The lectin domain polypeptide sequence comprises amino acid residues 492-657 of *GALNT7* (GALNT7 nucleotide sequence accession number is AJ505958). |
| |
| |
| **GalNAc-T8 lectin domain:** |
| The lectin domain polypeptide sequence comprises amino acid residues 459-637 of *GALNT8* (GALNT8 nucleotide sequence accession number is AJ505959). |
| |
| |
| **GalNAc-T9 lectin domain:** |
| The lectin domain polypeptide sequence comprises amino acid residues 427-603 of *GALNT9* (GALNT9 nucleotide sequence accession number is AJ505960). |
| |
| |
| |
| **GalNAc-T10 lectin domain:** |
| The lectin domain polypeptide sequence comprises amino acid residues 417-603 of *GALNT10* (GALNT10 nucleotide sequence accession number is AJ505950). |
| |
| |
| **GalNAc-T11 lectin domain:** |
| The lectin domain polypeptide sequence comprises amino acid residues 492-608 of *GALNT11* (GALNT11 nucleotide sequence accession number is Y12434). |
| |
| |
| **GalNAc-T12 lectin domain:** |
| The lectin domain polypeptide sequence comprises amino acid residues 428-581 of *GALNT12* (GALNT12 nucleotide sequence accession number is AJ505963). |
| |
| |
| **GalNAc-T13 lectin domain:** |
| The lectin domain polypeptide sequence comprises amino acid residues 466-639 of *GALNT13* (GALNT13 nucleotide sequence accession number is AJ505964). |
| |
| |
| **GalNAc-T14 lectin domain:** |
| The lectin domain polypeptide sequence comprises amino acid residues 352-516 of *GALNT14* (GALNT14 nucleotide sequence accession number is AJ505991). |
| |
| |
| |
| **GalNAc-T15 lectin domain:** |
| The lectin domain polypeptide sequence comprises amino acid residues 382-552 of *GALNT15* (GALNT15 nucleotide sequence accession number is AJ505966). |
| |
| |
| **GalNAc-T16 lectin domain:** |
| The lectin domain polypeptide sequence comprises amino acid residues 396-558 of GALNT16 (GALNT16 nucleotide sequence accession number is AJ505951). |
| |
| |

In this *Example* we have defined minimal sequences of functional lectin domains based on multiple sequence alignments. It is clear that changes in the length of sequences used may not affect functionality of the lectins. Such changes could constitute, for example, plus or minus 10-20 amino acid residues of the GalNAc-transferase sequence at their amino or carboxy termini.. For example, the GalNAc-T1 lectin domain may comprise 10-20 fewer amino acid residues at its carboxy and/or amino termini than shown in Table III's T1 lectin domain sequence; i.e. the T1 lectin domain could, for example, stretch from amino acids 403-549 of the GALNT1 sequence, or, for example, from amino acids 413-539 of the GALNT1 sequence. Additionally, the GalNAc-T1 lectin domain may comprise 10-20 more amino acid residues at its carboxy and/or amino termini than shown in Table III's T1 lection domain sequence; i.e. the T1 lectin domain could, for example, stretch from amino acids 383-569 of the GALNT1 sequence, or, for example, from amino acids 373-579 of the GALNT1 sequence.

Sf9 cells were co-transfected with pACGP67-GalNAc-transferase soluble expression constructs and Baculo-Gold^{™} DNA (Pharmingen) as previously described ³³. Briefly, 0.4 µg DNA was mixed with 0.1 µg Baculo-Gold DNA and co-transfected in Sf9 cells in 24-well plates. Ninety-six hours post-transfection recombinant virus was amplified in 6-well plates at dilutions of 1:10 and 1:50. Titer of amplified virus was estimated by titration in 24-well plates. For large scale production and purification of recombinant secreted enzymes and lectins the amplified vira were used to infect High Five^{™} cells grown in serum free medium (Invitrogen) in upright roller bottles shaking at 140 rpm in 27 °C waterbaths. Recombinant proteins were purified by nickel NiTA chromatography using nickel agarose (Qiagen) as recommended by the manufacturer or by consecutive chromatographies on Amberlite, S-sepharose and Mono-S as previously described ³⁹.

### 2. Direct binding assay for determination of carbohydrate specificity of polypeptide GalNAc-tranferase lectins using soluble GalNAc-transferase enzymes:

GalNAc-transferase lectins were previously shown to direct GalNAc-glycopeptide substrate specificities of some GalNAc-transferase ². The mechanism by which the lectin domains mediate this specificity is unknown but the finding that the monosaccharide GalNAc selectively inhibits GalNAc-glycopeptide specificity of some isoforms suggested that the putative lectin domains were involved in an interaction with the substrate at least partly through the GalNAc-residue. Nevertheless, it has not been possible in the past despite many different attempts to demonstrate direct binding of the enzyme protein or fragments hereof to glycopeptides or saccharides ²(PCT WO 01/85215 A2). In this *Example* a binding assay using HIS-tagged affinity purified and biotinylated secreted enzyme was developed. HIS-tagged secreted human GalNAc-T2 and -T4 were prepared from pAC-GP67-T2-sol and pAC-GP67-T4-sol cDNA ^{10,32} by PCR as described in *Example 1.*

Secreted GalNAc-T2 and -T4 and variant proteins were obtained from infected High Five^{™} cells grown in serum-free medium (Invitrogen) in upright roller bottles shaken 140 rpm in waterbaths at 27°C. Purification of the recombinant proteins were performed by iminodiacetic acid metal affinity chromatography (IMAC) Ni²⁺-charged (QIAGEN). Elution was achieved with 250 mM imidazole in 50 mM sodium phosphate (pH 8.0) and 500 mM NaCl. In some cases, recombinant proteins were purified by consecutive ionexchange chromatographies as developed and described previously ³⁹, before final purification by Ni²⁺-chromatography. Proteins eluted were dialyzed three times against PBS (10 mM sodium phosphate (pH 7.4) 150 mM NaCl) and concentrated by centrifugal filter device (Millipore; 10,000 kDa cut off). Purity was analyzed by SDS-PAGE under reducing conditions, and stained for proteins with Coomassie Blue R 250.

Protein biotinylation was made as previously reported ⁴⁹. The pH of 1 ml purified protein (0.3 mg/ml) in PBS was adjusted to pH 9 with 1 M NaOH and 40 µl N-hydroxy-succinimidobiotin (Sigma) dissolved in DMF (10 mg/ml) was added. The solution was mixed end-over-end for 2 hours at room temperature, and dialyzed three times against PBS and an equal volume of glycerol was added. The biotinylated proteins were stored at -20°C in 50% glycerol until use.

Glycosylation of MUC1 peptides (0.1 mM) was made in 20 mM cacodylate buffer (pH 8.0), 10 mM MnCl₂, 10 mM UDP-GalNAc, and 20 µg purified polypeptide GalNAc-T1 or -T2 with or without subsequent glycosylation with GalNAc-T4 at 37°C during overnight. Glycopeptides were purified by C-18 reverse phase HPLC. Peptides were custom synthesized by Neosystems (Strasbourg). Biotinylated Helix Pomatia lectin (HPA) was from KemEnTec (Denmark). Anti-MUC1 HMFG2 monoclonal antibody was a generous gift from Joyce Taylor-Papadimitriou. Anti-MUC1 5E5 monoclonal antibody was developed by immunizing Balb/c mice with 60-mer MUC1 tandem repeat peptide glycosylated with 5 moles GalNAc per repeat. Monoclonal antibodies to the lectin domains of human GalNAc-T2 and -T4 were developed as previously described ^{10,50}.

Direct binding ELISA assay was developed as follows: Polystyrene microtiter plates (Maxisorb, Nunc, Denmark) were coated with peptides or enzymatically glycosylated glycopeptides in PBS overnight at 4°C. Plates were washed and blocked with 0.1 % Tween20 and 0.2% BSA in PBS for 1 h at room temperature, followed by incubation with biotinylated proteins in PBS with 0.05% Tween20 for 2 h at room temperature. After four washes with PBS, plates were incubated with 1:2,000 dilution streptavidin-HRP (Sigma) in PBS for 30 min at room temperature and washed four times with PBS. Development was performed with 0.5 mg/ml o-phenylenediamine and 0.02% H₂O₂ at room temperature for 15 min, and reaction stopped by adding 100 µl/well of 0.5 N H₂SO₄. Competitive inhibition assays were done at end-point titers of GalNAc-transferase proteins with one hour preincubations with inhibitor.

In initial binding experiments it was determined that secreted GalNAc-T2 and - T4 could bind their peptides substrates in the presence of 5 mM UDP and Mn⁺⁺ (may be substituted with other divalent cation), whereas no binding was observed in the absence or when 10 mM EDTA was included. This correlates with our previous findings that GalNAc-T2 can be purified on an acceptor substrate peptide in the presence of UDP and Mn⁺⁺, and can be eluted by removing UDP in EDTA ³². This binding is mediated by the catalytic unit of the enzyme, which exposes the acceptor-binding pocket only in the presence of UDP and Mn⁺⁺, predicted by the ordered catalytic reaction.

In order to selectively evaluate the binding characteristics of the putative lectin domain, assays were carried out in the absence of UDP and Mn⁺⁺. Significant binding to GalNAc-peptides was found for both GalNAc-T2 and -T4 (Figure 4).

We have previously shown that GalNAc-T4 with a single amino acid change in the lectin domain selectively impairs the GalNAc-glycopeptide substrate specificity of this enzyme². In agreement with this the lectin mutated enzyme protein did not bind GalNAc-glycopeptides. The binding to GalNAc-glycopeptides were unaffected by Ca++ and EDTA further confirming that the catalytic units of the enzyme proteins are not involve in binding.

GalNAc and GalNAcα-benzyl have previously been shown to inhibit the GalNAc-glycopeptide substrate specificity of GalNAc-T4 ² (PCT WO 01/85215 A2). Lectins and antibodies to carbohydrates usually recognize the anomeric configuration of the sugar structures they bind. However, surprisingly, both GalNAc-T2 and -T4 exhibit equal inhibition with GalNAcα-benzyl (Sigma) and GalNAcβ-benzyl (NuRx, Alberta Research Council (Fig. 5).^{58,59} Similar results were obtained with other aryl derivatives.

The methods described in this *Example* utilize recombinant polypeptide GalNAc-transferases in binding assays and excludes potential binding activity through the catalytic unit. It is clear that recombinant polypeptide GalNAc-transferases with mutations that inactivates the binding activity of the catalytic unit can be used, as well as any truncation of the enzyme protein that eliminate the binding activity of the catalytic unit.

While this binding assay establishes a method for screening for inhibitors of lection mediated binding mediated through the lectins of human GalNAc-T2 and GalNAc-T4, it is clear that the same method with modifications can be applied to all animal and mammalian polypeptide GalNAc-transferases with a functional lectin domain. The ligand target used in this *Example* is a GalNAc-MUC1 glycopeptide produced enzymatically from synthetic peptides. It is clear that GalNAc-glycopeptides based on any number of peptides with GalNAc attached can be used as target for the binding assay. It is also clear that the assay developed can be modified to accommodate high through put screening by any assay method available in the prior art that can detect and quantify binding between the polypeptide GalNAc-tranferase mediated through its lectin domain and a suitable ligand.

### 3. Direct binding assay for determination of carbohydrate specificity of polypeptide GalNAc-tranferase lectins using truncated GalNAc-transferase lectin domains:

In *Example* 2 it was established that secreted soluble enzymatically active proteins of GalNAc-T2 and -T4 bind GalNAc-MUC1 glycopeptides, and that GalNAc could inhibit the binding. The catalytic unit of polypeptide GalNAc-transferase can interact and bind acceptor substrate peptides and possible glycopeptides ³⁸, however, binding studies without donor substrates (UDP), and in the presence of EDTA to chelate Mn²⁺, suggested that the binding was not mediated through the catalytic unit. In this *Example* direct binding to GalNAc-glycopeptides through the lectin domains of polypeptide GalNAc-tramferases GalNAc-T2 and -T4 was established. Attempts to express C-terminal truncated GalNAc-transferase proteins failed due to low secretion rate presumable related to folding problems and intracellular degradation. Similar phenomenon has recently been reported for GalNAc-T1 ¹⁴. Numerous attempts to express isolated lectin domains in insect cells and *P.pastoris* have failed due to low expression and apparent degradation. As described in *Example* I successful expression was finally achieved with constructs truncated as described in Table II using an expression vector with a N-terminal HIS tag and thrombin cleavage site as well as a T7 tag. HIS-tagged truncated GalNAc-T2 and -T4 lectins were expressed and purified as described in *Example* 2. Lectins were used in binding studies directly or after biotinylation as described in *Example* 2. In binding studies using lectins without biotinylation antibodies to the HIS-tag and the T7-tag, or in some experiments antibodies raised to GalNAc-T2 and - T4 enzymes were used to detect binding.

Inhibition experiments were used to further define the binding specificity of GalNAc-T2 and -T4 secreted soluble enzymes as well as lectin domains compared to Helix Pomatia lectin (Table IV). GalNAcα- and GalNAcβ-aryl structures inhibited binding of both enzymes and isolated lectins at comparable levels. In contrast, Helix Pomatia showed strong preference for GalNAcα-derivatives. Gal and other sugars had no inhibitory effect. Interestingly, UDP-GalNAc was not a significant inhibitor of the GalNAc-transferase lectin binding, but a strong inhibitor of Helix Pomatia binding.

**Table IV. Inhibition of carbohydrate-binding proteins by using saccharides and related structures.**

| Carbohydrates | T21d | sT2 | T41d | sT4 | HPA |
|---|---|---|---|---|---|
| Glc | >100^{a} | >100 | >100 | >100 | >100 |
| GlcNAc | >100 | >100 | >100 | >100 | 8 |
| BzlαGlcNAc | >100 | >100 | >100 | >100 | 2 |
| BzlβGlcNAc | >100 | >100 | >100 | >100 | >20 |
| Gal | >100 | >100 | >100 | 50 | >100 |
| MeαGal | >100 | >100 | >100 | 50 | >100 |
| MeβGal | >100 | >100 | >100 | 40 | >100 |
| GalNAc | 37 | 15 | 5 | 1 | 2 |
| BzlαGalNAc | 20 | 15 | 5 | 1 | 0.5 |
| PhlαGalNAc | 15 | 5 | 5 | 1 | 1 |
| oNPαGalNAc | 12 | 7 | 5 | 0.5 | 1.5 |
| oNPβGalNAc | >12 | 10 | 8 | 1 | >20 |
| pNPαGalNAc | >10 | >10 | 10 | 1 | 2 |
| pNPβGalNAc | >10 | >10 | 8 | 0.8 | >20 |
| UDPαGalNAc | >100 | 65 | 50 | 30 | 2 |
| UDP | >100 | >100 | >100 | >100 | >100 |
| Lactose | >100 | >100 | >100 | >100 | ND |
| EDTA | >10 | >10 | >10 | >10 | >10 |

| | | | | | |
|---|---|---|---|---|---|
| ^{a} Concentration (mM) required for 50% inhibition (IC50). ND, not determined. | | | | | |

While this binding assay establishes a method for screening for inhibitors of isolated human GalNAc-T2 and GalNAc-T4 lections, it is clear that the same method with modifications can be applied to all animal and mammalian polypeptide GalNAc-transferase lectins. The ligand target used in this *Example* is a GalNAc-MUC1 glycopeptide produced enzymatically from synthetic peptides. It is clear that GalNAc-glycopeptides based on any number of peptides with GalNAc attached can be used as target for the binding assay. It is also clear that the assay developed can be modified to accommodate high through put screening by any assay method available in the prior art that can detect and quantify binding between the isolated lectin and a suitable ligand.

The methods described in this *Example* utilize recombinant GalNAc-transferase lectins in binding assays which excludes potential binding activity through the catalytic unit. It is clear that recombinant polypeptide GalNAc-transferases with mutations that inactivates the binding activity of the catalytic unit can be used, as well as any truncation of the enzyme protein that eliminate the binding activity of the catalytic unit.

### 4. Establishment of cell line model systems for cell surface expression of mucin and secreted mucin - Stably transfected CHO and CHO 1d1D cells:

*Cell lines and expression constructs:* Wild type Chinese Hamster Ovary cells (CHO) and the glycosylation deficient mutant cell line CHO 1d1D⁴⁰ were stably transfected with a full coding MUC1 construct (MUC1F, supplied by M.A. Hollingsworth, Nebraska, USA) containing 32 tandem repeats using the pCDNA3 vector (Invitrogen). A secreted MUC1 construct (MUC1-IgGHIS) was generate by insertion of mouse IgGγ2a domain fused to 6x histidine tag at the BsU36I site downstream of the tandem repeat region of MUC1F⁵¹. Cells were generally grown in Hams F12 containing 10% Fetal Bovine Serum at 37° C at 5% CO₂, and plated 12-24 hours prior to transfection in 6 well plates and grown to approximately 50% confluency. One hour before transfection cells were washed in serum free medium Optimem (Invitrogen) and cells were transfected with 1-2 µg DNA using the Lipofectamine plus reagent (Invitrogen) in a total volume of 1 mL as recommended by the supplier. Three hours after the transfection one mL of Hams F12 containing 10% Fetal Bovine Serum was added and cells grown 24-48 hours before medium was replaced with 2 mL Hams F12 containing 10% Fetal Bovine Serum. Two to three days after transfection cells were trypzinized and plated in 75 mL T-flasks or in 24/96 well microtiter plates in the same medium containing the appropriate selection agent (1 mg/mL G418 or 0.4 mg/mL Zeocin). Selection medium was changed twice weekly until clones appeared. The medium used for CHO 1d1D cells included 1 mM GalNAc and 0.1 mM Gal. Transfectant clones were selected by immunocytology with anti-MUC1 antibodies and SDS-PAGE western blot analysis to demonstrate cell surface expression and secretion of MUC1.

*Immunocytology:* Two different procedures were applied: i) For general screening purposes, cells grown in plates or flasks were trypsinized, washed in saline, and air-dried on multiwell coverslides. Slides were fixed in ice-cold acetone and stained with monoclonal antibodies and FITC-conjugated rabbit anti-mouse Ig as previously described ⁵⁰. ii) For analysis of cell surface expression, cells were seeded in 6 well plates and grown for 6 hours in Hams F12 medium with serum until approximately 30-50 % subconfluent. Medium was hereafter replaced with Optimem supplemented with 1.0 mM GalNAc and 0.1 mM Gal and cells grown for 18 to 42 hours. Cells were washed once in PBS (phosphate buffered saline without Calcium and Magnesium) and subsequently fixed in 2 ml 3% paraformaldehyde at 25°C for 20 min followed by three washes with PBS. Free aldehyde groups were quenched by incubating in 2 mL 50 mM Ammonium Chloride in PBS for 10 min, followed by three washes with PBS and three washes with 5 min incubations each with PBS containing 0.2 % Fish Skin Gelatin (Sigma). Immunostaining of cells was performed by incubation with monoclonal antibodies for 40 min at 25°C, followed by three washes with PBS and three washes of 5 min each with PBS containing 0.2 % Fish Skin Gelatin. Subsequently, cells were incubated with FITC-conjugated rabbit anti-mouse Ig (Dako, F261) diluted 1: 150 in PBS containing 0.2 % Fish Skin Gelatin) for 20 min at 25°C, followed by the same washing procedure, after which wells were cut out of plates and mounted with glycerol as for glass slides.

*Characterization of wild type and MUC1 stable transfectant CHO cells:* Several representative clones expressing the full coding or secreted MUC1 construct were selected and characterized for expression of MUC1 as well as O-glycosylation. Wild type CHO/MUC1F-clonel expressed MUC1 at the cell surface as detected by anti-MUC1 monoclonal antibodies on non-permeabilized cells, while wild type CHO/MUC1solcloneC4 only was labeled weakly at the surface. Staining with a panel of anti-MUC1 antibodies of permeabilized cells showed intracellular accumulation of MUC1 detected by HMFG2 (general anti-MUC1 reactive, ⁵²), SM3 (reactive with cancer-associated MUC1, ⁵²), VU-4H5 (reactive with low density O-glycosylated MUC1, ⁵³), VU-2G7 (reactive with high density O-glycosylated MUC1, ⁵⁴), and a novel antibody 5E5 reactive exclusively with STn/Tn-glycosylated MUC1 glycoforms. In contrast, staining of non-permeabilized cells were only reactive with the anti-MUC1 antibodies HMFG2, SM3 and weakly VU-2G7. Analysis of O-glycosylation using a panel of anti-carbohydrate monoclonal antibodies revealed that wild type CHO cells label very weakly with anti-T antibodies (HH8, 3C9, ⁵⁵ at the surface after neuraminidase treatment, while untreated cells are negative indicating that wild type CHO cells express very little O-glycoproteins and the glycosylation is mainly of sialylated core I structure (ST) (Fig. 6). Antibodies to Tn (1E3, 5F4, ⁵⁵) were weakly reactive without and with neuraminidase treatment and antibodies to STn (TKH2, 3F1, ⁵⁵) were negative. Staining with the lectins PNA (T), HPA (Tn), SNA (α2,6sialic acid) and MAA (α2,3sialic acid) were in agreement except the finding of weak reactivity with SNA indicating some presence of α2,6 linked sialic acids which may be derived from N-linked or O-linked glycans. These results demonstrate that the main form of O-glycosylation found on MUC1 expressed in wild type CHO is the sialyl-T structure as found for other recombinant glycoproteins ⁵⁶.

Staining of permeabilized wild type CHO/MUC1sol-clone-C4 with anti-MLTC1 antibodies revealed strong intracellular expression of MUC1 with HMFG2, SM3, vu-4H5, and vu-2G7 (Fig. 6). Staining with anti-carbohydrate antibodies revealed strong intracellular staining with anti-T after neuraminidase only, while anti-Tn only labeled weakly. These results indicate that the main glycosylation of MUC1 in CHO wild type is ST similar to untransfected cells.

In order to characterize the secreted MUC1 product SDS-PAGE western blot analysis of harvested culture medium of confluent cultures were performed. Ten to twenty µL culture supernatant was analysed directly or treated with 0.1 U/mL neuraminidase (C. Perfringes VI, Sigma) for 30-60 min at 37°C. Samples were mixed with SDS sample buffer, reduced with DTT, and run on precast 4-20 % gels (Biorad). As shown in Figure 7 anti-MUC1 antibodies detected two forms of MUC1 in the medium; a low molecular weight form migrating as 130-140 kd corresponding to unglycosylated product, and a high molecular weight form migrating above 250 kd. The high molecular weight form was sensitive to neuraminidase treatment as evidenced by a marked shift and retardation in migration. It is known that sialylated glyoproteins migrate aberrantly and often desialylation results in slower migration by SDS-PAGE analysis regardless of the mass. Interestingly, the antibody VU-4H5 reacted mainly with the unglycosylated form and only a very weak band was found in the high molecular weight forms after neuraminidase treatment. This result indicates that the PDTR region is O-glycosylated as the VU-4H5 antibody was previously found to tolerate O-glycosylation most positions in the tandem repeat except the PDTR region ⁵³. In agreement with this the antibody VU-2G7 raised against a MUC1 GalNAc-glycopeptide with only one GalNAc per repeat attached in the PDTR region reacted strongly with the secreted MUC1. Furthermore, reactivity with the anti-T antibody after neuraminidase treatment showed that the main type of O-glycosylation on secreted MUC1 was sialylated-T. Anti-Tn and STn produced no staining.

Characterization *of mutant and MUC1 stable transfectant CHO 1d1D cells:* CHO 1d1D cells stably transfected with full coding MUC1, e.g. CHO1d1D/MUC1F-clone2, expressed MUC1 at the cell surface as detected by anti-MUC1 antibodies when cells were grown in GalNAc and Gal (Fig. 8). Cells were seeded at approximately 30-50 % confluency (approx. 0.2 X 10⁶ per 6 well plate) in Hams F 12 medium supplemented with 10% Fetal Bovine serum and grown for 6 hours. Medium was replaced with Optimem with or without 1.0 mM GalNAc and/or 0.1 mM Gal, and cells grown for 18-36 hours after which cells were trypsinised and washed in saline and processed as described for immunocytology. CHO1d1D/MUC1F-clone2 cells grown in the absence of sugars and analysed after permeabilization produced very little MUC1 detectable by HMFG2 but not by 5E5. In contrast, cells grown in the presence of only GalNAc strongly expressed MUC1 as evaluated by HMFG2 and 5E5, specifically reactive with GalNAc-MUC1. In agreement with reactivity with 5E5 these cells also labeled strongly with anti-Tn antibodies, 5F4 and 1E3, while anti-T antibodies, HH8 and 3C9, did not label the cells. Very weak or no staining with anti-STn antibodies (3F1 and TKH2) indicates that α2,6 sialylation to form STn does not occur in CHO 1d1D cells.
CHO1d1D/MUC1F-clone2 cells grown in the presence of both GalNAc and Gal show reactivity at the surface with anti-T antibodies (HH8 and 3C9) only after neuraminidase pretreatment, confirming previous results that the predominiant glycoform in CHO cells is sialyl-T (Fig 8). No staining with anti-Tn or STn antibodies was detected with cells grown in both Gal and GalNAc. CHO1d1D/MUC1F-clone2 cells grown in the absence of GalNAc and Gal or in the presence of GalNAc alone showed no reactivity with anti-Tn and T antibodies or lectins (DBA, HPA, VVA, PNA, not shown) were detected indicating complete lack of O-glycosylation (Fig 8). Cell surface expression of MUC1 was detected in CHO1d1D/MUC1F-clone2 cells grown in the presence of GalNAc, while cells grown without GalNAc showed no or only weak expression of MUC1 at the surface (Fig 8). Surface expression of MUC1 was detected with HMFG2 in cells grown in GalNAc as well as cells grown in both Gal and GalNAc, however, expression analysed with the Tn/STn-MUC1 glycoform specific antibody 5E5 revealed surface expression only with cells grown in the presence of GalNAc (Fig 8). This latter finding is in agreement with the O-glycosylation pattern determined above in these cells.

CHO1d1D/MUCsol-cloneD5 secretes MUC1 to the culture medium, and permeabilized cells immunostain with antibodies to MUC1 in the cytoplasm. Cells were grown in Hams F12 medium supplemented with 10% Fetal Bovine serum and seeded at a density of 0.2 x10⁶ in 6 well plates. Following growth for 6 hours, the medium was replaced with Optimem supplemented with 1 mM GalNAc, 0.1 mM Gal, or 1 mM GalNAc and 0.1 mM Gal, and cells grown for 18-72 hours. Secretion of MUC1 was monitored by immunochemical assays of culture supernatants at differing time points. SDS-PAGE western blot analysis was performed with 5 µl culture supernatant mixed with 5 µl of 2x SDS sample buffer containing 1 mM DTT. Samples were heated to 100°C for 2 min and loaded on a precast 4-20% gradient gel and run at 125 V for 75 min. Transfer to nitrocellulose membrane was performed by elecroblotting using Biorad Mini Trans Blot apparatus at 350 mA for 1 hour. Membranes were blocked with 15% skimmed milk prepared in dH₂O for 2 hours and stained with anti-MUC1 and anti-carbohydrate monoclonal antibodies for 18 hours at 4 °C, followed by washing with Tris buffered saline (TBS) (10 mM Tris pH 8,0 with 8.5% NaCl) 5 times for 5 min, and incubation with with biotinylated rabbit anti-mouse IgG subclass specific antibodies (1: 1000 dilution in TBS) for 1 hour at 25°C. Following 5 washes for 5 min each in TBS, membranes were incubated in HRP conjugated Streptavidin (1: 3000 dilution in TBS) for 30 min at 25°C. After 5 washings of 5 min each in TBS the blot was developed in 0.04 % 4-Chloro-1-Naphthol prepared in 50 mM Tris-HCl (pH 7,4) containing 0.025 % H₂O₂. Similar to the findings with full coding MUC1 expressed at the cell surface of CHO 1d1D cells, glycosylation of the secreted MUC1 was dependent on Gal and GalNAc sugars in culture medium. Cells grown without sugars produced and secreted low amounts of a low molecular weight MUC1 molecule of apparent mw of 120-130 kd without glycosylation detectable by HMFG2 but not 5E5 or anti-Tn and anti-T antibodies (Fig. 9). In contrast, cells grown in 1 mM GalNAc secreted MUC1 glycosylated with GalNAc (Tn) as evidenced by reactivity with both HMFG2 and 5E5 as well as anti-Tn antibodies (Fig. 9). The apparent molecular weight of secreted Tn-MUC1 was 250-300 kd and no significant shift in migration was observed with pretreatment with neuraminidase (0.1 U/ml for 30 min at 37°C), suggesting lack of α2,6sialylation (STn). This was confirmed by lack of staining with anti-STn antibodies. Cells grown in both 0.1 mM Gal and 1 mM GalNAc produced and secreted MUC1 with sialylated core 1 (T) glycoforms reactive with HMFG2 but not 5E5 (Fig. 9). Pretreatment with neuraminidase resulted in a significant shift in migration and reactivity with anti-T antibodies as well as the lectin PNA. Two novel anti-MUC1 antibodies described recently have been suggested to react with the MUC1 tandem repeat sequence without (Mab VU-4H5) or with (Mab VU-2G7) O-glycans attached in the central immunodominant epitope -PDTR-. Analysis of secreted MUC1 produced in CHO 1d1D cells grown without GalNAc show reactivity with unglycosylated MUC1 migrating at mw 120-130 kd with VU-4H5, while no or only weak reactivity was observed when grown in GalNAc with or without Gal (Fig. 10). In contrast, the Mab VU-2G7 reacted strongly with MUC1 migrating at 250-300 kd secreted from cells grown in GalNAc with or without Gal (Fig. 10). Although, Mab VU-2G7 reacted weakly with unglycosylated MUC1 the combined results suggest that MUC1 produced in CHO 1d1D cells carry O-glycans on all five sites of the tandem repeat.

### 5. The inhibitor GalNAcα-benzyl inhibits MUC1 expression independently of O-glycan processing.

As shown in *Example* 4 CHO1d1D/MUC1F-clone2 cells grown in the presence of GalNAc but not Gal have limited O-glycosylation capacity, only produce the Tn glycoform of MUC1, but expresses comparable levels of MUC1 at the cell surface as in wild type CHO cells or in CHO 1d1D cells grown in both GalNAc and Gal. This suggested that cell surface expression was not related to O-glycosylation and particular glycoforms as previously proposed (for a review see Huet). We therefore investigated the effect of treatment with GalNAcα-benzyl of CHO1d1D/MUC1F-clone2 cells grown in the presence of GalNAc. CHO1d1D/MUC1F-clone2 cells were seeded in 6 well plates at a density of 0.2 x 10⁶ per well and were grown for 6 hours in Hams F12 medium with serum until approximately 30% subconfluent. Medium was hereafter replaced with Optimem supplemented with 1.0 mM GalNAc or 1.0 mM GalNAc and 0.1mM Gal with or without the inhibitors GalNAcα-benzyl, GalNAcβ-benzyl or the control GlcNAcα-benzyl. After 18 hours, the medium was replaced with fresh Optimem containing the sugars and benzyl derivatives as above and grown for 12-48 hours. Initially we analyzed surface expression of MUC1 by immunocytology. Cells were washed once in PBS-CMF (phosphate buffered saline Calcium and Magnesium free) after carefully removing the medium from the wells and subsequently fixed in 2 ml 3% paraformaldehyde at 25°C for 20 min followed by three washes with PBS-CMF 3 times. Free aldehyde groups were quenched by incubating in 2 mL 50 mM Ammonium Chloride in PBS-CMF for 10 min, followed by three washes with PBS-CMF and three washes with 5 min incubations each with PBS-CMF containing 0.2 % Fish Skin Gelatin (Sigma). Immunostaining of cells was performed by incubation with monoclonal antibodies for 40 min at 25°C, followed by three washes with PBS-CMF and three washes of 5 min each with PBS-CMF containing 0.2 % Fish Skin Gelatin. Subsequently, cells were incubated with FITC-conjugated rabbit anti-mouse Ig (Dako F261) diluted 1:150 in PBS-CMF containing 0.2 % Fish Skin Gelatin) for 20 min at 25°C, followed by the same washing procedure, after which wells were cut out of plates and mounted with glycerol as for glass slides. Figure 11 shows that treatment with 1 mM GalNAcα-benzyl, produced strong inhibition of cell surface expression of MUC1, while treatment with a similar control benzyl derivative showed no inhibition. GlcNAcα-benzyl was chosen as a control because this sugar does not serve as a substrate for mammalian glycosyltransferases and hence was not expected to interfere with O-glycosylation in CHO cells. Most anti-MUC1 antibodies reacted with cells grown in GalNAc including VU-2G7 and 5E5, and only VU-4H5 did not react. Reactivity with VU-2G7 indicates that the - PDTR- region is O-glycosylated, while reactivity with 5E5 confirms that the glycoforms of surface MUC1 is mainly or exclusively Tn.

We next analysed the expression of MUC1 produced by CHO1d1D/MUC1F-clone2 cells by SDS-PAGE western analysis. Cells were grown for 24 hours or 48 hours in the presence of 1 mM GalNAc or 1 mM GalNAc and 0.1 mM Gal to limit core O-glycosylation to GalNAcα1-O-Ser/Thr and Galβ1-3GalNAcα1-O-Ser/Thr, respectively. Cells were further treated with 2 mM GalNAcα-benzyl, GlcNAcα-benzyl or no inhibitor. Cells were washed and lysed at 24 or 48 hours and the lysates subjected to immunoprecipitation with monoclonal antibody HMFG2, which broadly recognize MUC1 glycoforms. Immunoprecipitates were analysed by SDS-PAGE and western blot using HMFG2 antibody to detect MUC1 expression. As shown in Figure 12 the MUC1 glycoforms at 24 hours expressed by cells grown in GalNAc or Gal and GalNAc migrated similarly with only little high molecular weight forms in both, indicating that synthesis of sialylated core 1 O-glycans were time limited. At 48 hours, MUC1 glycoforms migrating as higher molecular weight species were expressed more pronounced and selectively by cells grown in Gal and GalNAc. Treatment with GlcNAcα-benzyl produced the same glycoforms at similar intensity as cells without treatment. In striking contrast, treatment with GalNAcα-benzyl had significant effect after 48 hours. A significant reduction in MUC1 expression was found in cells grown in GalNAc as well as in Gal and GalNAc. In the latter case a significant shift in migration into two bands further confirmed that GalNAcα-benzyl also serves as an inhibitor of O-glycan extension and reduces O-glycosylation to GalNAcα1-O-Ser/Thr. Analysis of the same blots with the anti-MUC1 antibody 5E5 (Figure 13) produced essentially the same results except that the antibody only labeled the lower migrating band of the two bands labeled by HMFG2 in cells grown in Gal and GalNAc and treated with GalNAcα-benzyl. This indicates some heterogeneity in glycosylation.

These results show for the first time that the effect of GalNAcα-benzyl on mucin transport and surface expression is independent of its effects on O-glycosylation in striking contrast to the prevailing hypothesis ²⁶. Because cells grown in the presence of GalNAc do not produce core 1 (Galβ1-3GalNAcα1-O-Ser/Thr) O-glycosylation, GalNAcα-benzyl cannot serve as a competitive substrate for the core 1 β3galactosyltransferase and subsequently for sialyltransferases. GalNAcα-benzyl must therefore exert its function on mucin transport by another unknown mechanism.

The *in vivo* cell line model system developed is one example of a method to screen for inhibitors effects of one or more compounds on transport of mucins and O-linked glycoproteins in cells. The *Example* utilizes MUC1 but any mucin or O-linked glycoprotein could be used with appropriate expression constructs, antibodies and reagents. The developed cell model and modifications hereof can be used for high throughput screens of inhibitors in combination with or as a second screen after the binding assays disclosed in *Examples* 2 and 3.

### 6. Identification of a novel selective inhibitor, GalNAcβ-benzyl, of polypeptide GalNAc-transferase lectins that inhibits MUC1 expression without affecting O-glycosylation.

As shown in Examples 2 and 3, polypeptide GalNAc-transferases contain lectin domains with binding properties for GalNAc-peptides including GalNAc-MUC1 peptides. Since GalNAcα-benzyl was found to inhibit the binding properties of GalNAc-transferase lectins, we tested the possibility that the independent effect on mucin expression this O-glycosylation inhibitor has, could be related to an inhibitory effect on GalNAc-transferase lectins. In Examples 2 and 3 we found surprisingly that the lectin domains of several GalNAc-transferases in addition to GalNAcα-benzyl, which mimics the GalNAc-glycopeptide targets of the lectins, also were inhibited by βGalNAc derivatives. Initial tests with commercially available GalNAcβ and GalNAcα derivatives, p-nitrophenyl and umbrelliferyl did not produce significant effects in our model system. GalNAcβ-benzyl, the β-anomeric configuration of GalNAc-benzyl (there is a β linkage between the N-acetylgalactosamine and the benzyl ring), was custom synthesized by Alberta Research Council (Canada), and its structure was confirmed by mass spectrometry and ¹H-NMR. CHO1d1D/MUC1F-clone2 cells were grown for 12 hours in the presence of 1 mM GalNAc or 1 mM GalNAc and 0.1 mM Gal to limit core O-glycosylation to GalNAcα1-O-Ser/Thr and Galβ1-3GalNAcα1-O-Ser/Thr, respectively. Cells were then treated with 2 mM GalNAca-benzyl, GalNAcβ-benzyl, or GlcNAcα-benzyl as control (GlcNAcα-benzyl was shown in Example 4 to have no effect). Cells were washed and lysed as described above after 36 hours and the lysates subjected to immunoprecipitation with anti-MUC1 monoclonal antibodies HMFG2 or 5E5. Figure 14 illustrates that treatment with GalNAcβ-benzyl produced the same or better reduction in MUC1 expression as treatment with GalNAcα-benzyl in cells grown in GalNAc as well as in Gal and GalNAc. In cells grown in Gal and GalNAc MUC1 expression was reduced with GalNAcβ-benzyl treatment, but in contrast to cells treated with GalNAcα-benzyl, GalNAcβ-benzyl produced no change in the migration of MUC1 demonstrating that this inhibitor does not affect the O-glycan processing. The lack of immunoprecipitation of MUC1 by antibody 5E5 in cells grown in Gal and GalNAc indicates that MUC1, is glycosylated with more complex structures than Gal-NAcα1-O-Ser/Thr as recognized by this antibody. As shown in Figure 16 the main O-glycan phenotype of CHO1d1D cells grown in Gal and GalNAc is sialylated-T, and 5E5 does not react with MUC1, with T or sialylated T glycoforms of MUC1. Figure 15 illustrates the same experiment as in Figure 14 except that the detection antibody is 5E5 and only Tn and STn MUC1 glycoforms are visualised. This experiment confirms the strong inhibition of MUC1 expression in GalNAcα-benzyl and GalNAcβ-benzyl treated cells.

The finding that GalNAcα-benzyl and GalNAcβ-benzyl exhibit the same inhibitory effect on GalNAc-transferase lectin binding, and that they have similar effects on inhibition of MUC1 expression, clearly indicate that the effects these compounds have on mucin expression and secretion are directed through interaction with the lectin domains of polypeptide GalNAc-transferases. The effect GalNAcα-benzyl has on O-glycan processing is a separate phenomenon directed by its ability to serve as a competitive substrate for the core 1 β1,3galactosyltransferase.

GalNAcβ-benzyl is the first identified selective inhibitor of polypeptide GalNAc-transferase lectins and their roles in transport and secretion, which does not modulate O-glycosylation in cells (i.e. does not serve a substrate for mainly core 1 β3galactosyltransferase activities, α2,6sialyltransferase activities, and core 3 β3GlcNAc-transferase activities). Inhibitors structurally related to GalNAcβ-benzyl with the same properties, as identifiable by the binding assays disclosed in *Examples 2* and 3, may be designed and syntesized to obtain higher affinity binders. Such inhibitors may be based on carbohydrates such as the monosaccharide GalNAc or modifications thereof, inhibitors may be based on structural and functional mimetics such as polypeptides, glycopeptides, DNA, RNA, antibodies, and antibody fragments including phage antibodies, and inhibitors may be natural or synthetic organic or inorganic compounds. One common feature for such preferred inhibitors is the ability to inhibit the binding of one or more polypeptide GalNAc-transferase lectins to its binding ligand, such as GalNAc-glycopeptides and mucins as exemplified in *Examples* 2 and 3. Another feature of the novel inhibitor GalNAcβ-benzyl is its ability to enter living cells and reach the Golgi apparatus for *in vivo* binding to polypeptide GalNAc-transferase. The hydrophic benzyl aglycone is one example of an aryl compound suitable for the β-anomeric configuration of GalNAc-R, but other aryl substituents include, without limitation, p-nitrophenyl, umbrelliferyl, and naphtalenmethanol. Any pharmaceutical carrier known in the art may be used to achieve the same effect. The appropriate carrier will be evident to those skilled in the art and will depend in large part upon the route of administration.

### 7. Inhibition of secretion of mucins:

Because GalNAca-benzyl exerts separate effects on O-glycan processing and mucin expression, the use of the novel selective inhibitor of mucin expression, Gal-NAcβ-benzyl, allow analysis of mucin expression and secretion in different cell line models. Examples of human cell lines (available from ATCC, USA) expressing and secreting mucins are without limitations LS174T, HT29, Colo205, CALU, MCF7, T47D, NCI-H292, and A549. Most human adenocarcinoma cell lines express and secret mucins and analysis with antibodies to detect protein expression or probes to detect mRNA can reveal the types and quantities of mucins. The human colon carcinoma cell line LS 174T was previously shown to exhibit reduced secretion of mucin following treatment with GalNAcα-benyl ⁵⁷. This and other cell lines can be used to treat with 2 mM GalNAcβ-benzyl. In this *Example* we used wild type CHO/MUC1sol-cloneC4 and western blot analysis of medium of cells treated with 1-2 mM GalNAcα-benzyl, Gal-NAcβ-benzyl, or the control GlcNAcα-benzyl. Treatment with both GalNAcα-benzyl and GalNAcβ-benzyl showed inhibition of secreted MUC1 compared to control treated or non-treated cells. The inhibitory effect on mucin secretion can be quantified by a number of assays known to the skilled in the art including western blot, ELISA, gelfiltration, immunocapture, and other assays.

The *in vivo* cell line model system developed is one example of a method to screen for inhibitors effects of one or more compounds on secretion of mucins and O-linked glycoproteins in cells. The *Example* utilizes MUC1 but any mucin or O-linked glycoprotein could be used with appropriate expression constructs, antibodies and reagents. The developed cell model and modifications hereof can be used for high throughput screens of inhibitors in combination with or as a second screen after the binding assays disclosed in Examples 2 and 3.

### 8. GalNAcβ-benzyl inhibits mucin glycosylation and MUC5AC production in HT29MTX cells.

In order to evaluate GalNAcβ-benzyl as an inhibitor of mucin glycosylation and secretion we used the cultured colon carcinoma cells of mucin secreting phenotypes: HT-29 metotrexate (MTX) cells selected from the HT-29 cell line by culture in the presence of MTX (kind gift from G. Huet, France). The HT-29 MTX cells were selected to yield a goblet cell like phenotype with constitutive production of MUC5AC. In order to ensure proper goblet cell differentiation the HT29MTX cell line were continuously grown up to 21 days in the presence of 5 mM GalNAcα-benzyl, 5 mM GalNAcβ-benzyl, 5 mM GlcNAcα-benzyl and without inhibitor using culture conditions as described in Hennebicq-Reig et al. (Permanent exposure of mucin-secreting HT-29 cells to benzyl-N-acetyl-alpha-D-galactosaminide induces abnormal O-glycosylation of mucins and inhibits constitutive and stimulated MUC5AC secretion. Biochem J. 1998;334:283-95). At 7, 14 and 21 days of continuous culture, media was collected and cells were fixed in 3% paraformaldehyde and stained for the presence of MUC5AC and specific carbohydrate structures using immuno-histochemistry with the following monoclonal antibodies: CLH2 recognizing MUC5AC (Reis C. et al. Immunohistochemical study of MUC5AC expression in human gastric carcinomas using a novel monoclonal antibody. Int. J. Cancer. 1997;74:112-21), 1E3 recognizing Tn (GalNAcα linked to serine or threonine), 3C9 recognizing T (Galβ1-3GalNAcα linked to serine or threonine, and 3F1 recognizing Sialyl-Tn (NeuAcα2-6GalNAcα linked to serine or threonine. In order to evaluate the amount of MUC5AC secreted by HT-29 MTX cells with and without GalNAcβ-benzyl treatment, media were subjected to SDS-PAGE (Tris-NuPAGE Gels 14-21 %) followed by Western blotting using polyclonal rabbit anti human MUC5AC antibody LUM5-1 (Carlstedt et al. MUC5AC, but not MUC2, is a prominent mucin in respiratory secretions. Glycoconj J. 1996;13:839-47). Furthermore the viscosity of the media from cells with and without inhibitor was evaluated using viscosity micro-measurement.

Treatment of cells with GalNAcβ-benzyl and GalNAcα-benzyl diminished secretion of MUC5AC as evaluated by western blot compared with GlcNAcβ-benzyl and cells treated with media alone (Fig. 17). Furthermore in accordance with earlier finding (Hennebicq-Reig et al. Permanent exposure of mucin-secreting HT-29 cells to benzyl-N-acetyl-alpha-D-galactosaminide induces abnormal O-glycosylation of mucins and inhibits constitutive and stimulated MUC5AC secretion. Biochem J. 1998;334:283-95) treatment of cells with GalNAcα-benzyl (Fig. 18, Panel A) induced a storage phenotype with increased intracellular staining of MUC5AC. In contrast cells treated with Gal-NAcβ-benzyl (Fig. 18, Panel B) did not lead to any storage disease but diminished intracellular staining of MUC5AC compared with and GlcNAcβ-benzyl (Fig 18, panel C) and cells treated with media alone (Fig 18, panel D). In addition the O-linked mucin carbohydrate structures T, Tn and sialyl-Tn were upregulated in cells treated with GlcNAcβ-benzyl compared to controls. Media from GalNAcβ-benzyl treated cells were less viscous compared with control media (data not shown). In conclusion the novel inhibitor GalNAcβ-benzyl inhibits both O-linked mucin glycosylation and MUC5AC secretion.

### Reference List

1. Paulson, J. C. and Colley, K. J. Glycosyltransferases. Structure, localization, and control of cell type-specific glycosylation. J.Biol.Chem., 264: 17615-17618, 1989.
2. Hassan, H., Reis, C. A., Bennett, E. P., Mirgorodskaya, E., Roepstorff, P., Hollingsworth, M. A., Burchell, J., Taylor-Papadimitriou, J., and Clausen, H. The lectin domain of UDP-N-acetyl-D-galactosamine: polypeptide N-acetylgalactosaminyltransferase-T4 directs its glycopeptide specificities. J Biol Chem, 275: 38197-38205, 2000.
3. Schwientek, T., Bennett, E. P., Flores, C., Thacker, J., Hollmann, M., Reis, C. A., Behrens, J., Mandel, U., Keck, B., Schafer, M. A., Haselmann, K., Zubarev, R., Roepstorff, P., Burchell, J. M., Taylor-Papadimitriou, J., Hollingsworth, M. A., and Clausen, H. Functional conservation of subfamilies of putative UDP-N-acetylgalactosamine:polypeptide N-acetylgalactosaminyltransferases in Drosophila, Caenorhabditis elegans, and mammals. One subfamily composed of 1(2)35Aa is essentail in Drosophila. J.Biol.Chem., 277: 22623-22638, 2002.
4. Hagen, F. K., Hazes, B., Raffo, R., deSa, D., and Tabak, L. A. Structure-Function Analysis of the UDP-N-acetyl-D- galactosamine:Polypeptide N-acetylgalactosaminyltransferase. Essential residues lie in a predicted active site cleft resembling a lactose repressor fold. J Biol Chem, 274: 6797-6803, 1999.
5. Hazes, B. The (QxW)3 domain: a flexible lectin scaffold. Protein Science, 5: 1490-1501, 1996.
6. Imberty A., Piller V., Piller F., and Breton C. Fold recognition and molecular modeling of a lectin-like domain in UDP-GalNac:polypeptide N-acetylgalactosaminyltransferases. Protein Eng., 10: 1353-1356, 1997.
7. BRETON, C. and Imberty, A. Structure/function studies of glycosyltransferases. Curr.Opin.Struct.Biol, 9: 563-571, 1999.
8. Bennett, E. P., Hassan, H., Hollingsworth, M. A., and Clausen, H. A novel human UDP-N-acetyl-D-Galactosamine:polypeptide N-acetylgalactosaminyltransferase, GalNAc-T7, with specificity for partial GalNAc-glycosylated acceptor substrates. FEBS Letters, 460: 226-230, 1999.
9. Ten Hagen, K. G., Bedi, G. S., Tetaert, D., Kingsley, P. D., Hagen, F., BALYS, M. M., BERES, T. M., Degand, P., and Tabak, L. A. Cloning and Characterization of a Ninth Member of the UDP-GalNAc:Polypeptide N-Acetylgalactosaminyltransferase Family, ppGaNTase-T9. J Biol Chem, 276: 17395-17404, 2001.
10. Bennett, E. P., Hassan, H., Mandel, U., Mirgorodskaya, E., Roepstorff, P., Burchell, J., Taylor-Papadamitriou, J., Hollingsworth, M. A., Merkx, G., Geurts van Kessel, A., Eiberg, H., Steffensen, R., and Clausen, H. Cloning of a human UDP-N-acetyl-α-D-galactosamine: polypeptide N-acetylgalactosaminyltransferase that complements other GalNAc-transferases in complete O-glycosylation of the MUC1 tandem repeat. J.Biol.Chem., 273: 30472-30481, 1998.
11. Ten Hagen, K. G., Tetaert, D., Hagen, F. K., Richet, C., BERES, T. M., Gagnon, J., BALYS, M. M., VanWuyckhuyse, B., Bedi, G. S., Degand, P., and Tabak, L. A. Characterization of a UDP-GalNAc:polypeptide N-acetylgalactosaminyltransferase that displays glycopeptide N-acetylgalactosaminyltransferase activity. J.Biol.Chem., 274: 27867-27874, 1999.
12. Muller, S., Alving, K., Peter-Katalinic, J., Zachara, N., Gooley, A. A., and Hanisch, F. G. High density O-glycosylation on tandem repeat peptide from secretory MUC1 of T47D breast cancer cells. J.Biol.Chem., 274: 18165-18172, 1999.
13. Muller, S., Goletz, S., Packer, N., Gooley, A. A., Lawson, A. M., and Hanisch, F. G. Localization of O-glycosylation sites on glycopeptide fragments from lactation-associated MUC1. J.Biol.Chem., 272: 24780-24793, 1997.
14. Tenno, M., Saeki, A., Kezdy, F. J., Elhammer, A. P., and Kurosaka, A. The lectin domain of UDP-GalNAc: Polypeptide N-acetylgalactosaminyltransferase I (GalNAc-T1) is involved in O-glycosylation of a polypeptide with multiple acceptor sites. J.Biol.Chem., 2002.
15. Jentoft, N. Why are proteins O-glycosylated? Trends.Biochem Sci 1990.Aug., 15: 291-294.
16. Tabak, L. A. In defense of the oral cavity: structure, biosynthesis, and function of salivary mucins. Annual Review of Physiology, 57: 547-564, 1995.
17. Van den Steen, P., Rudd, P. M., Dwek, R. A., and Opdenakker, G. Concepts and principles of O-linked glycosylation. Crit.Rev.Biochem Mol.Biol 1998., 33: 151-208.
18. Taylor-Papadimitriou, J. and Epenetos, A. A. Exploiting altered glycosylation patterns in cancer: progress and challenges in diagnosis and therapy. Trends In Biotechnology, 12: 227-233, 1994.
19. Taylor-Papadimitriou, J. and Finn, O. J. Biology, biochemistry and immunology of carcinoma-associated mucins. Immunology Today, 18: 105-107, 1997.
20. Scharfman, A., Lamblin, G., and Roussel, P. Interactions between human respiratory mucins and pathogens. Biochemical Society Transactions, 23: 836-839, 1995.
21. Rose, M. C. Mucins: structure, function, and role in pulmonary diseases. Am.J Physiol. 1992.Oct., 263: L413-L429.
22. Thomsson, K. A., Carlstedt, I., Karlsson, N. G., Karlsson, H., and Hansson, G. C. Different O-glycosylation of respiratory mucin glycopeptides from a patient with cystic fibrosis. Glycoconj.J 1998.Aug., 15: 823-833.
23. Kuan, S. F., Byrd, J. C., Basbaum, C., and Kim, Y. S. Inhibition of mucin glycosylation by aryl-N-acetyl-alpha-galactosaminides in human colon cancer cells. J.Biol.Chem., 264: 19271-19277, 1989.
24. Huet, G., Hennebicq-Reig, S., de Bolos, C., Ulloa, F., Lesuffleur, T., Barbat, A., Carriere, V., Kim, I., Real, F. X., Delannoy, P., and Zweibaum, A. GalNAc-alpha-O-benzyl inhibits NeuA-calpha2-3 glycosylation and blocks the intracellular transport of apical glycoproteins and mucus in differentiated HT-29 cells. J.Cell.Biol., 141: 1311-1322, 1998.
25. Byrd, J. C., Dahiya, R., Huang, J., and Kim, Y. S. Inhibition of mucin synthesis by benzyl-alpha-GalNAc in KATO III gastric cancer and Caco-2 colon cancer cells. Eur J Cancer 1995., 31A: 1498-1505.
26. Gouyer, V., Leteurtre, E., Zanetta, J. P., Lesuffleur, T., Delannoy, P., and Huet, G. Inhibition of the glycosylation and alteration in the intracellular trafficking of mucins and other glycoproteins by GalNAcalpha-O-bn in mucosal cell lines: an effect mediated through the intracellular synthesis of complex GalNAcalpha-O-bn oligosaccharides. Front Biosci., 6: D1235-D1244, 2001.
27. Alfalah, M., Jacob, R., Preuss, U., Zimmer, K. P., Naim, H., and Naim, H. Y. O-linked glycans mediate apical sorting of human intestinal sucrase-isomaltase through association with lipid rafts. Curr.Biol, 9: 593-596, 1999.
28. Yeaman, C., Le Gall, A. H., Baldwin, A. N., Monlauzeur, L., Le Bivic, A., and Rodriguez-Boulan, E. The O-glycosylated stalk domain is required for apical sorting of neurotrophin receptors in polarized MDCK cells. J Cell Biol 1997.Nov.17., 139: 929-940.
29. Ulloa, F., Franci, C., and Real, F. X. GalNAc-a-O-Benzyl inhibits sialylation of de novo synthesized apical, but not basolateral, sialoglycoproteins and blocks lysosomal enzyme processing in a post-TGN compartment. J Biol Chem 2000.Apr.5..
30. Muller, S. and Hanisch, F. G. Recombinant MUC1 probe authentically reflects cell-specific O-glycosylation profiles of endogenous breast cancer mucin: High-density and prevalent core2-based glycosylation. J.Biol.Chem., 2002.
31. Hassan, H., Bennett, E. P., Mandel, U., Hollingsworth, M. A., and Clausen, H. Control of Mucin-Type O-Glycosylation: O-Glycan Occupancy is Directed by Substrate Specificities of Polypeptide GalNAc-Transferases. In B. Ernst, G. W. Hart, and P. Sinaÿ (eds.), Carbohydrates in Chemistry and Biology, vol. 3, 1 ed, pp. 273-292. New York: Wiley-VCH, 2000.
32. White, T., Bennett, E. P., Takio, K., Sorensen, T., Bonding, N., and Clausen, H. Purification and cDNA cloning of a human UDP-N-acetyl-alpha-D- galactosamine:polypeptide N-acetylgalactosaminyltransferase. J.Biol.Chem., 270: 24156-24165, 1995.
33. Bennett, E. P., Hassan, H., and Clausen, H. cDNA cloning and expression of a novel human UDP-N-acetyl-alpha- D-galactosamine. Polypeptide N-acetylgalactosaminyltransferase, GalNAc-T3. J.Biol.Chem., 271: 17006-17012, 1996.
34. Bennett, E. P., Hassan, H., Mandel, U., Hollingsworth, M. A., Akisawa, N., Ikematsu, Y., Merkx, G., Geurts van Kessel, A., Olofsson, S., and Clausen, H. Cloning and Characterization of a Close Homologue of Human UDP-N-acetyl-α-D-galactosamine: Polypeptide N-Acetylgalactosaminyltransferase T3, designated GalNAc-T6: Evidence for Genetic but not Functional Redundancy. J.Biol.Chem., 274: 25362-25370, 1999.
35. White, K. E., Lorenz, B., Evans, W. E., Meitinger, T., Strom, T. M., and Econs, M. J. Molecular cloning of a novel human UDP-GalNAc:polypeptide N-acetylgalactosaminyltransferase, GalNAc-T8, and analysis as a candidate autosomal dominant hypophosphatemic rickets (ADHR) gene. Gene, 246: 347-356, 2000.
36. Toba, S., Tenno, M., Konishi, M., Mikami, T., Itoh, N., and Kurosaka, A. Brain-specific expression of a novel human UDP-GalNAc:polypeptide N-acetylgalactosaminyltransferase (GalNAc-T9). Biochim.Biophys.Acta, 1493: 264-268, 2000.
37. Homa, F. L., Hollander, T., Lehman, D. J., Thomsen, D. R., and Elhammer, A. P. Isolation and expression of a cDNA clone encoding a bovine UDP- GalNAc:polypeptide N-acetylgalactosaminyltransferase. J.Biol.Chem., 268: 12609-12616, 1993.
38. Sorensen, T., White, T., Wandall, H. H., Kristensen, A. K., Roepstorff, P., and Clausen, H. UDP-N-acetyl-alpha-D-galactosamine:polypeptide N- acetylgalactosaminyltransferase. Identification and separation of two distinct transferase activities. J.Biol.Chem., 270:24166-24173, 1995.
39. Wandall, H. H., Hassan, H., Mirgorodskaya, E., Kristensen, A. K., Roepstorff, P., Bennett, E. P., Nielsen, P. A., Hollingsworth, M. A., Burchell, J., Taylor-Papadimitriou, J., and Clausen, H. Substrate specificities of three members of the human UDP-N-acetyl-alpha-D-galactosamine:Polypeptide N-acetylgalactosaminyltransferase family, GalNAc-T1, -T2, and - T3. J.Biol.Chem., 272: 23503-23514, 1997.
40. Kingsley, D. M., Kozarsky, K. F., Segal, M., and Krieger, M. Three types of low density lipoprotein receptor-deficient mutant have pleiotropic defects in the synthesis of N-linked, O-linked, and lipid-linked carbohydrate chains. J.Cell Biol., 102: 1576-1585, 1986.
41. Altschuler, Y., Kinlough, C. L., Poland, P. A., Bruns, J. B., Apodaca, G., Weisz, O. A., and Hughey, R. P. Clathrin-mediated endocytosis of MUC1 is modulated by its glycosylation state. Mol.Biol.Cell, 11: 819-831, 2000.
42. Zanetta, J. P., Gouyer, V., Maes, E., Pons, A., Hemon, B., Zweibaum, A., Delannoy, P., and Huet, G. Massive in vitro synthesis of tagged oligosaccharides in 1-benzyl-2-acetamido-2-deoxy-alpha-D-galactopyranoside treated HT-29 cells. Glycobiology, 10: 565-575, 2000.
43. Trombetta, E. S. and Helenius, A. Lectins as chaperones in glycoprotein folding. Curr.Opin.Struct.Biol, 8: 587-592,1998.
44. Amado, M., Almeida, R., Carneiro, F., Levery, S. B., Holmes, E. H., Nomoto, M., Hollingsworth, M. A., Hassan, H., Schwientek, T., Nielsen, P. A., Bennett, E. P., and Clausen, H. A family of human beta3-galactosyltransferases. Characterization of four members of a UDP-galactose:beta-N-acetyl-glucosamine/beta-nacetyl-galactosamine beta-1,3-galactosyltransferase family. J.Biol.Chem., 273: 12770-12778, 1998.
45. Amado, M., Almeida, R., Schwientek, T., and Clausen, H. Identification and characterization of large galactosyltransferase gene families: galactosyltransferases for all functions. Biochim.Biophys.Acta, 1473: 35-53, 1999.
46. Dohi, T., Yuyama, Y., Natori, Y., SMITH, P. L., Lowe, J. B., and Oshima, M. Detection of N-acetylgalactosaminyltransferase mRNA which determines expression of Sda blood group carbohydrate structure in human gastrointestinal mucosa and cancer. Int.J.Cancer, 67: 626-631, 1996.
47. Colley, K. J. Golgi localization of glycosyltransferases: more questions than answers. Glycobiology, 7: 1-13, 1997.
48. Schwientek, T. J., Bennett, E. P., Flores, C., Thacker, J., Hollman, M., Reis, C. A., Behrens, J., Mandel, U., Keck, B., Schafer, M. A., Hazelmann, K., Zubarev, R., Roepstorff, P., Hollingsworth, M. A., and Clausen, H. Functional conservation of subfamilies of putative UDP-N-acetylgalactosamine: Polypeptide N-acetylgalactosaminyltransferases in drosophila, C. elegans and mammals: One subfamily comprised of 1(2)35Aa is essential in drosophila. J.Biol.Chem., 2002.
49. Jorgensen, C. S., Heegaard, N. H., Holm, A., Hojrup, P., and Houen, G. Polypeptide binding properties of the chaperone calreticulin. Eur.J.Biochem., 267: 2945-2954, 2000.
50. Mandel, U., Hassan, H., Therkildsen, M. H., Rygaard, J., Jacobsen, M., Juhl, B. R., Dabelsteen, E., and Clausen, H. Expression of polypeptide GalNAc-transferases in stratified epithelia and squamous cell carcinomas: immunohistological evaluation using monoclonal antibodies to three members of the GalNAc-transferase family. Glycobiology, 9: 43-52, 1999.
51. Burdick, M. D., Harris, A., Reid, C. J., Iwamura, T., and Hollingsworth, M. A. Oligosaccharides expressed on MUC1 produced by pancreatic and colon tumor cell lines. J.Biol.Chem., 272: 24198-24202, 1997.
52. Girling, A., Bartkova, J., Burchell, J., Gendler, S., Gillett, C., and Taylor-Papadimitriou, J. A core protein epitope of the polymorphic epithelial mucin detected by the monoclonal antibody SM-3 is selectively exposed in a range of primary carcinomas. International Journal of Cancer, 43: 1072-1076, 1989.
53. Reis, C. A., Hassan, H., Bennett, E. P., and Clausen, H. Characterization of a panel of monoclonal antibodies using GalNAc glycosylated peptides and recombinant MUC1. Tumour Biology, 19 Suppl 1: 127-133, 1998.
54. Ryuko, K., Schol, D. J., Snijdewint, F. G., Mensdorff-Pouilly, S., Poort-Keesom, R. J., Karuntu-Wanamarta, Y. A., Verstraeten, R. A., Miyazaki, K., Kenemans, P., and Hilgers, J. Characterization of a new MUC1 monoclonal antibody (VU-2-G7) directed to the glycosylated PDTR sequence of MUC1. Tumour.Biol., 21: 197-210, 2000.
55. Mandel, U., Petersen, O. W., Sorensen, H., Vedtofte, P., Hakomori, S., Clausen, H., and Dabelsteen, E. Simple mucin-type carbohydrates in oral stratified squamous and salivary gland epithelia. Journal of Investigative Dermatology, 97: 713-721, 1991.
56. Sasaki, H., Bothner, B., Dell, A., and Fukuda, M. Carbohydrate structure of erythropoietin expressed in Chinese hamster ovary cells by a human erythropoietin cDNA. J.Biol.Chem., 262: 12059-12076, 1987.
57. Kuan, S. F., Byrd, J. C., Basbaum, C., and Kim, Y. S. Inhibition of mucin glycosylation by aryl-N-acetyl-alpha- galactosaminides in human colon cancer cells. J.Biol.Chem., 264: 19271-19277, 1989.
58. Ramakrishnan, B., Balaji, P.V., and Qasba, P.K. Crystal Structure of β-1,4-Galactosyltransferase Complex with UDP-Gal Reveals an Oligosaccharide Acceptor Binding Site. J. Mol Biol., 318:491-502, 2002.
59. Ramakrishnan, B., and Qasba, P.K. Structure-based Design of β4Gal-T1 with Equally Efficient N-Acetylgalactosaminyltransferase Activity. J. Bio. Chem., 277, 23, 20833-20839, 2002.

## Claims

1. An isolated nucleic acid molecule consisting of a nucleic acid sequence encoding a truncated mammalian GalNAc-transferase polypeptide comprising a domain selected from the group consisting of the lectin domain of a mammalian polypeptide GalNAc-transferase, and a lectin-functional variant or fragment of said lectin domain, wherein:
(i) the lectin domain has lectin binding activity
(ii) the polypeptide does not encompass the intact, functioning catalytic domain of the enzyme.

2. A nucleic acid molecule according to claim 1 comprising a nucleic acid sequence selected from the group consisting of the nucleic acid sequences encoding the GalNAc-T1 [SEQ ID NO: 2], GalNAc-T2 [SEQ ID NO: 4], GalNAc-T3 [SEQ ID NO: 6], GalNAc-T4 [SEQ ID NO: 8], GalNAc-T5 [SEQ ID NO: 10], GalNAc-T6 [SEQ ID NO: 12], GalNAc-T7 [SEQ ID NO: 14], GalNAc-T8 [SEQ ID NO: 16], GalNAc-T9 [SEQ ID NO: 18], GalNAc-T10 [SEQ ID NO: 20], GalNAc-T11 [SEQ ID NO: 22], GalNAc-T12 [SEQ ID NO: 24], GalNAc-T13 [SEQ ID NO: 26], GalNAc-T14 [SEQ ID NO: 28], GalNAc-T15 [SEQ ID NO: 30] and GalNAc-T16 [SEQ ID NO: 32] lectin domains set forth in Table III herein.

3. The nucleic acid of claim 2 further comprising 30-60 nucleotides of the corresponding GalNAc-transferase sequence at its 5' or 3' end.

4. The nucleic acid of any one of claims 1-3 wherein the truncated GalNAc-transferase polypeptide is human.

5. An isolated lectin polypeptide consisting of a truncated mammalian GalNAc-transferase polypeptide comprising a domain selected from the group consisting of the lectin domain of a mammalian polypeptide GalNAc-transferase or a lectin-functional variant or fragment thereof, wherein:
(i) the lectin domain has lectin binding activity
(ii) the truncated polypeptide does not encompass the intact, functioning catalytic domain of the enzyme.

6. A lectin polypeptide according to claim 5 having an amino acid sequence selected from the group consisting of the amino acid sequences of GalNAc-T1 [SEQ ID NO: 2], GalNAc-T2 [SEQ ID NO: 4], GalNAc-T3 [SEQ ID NO: 6], GalNAc-T4 [SEQ ID NO: 8], GalNAc-T5 [SEQ ID NO: 10], GalNAc-T6 [SEQ ID NO: 12], GalNAc-T7 [SEQ ID NO: 14], GalNAc-T8 [SEQ ID NO: 16], GalNAc-T9 [SEQ ID NO: 18], GalNAc-T10 [SEQ ID NO: 20], GalNAc-T11 [SEQ ID NO: 22], GalNAc-T12 [SEQ ID NO: 24], GalNAc-T13 [SEQ ID NO: 26], GalNAc-T14 [SEQ ID NO: 28], GalNAc-T15 [SEQ ID NO: 30] and GalNAc-T16 [SEQ ID NO: 32] set forth in Table III herein and lectin-functional variants and fragments thereof.

7. The polypeptide of claim 6 further comprising 10-20 amino acid residues of the corresponding GalNAc-transferase sequence at its carboxy or amino terminus.

8. The polypeptide of any one of claims 5-7 wherein the truncated polypeptide is human.

9. A method of producing a lectin polypeptide according to claim 5, comprising:
(i) growing a host cell transfected with a nucleic acid sequence encoding a truncated mammalian GalNAc-transferase polypeptide comprising a domain selected from the group consisting of the lectin domain of a mammalian polypeptide GalNAc-transferase, and a lectin-functional variant or fragment of said lectin domain, wherein,
a. the lectin domain has lectin binding activity
b. the polypeptide does not encompass the intact, functioning catalytic domain of the enzyme, and
(ii) isolating the lectin polypeptide produced by the host cell.

10. A method according to claim 9 wherein said nucleic acid sequence is selected from the group consisting of the sequences encoding the GalNAc-T1[SEQ ID NO: 2], GalNAc-T2 [SEQ ID NO: 4], GalNAe-T3 [SEQ ID NO: 6], GalNAc-T4 [SEQ ID NO: 8], GalNAc-T5 [SEQ ID NO: 10], GalNAc-T6 [SEQ ID NO: 12], GalNAc-T7 [SEQ ID NO: 14], GalNAc-T8 [SEQ ID NO: 16], GalNAc-T9 [SEQ ID NO: 18], GalNAc-T10 [SEQ ID NO: 20], GalNAc-T11 [SEQ ID NO: 22], Ga1NAc-T12 [SEQ ID NO: 24], GalNAc-T13 [SEQ ID NO: 26], Ga1NAc-T14 [SEQ ID NO: 28], Ga1NAc-T15 [SEQ ID NO: 30] and Ga1NAc-T16 [SEQ ID NO: 32] lectin domains stated in Table III herein and lectin-functional variants and fragments thereof.

11. The method of claim 9 wherein the truncated polypeptide is human.

12. A method of identifying a substance that binds to the lectin domain of a GalNAc-transferase, which comprises:
(i) reacting a lectin polypeptide with at least one substance which potentially may bind to the polypeptide, under conditions which permit the association between the substance and the polypeptide;
(ii) removing and/or detecting the polypeptide with associated substance which, if present, indicates that the substance binds to the polypeptide,
wherein said polypeptide is a recombinant soluble GalNAc-transferase polypeptide and whose binding activity through the catalytic site is inactivated.

13. The method according to claim 12, wherein said recombinant GalNAc-transferase polypeptide comprises an affinity tag.

14. The method according to claim 13, wherein said affinity tag is biotin.

15. The method according to claim 12, wherein the catalytic site binding activity of the recombinant soluble GalNAc-transferase polypeptide is inactivated by mutation.

16. The method according to any one of claims 12-14, wherein the catalytic site of the recombinant soluble GalNAc-transferase polypeptide is inactivated by truncation.

17. The method according to claim 16, wherein the polypeptide is a truncated mammalian GalNAc-transferase polypeptide according to any one of claims 5-8.

18. A method of screening of test substances for inhibitors of functions mediated by polypeptide GalNAc-transferase lectin domains which comprises:
a) using a lectin polypeptide capable of displaying lectin-mediated binding in a binding assay where it interacts with a GalNAc or Galβ1-3GalNAc O-glycopeptide ligand or a molecular mimic hereof, under assay conditions suitable for the detection of said binding; and
b) measuring the binding inhibition of a test substance to identify and evaluate efficiency of a potential inhibitor,
wherein said polypeptide is a recombinant soluble GalNAc-transferase polypeptide and whose binding activity through the catalytic site is inactivated.

19. The method according to claim 18, wherein the catalytic site binding activity of the recombinant soluble GalNAc-transferase polypeptide is inactivated by mutation.

20. The method according to claim 18, wherein the polypeptide is a truncated mammalian GalNAc-transferase polypeptide according to any one of claims 5-8.

21. A method of screening of test substances for inhibitors of functions mediated by polypeptide GalNAc-transferase lectin domains which comprises:
a) using a polypeptide GalNAc-transferase or a fragment thereof retaining functional lectin binding in a binding assay where it interacts with a GalNAc or Galβ1-3GalNAc O-glycopeptide ligand or a molecular mimic hereof, while the binding capacity of the catalytic domain of said polypeptide is inactivated by the presence of EDTA or the absence of UDP or UDP-GalNAc or Mn⁺⁺ or other divalent metal ion, and
b) measuring the binding inhibition of a test substance to identify and evaluate efficiency of a potential inhibitor.

## Patentansprüche

1. Ein isoliertes Nukleinsäure-Molekül bestehend aus einer Nukleinsäuresequenz, die ein gekürztes Säuger-GalNAc-Transferase-Polypeptid codiert, welches eine Domäne ausgewählt aus der Gruppe bestehend aus einer Lectin-Domäne einer Säuger-Polypeptid-GalNAc-Transferase und einer/s Lectin-funktionellen Variante oder Fragments dieser Lectin-Domäne umfasst, wobei:
(i) die Lectin-Domäne Lectin-Bindungsaffinität aufweist
(ii) das Polypeptid die intakte, funktionierende katalytische Domäne des Enzyms nicht umfasst.

2. Ein Nukleinsäure-Molekül nach Anspruch 1 enthaltend eine Nukleinsäuresequenz ausgewählt aus der Gruppe bestehend aus den Nukleinsäuresequenzen, die die GalNAc-T1 [SEQ ID NO: 2], GalNAc-T2 [SEQ ID NO: 4], GalNAc-T3 [SEQ ID NO: 6], GalNAc-T4 [SEQ ID NO: 8], GalNAc-T5 [SEQ ID NO: 10], GalNAc-T6 [SEQ ID NO: 12], GalNAc-T7 [SEQ ID NO: 14], GalNAc-T8 [SEQ ID NO: 16], GalNAc-T9 [SEQ ID NO: 18], GalNAc-T10 [SEQ ID NO: 20], GalNAc-T11 [SEQ ID NO: 22], GalNAc-T12 [SEQ ID NO: 24], GalNAc-T13 [SEQ ID NO: 26], GalNAc-T14 [SEQ ID NO: 28], GalNAc-T15 [SEQ ID NO: 30] und GalNAc-T16 [SEQ ID NO: 32]-Lectindomänen, die in Tabelle III dargelegt sind, codieren.

3. Die Nukleinsäure des Anspruchs 2, weiter enthaltend 30 bis 60 Nukleotide der entsprechenden GalNAc-Transferase-Sequenz an ihrem 5' oder 3'-Ende.

4. Die Nukleinsäure nach einem der Ansprüche 1 bis 3, wobei das gekürzte GalNAc-Transferase-Polypeptid menschlichen Ursprungs ist.

5. Ein isoliertes Lectin-Polypeptid bestehend aus einem gekürzten Säuger-GalNAc-Tansferase-Polypeptid, enthaltend eine Domäne ausgewählt aus der Gruppe bestehend aus der Lectin-Domäne einer Säuger-Polypeptid-GalNAc-Transferase oder einer/s Lectin-funktionellen Variante oder Fragments davon, wobei:
(i) die Lectin-Domäne Lectin-Bindungsaffinität aufweist
(ii) das gekürzte Polypeptid die intakte, funktionierende katalytische Domäne des Enzyms nicht umfasst.

6. Ein Lectin-Polypeptid nach Anspruch 5 mit einer Aminosäuresequenz ausgewählt aus der Gruppe bestehend aus den Aminosäuresequenzen von GalNAc-T1 [SEQ ID NO: 2], GalNAc-T2 [SEQ ID NO: 4], GalNAc-T3 [SEQ ID NO: 6], GalNAc-T4 [SEQ ID NO: 8], GalNAc-T5 [SEQ ID NO: 10], GalNAc-T6 [SEQ ID NO: 12], GalNAc-T7 [SEQ ID NO: 14], GalNAc-T8 [SEQ ID NO: 16], GalNAc-T9 [SEQ ID NO: 18], GalNAc-T10 [SEQ ID NO: 20], GalNAc-T11 [SEQ ID NO: 22], GalNAc-T12 [SEQ ID NO: 24], GalNAc-T13 [SEQ ID NO: 26], GalNAc-T14 [SEQ ID NO: 28], GalNAc-T15 [SEQ ID NO: 30] und GalNAc-T16 [SEQ ID NO: 32], die in Tabelle III dargelegt sind, und Lectin-funktionelle Varianten und Fragmenten davon.

7. Das Polypeptid nach Anspruch 6, weiter enthaltend 10 bis 20 Aminosäurereste der entsprechenden GalNAc-Transferase-Sequenz an ihrem Carboxy- oder Amino-Terminus.

8. Das Polypeptid einer der Ansprüche 5 bis 7, wobei das gekürzte Polypeptid menschlichen Ursprungs ist.

9. Ein Verfahren zur Herstellung eines Lectin-Polypeptids nach Anspruch 5, enthaltend:
(i) das Züchten einer Wirtszelle, die mit einer Nukleinsäure-Sequenz transfiziert ist, welche ein gekürztes Säuger-GalNAc-Transferase-Polypeptid codiert, das eine Domäne ausgewählt aus der Gruppe bestehend aus der Lectin-Domäne einer Säuger-Polypeptid-GalNAc-Transferase und einer/s Lectin-funktionellen Variante oder Fragments dieser Lectin-Domäne umfasst, wobei
a. die Lectin-Domäne Lectin-Bindungsaffinität aufweist
b. das Polypeptid die intakte, funktionierende katalytische Domäne des Enzyms nicht umfasst, und
(ii) das Isolieren des durch die Wirtszelle hergestellten Lectin-Polypeptids.

10. Ein Verfahren nach Anspruch 9, wobei die Nukleinsäure-Sequenz ausgewählt ist aus der Gruppe bestehend aus den Sequenzen, die die GalNAc-T1 [SEQ ID NO: 2], GalNAc-T2 [SEQ ID NO: 4], GalNAc-T3 [SEQ ID NO: 6], GalNAc-T4 [SEQ ID NO: 8], GalNAc-T5 [SEQ ID NO: 10], GalNAc-T6 [SEQ ID NO: 12], GalNAc-T7 [SEQ ID NO: 14], GalNAc-T8 [SEQ ID NO: 16], GalNAc-T9 [SEQ ID NO: 18], GalNAc-T10 [SEQ ID NO: 20], GalNAc-T11 [SEQ ID NO: 22], GalNAc-T12 [SEQ ID NO: 24], GalNAc-T13 [SEQ ID NO: 26], GalNAc-T14 [SEQ ID NO: 28], GalNAc-T15 [SEQ ID NO: 30] und GalNAc-T16 [SEQ ID NO: 32]-Lectin-Domänen, die in Tabelle III angegeben sind, und Lectin-funktionelle Varianten und Fragmente davon codieren.

11. Das Verfahren nach Anspruch 9, wobei das gekürzte Polypeptid menschlichen Ursprungs ist.

12. Ein Verfahren zur Identifizierung eines Stoffes, welcher an die Lectin-Domäne einer GalNAc-Transferase bindet, welches umfasst:
(i) das Umsetzen eines Lectin-Polypeptids mit wenigstens einem Stoff, welcher potentiell an das Polypeptid binden kann, unter Bedingungen, die die Assoziation zwischen dem Stoff und dem Polypeptid erlauben;
(ii) das Entfernen und/oder Ermitteln des Polypeptids mit dem assoziierten Stoff, welcher, sofern anwesend, anzeigt, dass der Stoff an das Polypeptid bindet,
wobei das Polypeptid ein rekombinantes lösliches GalNAc-Transferase-Polypeptid ist, dessen Bindungsaktivität des katalytischen Zentrums inaktiviert ist.

13. Das Verfahren nach Anspruch 12, wobei das rekombinante GalNAc-Transferase-Polypeptid eine Affinitätsmarkierung aufweist.

14. Das Verfahren nach Anspruch 13, wobei die Affinitätsmarkierung Biotin ist.

15. Das Verfahren nach Anspruch 12, wobei die Bindungsaktivität des katalytischen Zentrums des rekombinanten löslichen GalNAc-Transferase-Polypeptids durch Mutation inaktiviert ist.

16. Das Verfahren nach einem der Ansprüche 12 bis 14, wobei das katalytische Zentrum des rekombinanten löslichen GalNAc-Transferase-Polypeptids durch Kürzung inaktiviert ist.

17. Das Verfahren nach Anspruch 16, wobei das Polypeptid ein gekürztes Säuger-GalNAc-Transferase-Polypeptid nach einem der Ansprüche 5 bis 8 ist.

18. Ein Verfahren zur Selektion von Teststoffen als Inhibitoren von Funktionen, die durch Polypeptid-GalNAc-Transferase-Lectindomänen vermittelt werden, welches umfasst:
a) das Verwenden eines Lectin-Polypeptids, das Lectin-vermittelte Bindung in einer Bindungsuntersuchung aufzeigen kann, in der es mit einem GalNAc- oder Galβ1-3GalNAc-O-Glycopeptid-Liganden oder einem molekularen Analogen davon interagiert, unter Untersuchungsbedingungen, die für die Detektion dieser Bindung geeignet sind; und
b) das Messen der Bindungsinhibition eines Teststoffes zur Identifizierung und Auswertung der Effizienz eines potentiellen Inhibitors,
wobei das Polypeptid ein rekombinantes lösliches GalNAc-Transferase-Polypeptid ist, dessen Bindungsaktivität des katalytischen Zentrums inaktiviert ist.

19. Das Verfahren nach Anspruch 18, wobei die Bindungsaktivität des katalytischen Zentrums des rekombinanten löslichen GalNAc-Transferase-Polypeptids durch Mutation inaktiviert ist.

20. Das Verfahren nach Anspruch 18, wobei das Polypeptid ein gekürztes Säuger-GalNAc-Transferase-Polypeptid nach einem der Ansprüche 5 bis 8 ist.

21. Ein Verfahren zur Selektion von Teststoffen als Inhibitoren von Funktionen, die durch Polypeptid-GalNAc-Transferase-Lectin-Domänen vermittelt werden, welches umfasst:
a) das Verwenden einer Polypeptid-GalNAc-Transferase oder eines Fragments davon, welches eine funktionelle Lectin-Bindung in einer Bindungsuntersuchung beibehält, in der es mit einem GalNAc- oder Galβ1-3GalNAc-O-Glycopeptid-Liganden oder einem molekularen Analogen davon interagiert, während die Bindungskapazität der katalytischen Domäne des Polypeptids durch die Anwesenheit von EDTA oder die Abwesenheit von UDP oder UDP-GalNAc oder Mn⁺⁺ oder anderen divalenten Metallionen inaktiviert ist, und
b) das Messen der Bindungsinhibition eines Teststoffes zur Identifizierung und Auswertung der Effizienz eines potentiellen Inhibitors.

## Revendications

1. Molécule d'acide nucléique isolée constituée d'une séquence d'acide nucléique codant pour un polypeptide tronqué de GalNAc-transférase de mammifère comprenant un domaine choisi dans le groupe constitué par le domaine lectine d'une polypeptide-GalNAc-transférase de mammifère, et un variant ou fragment à lectine fonctionnelle dudit domaine lectine, **caractérisée en ce que** :
(i) le domaine lectine possède une activité de liaison de lectine
(ii) le polypeptide ne contient pas le domaine catalytique fonctionnant et intact de l'enzyme.

2. Molécule d'acide nucléique selon la revendication 1, comprenant une séquence d'acide nucléique choisie dans le groupe constitué par les séquences d'acides nucléiques codant pour les domaines lectine de GalNAc-T1 [SEQ ID NO : 2], GalNAc-T2 [SEQ ID NO : 4], GalNAc-T3 [SEQ ID NO : 6], GalNAc-T4 [SEQ ID NO : 8], GalNAc-T5 [SEQ ID NO : 10], GalNAc-T6 [SEQ ID NO : 12], GalNAc-T7 [SEQ ID NO : 14], GalNAc-T8 [SEQ ID NO : 16], GalNAc-T9 [SEQ ID NO : 18], GalNAc-T10 [SEQ ID NO : 20], GalNAc-T11 [SEQ ID NO : 22], GalNAc-T12 [SEQ ID NO : 24], GalNAc-T13 [SEQ ID NO : 26], GalNAc-T14 [SEQ ID NO : 28], GalNAc-T15 [SEQ ID NO : 30] et GalNAc-T16 [SEQ ID NO : 32], exposées dans le tableau III ci-inclus.

3. Acide nucléique selon la revendication 2, comprenant en outre 30 à 60 nucléotides de la séquence de GalNAc-transférase correspondante au niveau de son extrémité 5' ou 3'.

4. Acide nucléique selon l'une quelconque des revendications 1 à 3, **caractérisé en ce que** le polypeptide tronqué de GalNAc-transférase est humain.

5. Polypeptide de lectine isolé constitué d'un polypeptide tronqué de GalNAc-transférase de mammifère comprenant un domaine choisi dans le groupe constitué par le domaine lectine d'une polypeptide-GalNAc-transférase de mammifère, et un variant ou fragment à lectine fonctionnelle de celui-ci, **caractérisé en ce que** :
(i) le domaine lectine possède une activité de liaison de lectine
(ii) le polypeptide tronqué ne contient pas le domaine catalytique fonctionnant et intact de l'enzyme.

6. Polypeptide de lectine selon la revendication 5, possédant une séquence d'acides aminés choisie dans le groupe constitué par les séquences d'acides aminés de GalNAc-T1 [SEQ ID NO : 2], GalNAc-T2 [SEQ ID NO : 4], GalNAc-T3 [SEQ ID NO : 6], GalNAc-T4 [SEQ ID NO : 8], GalNAc-T5 [SEQ ID NO : 10], GalNAc-T6 [SEQ ID NO : 12], GalNAc-T7 [SEQ ID NO : 14], GalNAc-T8 [SEQ ID NO : 16], GalNAc-T9 [SEQ ID NO : 18], GalNAc-T10 [SEQ ID NO : = 20], GalNAc-T11 [SEQ ID NO : 22], GalNAc-T12 [SEQ ID NO : 24], GalNAc-T13 [SEQ ID NO : 26], GalNAc-T14 [SEQ ID NO : 28], GalNAc-T15 [SEQ ID NO : 30] et GalNAc-T16 [SEQ ID NO : 32], exposées dans le tableau III = ci-inclus, et les variants et fragments à lectine fonctionnelle de celles-ci.

7. Polypeptide selon la revendication 6, comprenant en outre 10 à 20 résidus d'acides aminés de la séquence de GalNAc-transférase correspondante as niveau de son extrémité carboxy ou amino terminale.

8. Polypeptide selon l'une quelconque des revendications 5 à 7, **caractérisé en ce que** le polypeptide tronqué est humain.

9. Procédé de production d'un polypeptide de lectine selon la revendication 5, comprenant :
(i) la culture d'une cellule hôte transfectée avec une séquence d'acide nucléique codant pour un polypeptide tronqué de GalNAc-transférase de mammifère comprenant un domaine choisi dans le groupe constitué par le domaine lectine d'une polypeptide-GalNAc-transférase de mammifère et un variant ou fragment à lectine fonctionnelle dudit domaine lectine, où
a. le domaine lectine possède une activité de liaison de lectine
b. le polypeptide ne contient pas le domaine catalytique fonctionnant et intact de l'enzyme, et
(ii) l'isolement du polypeptide de lectine produit par la cellule hôte.

10. Procédé selon la revendication 9, **caractérise en ce que** ladite séquence d'acide nucléique est choisie dans le groupe constitué par les séquences codant pour les domaines lectine de GalNAc-T1 [SEQ ID NO : 2], GalNAc-T2 [SEQ ID NO : 4], GalNAc-T3 [SEQ ID NO : 6], GalNAc-T4 [SEQ ID NO : 8], GalNAc-T5 [SEQ ID NO : 10], GalNAc-T6 [SEQ ID NO : 12], GalNAc-T7 [SEQ ID NO : 14], GalNAc-T8 : [SEQ ID NO : 16], GalNAc-T9 [SEQ ID NO : 18], GalNAc-T10 [SEQ ID NO : 20], GalNAc-T11 [SEO ID NO : 22], GalNAc-T12 [SEQ ID NO : 24], GalNAc-T13 [SEQ ID NO : 26], GalNAc-T14 [SEQ ID NO : 28], GalNAc-T15 [SEQ ID NO : 30] et GalNAc-T16 [SEQ ID NO : 32], exposées dans le tableau III ci-inclus et les variants et fragments à lectine fonctionnelle de celles-ci.

11. Procédé selon la revendication 9, **caractérisé en ce que** le polypeptide tronqué est humain.

12. Procédé d'identification d'une substance qui se lie au domaine lectine d'une GalNAc-transférase, qui comprend :
(i) la réaction d'un polypeptide de lectine avec au moins une substance qui peut potentiellement se lier au polypeptide, dans des conditions qui permettent l'association entre la substance et le polypeptide ;
(ii) l'élimination et/ou la détection du polypeptide et la substance associée qui, s'ils sont présents, indiquent que la substance se lie au polypeptide,
**caractérisé en ce que** ledit polypeptide est un polypeptide de GalNAc-transférase soluble recombinant et dont l'activité de liaison par le site catalytique est inactivée.

13. Procédé selon la revendication 12, **caractérisé en ce que** ledit polypeptide de GalNAc-transférase recombinant comprend une étiquette d'affinité.

14. Procédé selon la revendication 13, **caractérisé en ce que** ladite étiquette d'affinité est la biotine.

15. Procédé selon la revendication 12, **caractérisé en ce que** l'activité de liaison du site catalytique du polypeptide de GalNAc-transférase soluble recombinant est inactivée par mutation.

16. Procédé selon l'une quelconque des revendications 12 à 14, **caractérisé en ce que** le site catalytique du polypeptide de GalNAc-transférase soluble recombinant est inactivé par troncature.

17. Procédé selon la revendication 16, **caractérisé en ce que** le polypeptide est un polypeptide tronqué de GalNAc-transférase de mammifère selon l'une quelconque des revendications 5 à 8.

18. Procédé de criblage de substances à l'essai pour des inhibiteurs de fonctions médiées par les domaines lectine de polypeptide-GalNAc-transférases qui comprend :
a) l'utilisation d'un polypeptide de lectine susceptible de présenter une liaison médiée par la lectine dans un dosage de liaison où il interagit avec un ligand glycopeptidique O-glycosylé par GalNAc ou Galβ1-3GalNAc ou un mime moléculaire de celui-ci, dans des conditions de dosage appropriées à la détection de ladite liaison ; et
b) la mesure de l'inhibition de liaison par une substance à l'essai pour identifier et évaluer l'efficacité d'un inhibiteur potentiel,
**caractérisé en ce que** ledit polypeptide est un polypeptide de GalNAc-transférase soluble recombinant et dont l'activité de liaison par le site catalytique est inactivée.

19. Procédé selon la revendication 18, **caractérisé en ce que** l'activité de liaison du site catalytique du polypeptide de GalNAc-transférase soluble recombinant est inactivée par mutation.

20. Procédé selon la revendication 18, **caractérisé en ce que** le polypeptide est un polypeptide tronqué de GalNAc-transférase de mammifère selon l'une quelconque des revendications 5 à 8.

21. Procédé de criblage de substances à l'essai pour des inhibiteurs de fonctions médiées par les domaines lectine de polypeptide-GalNAc-transférases qui comprend :
a) l'utilisation d'une polypeptide-GalNac-transférase ou d'un fragment de celle-ci conservant une liaison de lectine fonctionnelle dans un dosage de liaison où elle interagit avec un ligand glycopeptidique O-glycosylé par GalNAc ou Galβ1-3GalNAc ou un mime moléculaire de celui-ci, tandis que la capacité de liaison du domaine catalytique dudit polypeptide est inactivée par la présence d'EDTA ou l'absence d'UDP ou d'UDP-GalNAc ou de Mn⁺⁺ ou autre ion métallique divalent, et
b) la mesure de l'inhibition de liaison par une substance à l'essai pour identifier et évaluer l'efficacité d'un inhibiteur potentiel.
